# EUROPEAN PATENT APPLICATION

(11) **EP 4 173 636 A1**
(43) Date of publication of application: **03.05.2023**
(21) Application number: 21832297.2
(22) Date of filing: 30.06.2021
(51) Int. Cl.: A61K 39/395, A61K 45/00, A61P 35/00, A61P 43/00, C07K 16/28, C07K 16/46, C12N 15/13, C12N 15/62, A61K 33/24, A61K 31/282, A61K 31/337, A61K 31/4745, A61K 31/513, A61K 31/519, A61K 31/704, A61K 31/7048, A61K 31/7068

(54) **COMBINED USE OF ANTI-CCR8 ANTIBODY AND CHEMOTHERAPEUTIC AGENT**

(30) Priority: 30.06.2020 JP 2020112259; 26.11.2020 JP 2020196405
(71) Applicant: Shionogi & Co., Ltd, Osaka-shi, Osaka 541-0045 (JP)
(72) Inventor: TANAKA Hidekazu, Toyonaka-shi, Osaka 561-0825 (JP); NOGAMI Wataru, Toyonaka-shi, Osaka 561-0825 (JP); NAGAI Ryohei, Toyonaka-shi, Osaka 561-0825 (JP); SONODA Yudai, Toyonaka-shi, Osaka 561-0825 (JP)
(74) Representative: Mewburn Ellis LLP
(86) International application number: PCT/JP2021/024651
(87) International publication number: WO 2022/004760

(57) **Abstract**

The combined use of an anti-CCR8 antibody and a chemotherapeutic agent was found to be useful in the treatment or prevention of cancer.

## Description

### [TECHNICAL FIELD]

The present invention relates to combination of an anti-CCR8 antibody and a chemotherapeutic agent.

### [BACKGROUND ART]

Potent negative regulation mechanisms, including immunosuppression, mediated by regulatory T cells (Treg cells) in the tumor microenvironment are major obstacles to the treatment of tumors (Non-patent Document 1).

For example, CD4-positive Treg cells which infiltrate tumors may be able to strongly inhibit antitumor immune response and may become a major obstacle to effective cancer treatment.

Tumor immunosuppression mediated by CD4-positive FoxP3-positive Treg cells has been sufficiently demonstrated in animal tumor models. It has been reported that systemic (including intratumoral) Treg cell removal produces an antitumor effect, wherein the removal of approximately 50% tumor-infiltrating Treg cells is not effective (Non-patent Document 2).

It has been reported that the increased ratio of CD4-positive CD25-positive Treg cells (cell population including Treg cells) to the whole CD4-positive T cell population in humans is intratumorally detected in patients with various cancers including lung, breast, and ovary tumors, and the abundance ratio correlates negatively with the survival probabilities of the patients (Non-patent Documents 3 to 8).

CCR8, also previously called CY6, CKR-L1 or TER1, is a G protein-coupled 7-transmembrane CC chemokine receptor protein expressed in the thymus, the spleen, etc. A gene encoding this protein resides on human chromosome 3p21. Human CCR8 consists of 355 amino acids (Non-patent Document 9). CCL1 is known as an endogenous ligand for CCR8 (Non-patent Document 10). Human CCR8 cDNA is constituted by the nucleotide sequence represented by GenBank ACC No. NM_005201.3, and mouse CCR8 cDNA is constituted by the nucleotide sequence represented by GenBank ACC No. NM_007720.2.

CCR8 is also specifically expressed in tumor-infiltrating Treg cells, and it has been demonstrated that, when breast cancer cells were inoculated in CCR8-deficient and wild-type mice, breast cancer proliferation and metastasis were more suppressed in the CCR8-deficient mouse compared to the wild-type mice (Patent Document 1 and Non-patent Document 11). Furthermore, it has been disclosed that anti-CCR8 antibody administration to a cancer model animal showed an antitumor effect (Patent Documents 2, 3 and 9).

Regarding combination of an anti-CCR8 antibody and other drugs, the following reports have been made. Patent Documents 2 and 3 show an antitumor effect of combination of an anti-CCR8 antibody and an anti-PD-1 antibody. Patent Document 4 shows an antitumor effect of combination of an anti-CCR8 antibody and a Listeria cancer vaccine. Patent Document 5 discloses a double antibody against CCR8 and CTLA-4. However, the documents relate to combination of an anti-CCR8 antibody and an immunotherapeutic agent, and do not show combination of an anti-CCR8 antibody and a chemotherapeutic agent at all.

Regarding combination of an immunotherapeutic agent other than an anti-CCR8 antibody and a chemotherapeutic agent, the following reports have been made. Patent Document 6 shows an antitumor effect of combination of an anti-PD-1 antibody and axitinib as a VEGF receptor inhibitor. Patent Document 7 shows an antitumor effect of combination of an anti-PD-1 antibody and dinaciclib. Patent Document 8 shows an antitumor effect of combination of an anti-CTLA-4 antibody and various chemotherapeutic agents such as etoposide.

### [PRIOR ART REFERENCES]

### [Patent Documents]

[Patent Document 1] US 10087259
[Patent Document 2] International Publication WO 2018/181425
[Patent Document 3] International Publication WO 2018/112032
[Patent Document 4] International Publication WO 2019/157098
[Patent Document 5] CN 110835374 A
[Patent Document 6] JP 6591428
[Patent Document 7] JP 6586087
[Patent Document 8] JP 5589077
[Patent Document 9] International Publication WO 2020/138489

### [Non-patent Documents]

[Non-patent Document 1] Nat. Rev. Immunol., 2006, Vol. 6, No. 4, p.295-307
[Non-patent Document 2] Eur. J. Immunol., 2010, Vol. 40, p.3325-3335
[Non-patent Document 3] J. Clin. Oncol., 2006, Vol. 24, p.5373-5380
[Non-patent Document 4] Nat. Med., 2004, Vol. 10, p.942-949
[Non-patent Document 5] J. Clin. Oncol., 2007, Vol. 25, p.2586-2593
[Non-patent Document 6] Cancer, 2006, Vol. 107, p.2866-2872
[Non-patent Document 7] Eur. J. Cancer, 2008, Vol. 44, p.1875-1882
[Non-patent Document 8] Cell. Mol. Immunol. 2011, Vol. 8, p.59-66
[Non-patent Document 9] J. Immunol., 1996, Vol. 157, No. 7, p. 2759-63
[Non-patent Document 10] J. Biol. Chem., 1997, Vol. 272, No. 28, p. 17251-4
[Non-patent Document 11] Cancer Res., 2016, Vol. 76, No. 4, Supp.1, P4-04-11

### [SUMMARY OF THE INVENTION]

### [PROBLEMS TO BE SOLVED BY THE INVENTION]

An object of the present invention is to provide combination of an anti-CCR8 antibody and a chemotherapeutic agent. Another object of the present invention is to provide combination of an anti-CCR8 antibody and a chemotherapeutic agent which is useful for cancer treatment.

### [MEANS FOR SOLVING THE PROBLEMS]

The present inventors have found that administration of an anti-CCR8 antibody and a chemotherapeutic agent exhibits a combination effect. Further, the present inventors have found that the combination of an anti-CCR8 antibody and a chemotherapeutic agent according to the present invention is useful for treating or preventing cancer.

Specifically, the present invention relates to the following.
(1) A pharmaceutical composition which is used with a chemotherapeutic agent, the pharmaceutical composition comprising an anti-CCR8 antibody.
(2) A pharmaceutical composition which is used with an anti-CCR8 antibody, the pharmaceutical composition comprising a chemotherapeutic agent.
(3) The pharmaceutical composition according to (1) or (2), for cancer treatment.
(4) The pharmaceutical composition according to (3), wherein the treatment with an anti-PD-1 antibody or an anti-PD-L1 antibody is ineffective.
(5) The pharmaceutical composition according to any one of (1) to (4), wherein the cancer is breast cancer, lung cancer, colon cancer, kidney cancer, sarcoma, liver cancer, bladder cancer, or ovary cancer.
(6) The pharmaceutical composition according to (5), wherein the cancer is breast cancer, lung cancer, bladder cancer, kidney cancer, or colon cancer.
(6') The pharmaceutical composition according to (5), wherein the cancer is breast cancer, lung cancer, bladder cancer, or colon cancer.
(7) The pharmaceutical composition according to (5), wherein the cancer is breast cancer, lung cancer, or colon cancer.
(8) The pharmaceutical composition according to any one of (1) to (7), wherein the chemotherapeutic agent is a platinum complex, taxane, pemetrexed, gemcitabine, fluorouracil, irinotecan, etoposide, or doxorubicin.
(8') The pharmaceutical composition according to any one of (1) to (7), wherein the chemotherapeutic agent is a platinum complex, taxane, gemcitabine, or fluorouracil.
(9) The pharmaceutical composition according to (8), wherein the chemotherapeutic agent is gemcitabine.
(10) The pharmaceutical composition according to (8), wherein the chemotherapeutic agent is a platinum complex, and the platinum complex is carboplatin, cisplatin, or oxaliplatin.
(11) The pharmaceutical composition according to (10), wherein the platinum complex is carboplatin.
(12) The pharmaceutical composition according to (8), wherein the chemotherapeutic agent is a taxane, and the taxane is paclitaxel or docetaxel.
(13) The pharmaceutical composition according to (8), wherein the chemotherapeutic agent is fluorouracil.
(14) The pharmaceutical composition according to any one of (1) to (13), wherein the anti-CCR8 antibody is a monoclonal antibody or an antibody fragment comprising:
   1) a light chain variable region including CDR1 having the amino acid sequence of SEQ ID NO: 2,
      CDR2 having the amino acid sequence of SEQ ID NO: 3, and
      CDR3 having the amino acid sequence of SEQ ID NO: 4, and
         a heavy chain variable region including CDR1 having the amino acid sequence of SEQ ID NO: 5,
      CDR2 having the amino acid sequence of SEQ ID NO: 6, and
      CDR3 having the amino acid sequence of SEQ ID NO: 7,
         wherein one or more of the following substitutions are optionally present:
         A) substitution of asparagine with lysine at position 10 in the amino acid sequence of SEQ ID NO: 2,
         B) substitution of glycine with glutamine, threonine, alanine, lysine, leucine or arginine at position 11 in the amino acid sequence of SEQ ID NO: 2,
         C) substitution of asparagine with glutamine at position 4 in the amino acid sequence of SEQ ID NO: 3,
         D) substitution of leucine with isoleucine at position 5 in the amino acid sequence of SEQ ID NO: 3,
         E) substitution of leucine with isoleucine at position 4 in the amino acid sequence of SEQ ID NO: 4,
         F) substitution of tyrosine with phenylalanine at position 6 in the amino acid sequence of SEQ ID NO: 4,
         G) substitution of serine with threonine at position 16 in the amino acid sequence of SEQ ID NO: 6, and
         H) substitution of aspartic acid with glutamic acid at position 19 in the amino acid sequence of SEQ ID NO: 6;
   2) a light chain variable region including CDR1 having the amino acid sequence of SEQ ID NO: 2,
      CDR2 having the amino acid sequence of SEQ ID NO: 3, and
      CDR3 having the amino acid sequence of SEQ ID NO: 4, and
         a heavy chain variable region including CDR1 having the amino acid sequence of SEQ ID NO: 8,
      CDR2 having the amino acid sequence of SEQ ID NO: 6, and
      CDR3 having the amino acid sequence of SEQ ID NO: 7;
   3) a light chain variable region including CDR1 having the amino acid sequence of SEQ ID NO: 2,
      CDR2 having the amino acid sequence of SEQ ID NO: 9, and
      CDR3 having the amino acid sequence of SEQ ID NO: 10, and
         a heavy chain variable region including CDR1 having the amino acid sequence of SEQ ID NO: 8,
      CDR2 having the amino acid sequence of SEQ ID NO: 6, and
      CDR3 having the amino acid sequence of SEQ ID NO: 11,
         wherein one or more of the following substitutions are optionally present:
         A) substitution of serine with threonine at position 2 in the amino acid sequence of SEQ ID NO: 2,
         B) substitution of serine with threonine at position 3 in the amino acid sequence of SEQ ID NO: 2,
         C) substitution of serine with threonine at position 5 in the amino acid sequence of SEQ ID NO: 2,
         D) substitution of glutamine with asparagine at position 2 in the amino acid sequence of SEQ ID NO: 10,
         E) substitution of threonine with serine at position 1 in the amino acid sequence of SEQ ID NO: 8,
         F) substitution of alanine with valine at position 14 in the amino acid sequence of SEQ ID NO: 6,
         G) substitution of lysine with arginine at position 18 in the amino acid sequence of SEQ ID NO: 6,
         H) substitution of aspartic acid with glutamic acid at position 19 in the amino acid sequence of SEQ ID NO: 6,
         I) substitution of asparagine with glutamine at position 5 in the amino acid sequence of SEQ ID NO: 11, and
         J) substitution of tyrosine with phenylalanine at position 12 in the amino acid sequence of SEQ ID NO: 11;
   4) a light chain variable region including CDR1 having the amino acid sequence of SEQ ID NO: 2,
      CDR2 having the amino acid sequence of SEQ ID NO: 3, and
      CDR3 having the amino acid sequence of SEQ ID NO: 10, and
         a heavy chain variable region including CDR1 having the amino acid sequence of SEQ ID NO: 12,
      CDR2 having the amino acid sequence of SEQ ID NO: 6, and
      CDR3 having the amino acid sequence of SEQ ID NO: 13;
   5) a light chain variable region including CDR1 having the amino acid sequence of SEQ ID NO: 14,
      CDR2 having the amino acid sequence of SEQ ID NO: 15, and
      CDR3 having the amino acid sequence of SEQ ID NO: 16, and
         a heavy chain variable region including CDR1 having the amino acid sequence of SEQ ID NO: 17,
      CDR2 having the amino acid sequence of SEQ ID NO: 18, and
      CDR3 having the amino acid sequence of SEQ ID NO: 19; or
   6) a light chain variable region including CDR1 having the amino acid sequence of SEQ ID NO: 20,
      CDR2 having the amino acid sequence of SEQ ID NO: 21, and
      CDR3 having the amino acid sequence of SEQ ID NO: 22, and
      a heavy chain variable region including CDR1 having the amino acid sequence of SEQ ID NO: 23,
      CDR2 having the amino acid sequence of SEQ ID NO: 24, and
      CDR3 having the amino acid sequence of SEQ ID NO: 25.
(15) The pharmaceutical composition according to (14), wherein the anti-CCR8 antibody is a monoclonal antibody or an antibody fragment comprising:
   a light chain variable region including CDR1 having the amino acid sequence of SEQ ID NO: 2,
   CDR2 having the amino acid sequence of SEQ ID NO: 3, and
   CDR3 having the amino acid sequence of SEQ ID NO: 4, and
   a heavy chain variable region including CDR1 having the amino acid sequence of SEQ ID NO: 5,
   CDR2 having the amino acid sequence of SEQ ID NO: 6, and
   CDR3 having the amino acid sequence of SEQ ID NO: 7,
   wherein one or more of the following substitutions are present:
      A) substitution of asparagine with lysine at position 10 in the amino acid sequence of SEQ ID NO: 2; and
      B) substitution of glycine with glutamine, threonine, alanine, lysine, leucine or arginine at position 11 in the amino acid sequence of SEQ ID NO: 2;
      C) substitution of asparagine with glutamine at position 4 in the amino acid sequence of SEQ ID NO: 3;
      D) substitution of leucine with isoleucine at position 5 in the amino acid sequence of SEQ ID NO: 3;
      E) substitution of leucine with isoleucine at position 4 in the amino acid sequence of SEQ ID NO: 4;
      F) substitution of tyrosine with phenylalanine at position 6 in the amino acid sequence of SEQ ID NO: 4,
      G) substitution of serine with threonine at position 16 in the amino acid sequence of SEQ ID NO: 6, and
      H) substitution of aspartic acid with glutamic acid at position 19 in the amino acid sequence of SEQ ID NO: 6,
(16) The pharmaceutical composition according to (15), wherein the anti-CCR8 antibody is a monoclonal antibody or an antibody fragment having one or more of the following substitutions:
   A) substitution of asparagine with lysine at position 10 in the amino acid sequence of SEQ ID NO: 2;
   B) substitution of glycine with leucine or arginine at position 11 in the amino acid sequence of SEQ ID NO: 2; and
   C) substitution of asparagine with glutamine at position 4 in the amino acid sequence of SEQ ID NO: 3.
(17) The pharmaceutical composition according to (15) or (16), wherein the anti-CCR8 antibody is a monoclonal antibody or an antibody fragment in which asparagine at position 4 in the amino acid sequence of SEQ ID NO: 3 is substituted with glutamine, and
   one of the following substitutions is optionally present:
   A) substitution of asparagine with lysine at position 10 in the amino acid sequence of SEQ ID NO: 2; and
   B) substitution of glycine with leucine or arginine at position 11 in the amino acid sequence of SEQ ID NO: 2.
(18) The pharmaceutical composition according to (17), wherein the anti-CCR8 antibody is a monoclonal antibody or an antibody fragment in which asparagine at position 4 in the amino acid sequence of SEQ ID NO: 3 is substituted with glutamine, and glycine at position 11 in the amino acid sequence of SEQ ID NO: 2 is substituted with arginine.
(18') The pharmaceutical composition according to (14), wherein the anti-CCR8 antibody is a monoclonal antibody or an antibody fragment comprising:
   a light chain variable region including CDR1 having the amino acid sequence of SEQ ID NO: 2,
   CDR2 having the amino acid sequence of SEQ ID NO: 9, and
   CDR3 having the amino acid sequence of SEQ ID NO: 10, and
   a heavy chain variable region including CDR1 having the amino acid sequence of SEQ ID NO: 8,
   CDR2 having the amino acid sequence of SEQ ID NO: 6, and
   CDR3 having the amino acid sequence of SEQ ID NO: 11,
   wherein lysine at position 18 in the amino acid sequence of SEQ ID NO: 6 is substituted with arginine.
(18") The pharmaceutical composition according to (14), wherein the anti-CCR8 antibody is a monoclonal antibody or an antibody fragment comprising:
   a light chain variable region including CDR1 having the amino acid sequence of SEQ ID NO: 20,
   CDR2 having the amino acid sequence of SEQ ID NO: 21, and
   CDR3 having the amino acid sequence of SEQ ID NO: 22, and
   a heavy chain variable region including CDR1 having the amino acid sequence of SEQ ID NO: 23,
   CDR2 having the amino acid sequence of SEQ ID NO: 24, and
   CDR3 having the amino acid sequence of SEQ ID NO: 25.
(19) The pharmaceutical composition according to any one of (14) to (18),
   wherein the anti-CCR8 antibody is a humanized monoclonal antibody or an antibody fragment thereof.
(20) The pharmaceutical composition according to (19), wherein the humanized monoclonal antibody or an antibody fragment thereof comprises:
   1) a light chain variable region having the amino acid sequence of SEQ ID NO: 40, and
      a heavy chain variable region having the amino acid sequence of SEQ ID NO: 41;
   2) a light chain variable region having the amino acid sequence of SEQ ID NO: 42, and
      a heavy chain variable region having the amino acid sequence of SEQ ID NO: 41;
   3) a light chain variable region having the amino acid sequence of SEQ ID NO: 43, and
      a heavy chain variable region having the amino acid sequence of SEQ ID NO: 41;
   4) a light chain variable region having the amino acid sequence of SEQ ID NO: 44, and
      a heavy chain variable region having the amino acid sequence of SEQ ID NO: 41;
   5) a light chain variable region having the amino acid sequence of SEQ ID NO: 45, and
      a heavy chain variable region having the amino acid sequence of SEQ ID NO: 41;
   6) a light chain variable region having the amino acid sequence of SEQ ID NO: 47, and
      a heavy chain variable region having the amino acid sequence of SEQ ID NO: 41;
   7) a light chain variable region having the amino acid sequence of SEQ ID NO: 40, and
      a heavy chain variable region having the amino acid sequence of SEQ ID NO: 46;
   8) a light chain variable region having the amino acid sequence of SEQ ID NO: 42, and
      a heavy chain variable region having the amino acid sequence of SEQ ID NO: 46;
   9) a light chain variable region having the amino acid sequence of SEQ ID NO: 43, and
      a heavy chain variable region having the amino acid sequence of SEQ ID NO: 46;
   10) a light chain variable region having the amino acid sequence of SEQ ID NO: 44, and
      a heavy chain variable region having the amino acid sequence of SEQ ID NO: 46;
   11) a light chain variable region having the amino acid sequence of SEQ ID NO: 45, and
      a heavy chain variable region having the amino acid sequence of SEQ ID NO: 46; or
   12) a light chain variable region having the amino acid sequence of SEQ ID NO: 47, and
      a heavy chain variable region having the amino acid sequence of SEQ ID NO: 46,
      wherein one or more of the following substitutions are optionally present:
         A) substitution of asparagine with lysine at position 33 in the amino acid sequence of SEQ ID NO: 40, 42, 43, 44, 45 or 47;
         B) substitution of glycine with glutamine, threonine, alanine, lysine, leucine or arginine at position 34 in the amino acid sequence of SEQ ID NO: 40, 42, 43, 44, 45 or 47;
         C) substitution of asparagine with glutamine at position 58 in the amino acid sequence of SEQ ID NO: 40, 42, 43, 44, 45 or 47;
         D) substitution of leucine with isoleucine at position 59 in the amino acid sequence of SEQ ID NO: 40, 42, 43, 44, 45 or 47;
         E) substitution of leucine with isoleucine at position 97 in the amino acid sequence of SEQ ID NO: 40, 42, 43, 44, 45 or 47;
         F) substitution of tyrosine with phenylalanine at position 99 in the amino acid sequence of SEQ ID NO: 40, 42, 43, 44, 45 or 47;
         G) substitution of serine with threonine at position 65 in the amino acid sequence of SEQ ID NO: 41 or 46; and
         H) substitution of aspartic acid with glutamic acid at position 68 in the amino acid sequence of SEQ ID NO: 41 or 46.
(21) The pharmaceutical composition according to (20), wherein the humanized monoclonal antibody or an antibody fragment thereof has one or more of the following substitutions:
   A) substitution of asparagine with lysine at position 33 in the amino acid sequence of SEQ ID NO: 40, 42, 43, 44, 45 or 47;
   B) substitution of glycine with glutamine, threonine, alanine, lysine, leucine or arginine at position 34 in the amino acid sequence of SEQ ID NO: 40, 42, 43, 44, 45 or 47;
   C) substitution of asparagine with glutamine at position 58 in the amino acid sequence of SEQ ID NO: 40, 42, 43, 44, 45 or 47;
   D) substitution of leucine with isoleucine at position 59 in the amino acid sequence of SEQ ID NO: 40, 42, 43, 44, 45 or 47;
   E) substitution of leucine with isoleucine at position 97 in the amino acid sequence of SEQ ID NO: 40, 42, 43, 44, 45 or 47;
   F) substitution of tyrosine with phenylalanine at position 99 in the amino acid sequence of SEQ ID NO: 40, 42, 43, 44, 45 or 47;
   G) substitution of serine with threonine at position 65 in the amino acid sequence of SEQ ID NO: 41 or 46; and
   H) substitution of aspartic acid with glutamic acid at position 68 in the amino acid sequence of SEQ ID NO: 41 or 46.
(22) The pharmaceutical composition according to (21), wherein the humanized monoclonal antibody or an antibody fragment thereof comprises:
   a light chain variable region having the amino acid sequence of SEQ ID NO: 40 or 42; and
   a heavy chain variable region having the amino acid sequence of SEQ ID NO: 41,
   wherein one or more of the following substitutions are present:
      A) substitution of asparagine with lysine at position 33 in the amino acid sequence of SEQ ID NO: 40 or 42; and
      B) substitution of glycine with glutamine, threonine, alanine, lysine, leucine or arginine at position 34 in the amino acid sequence of SEQ ID NO: 40 or 42;
      C) substitution of asparagine with glutamine at position 58 in the amino acid sequence of SEQ ID NO: 40 or 42;
      D) substitution of leucine with isoleucine at position 59 in the amino acid sequence of SEQ ID NO: 40 or 42;
      E) substitution of leucine with isoleucine at position 97 in the amino acid sequence of SEQ ID NO: 40 or 42;
      F) substitution of tyrosine with phenylalanine at position 99 in the amino acid sequence of SEQ ID NO: 40 or 42;
      G) substitution of serine with threonine at position 65 in the amino acid sequence of SEQ ID NO: 41; and
      H) substitution of aspartic acid with glutamic acid at position 68 in the amino acid sequence of SEQ ID NO: 41.
(23) The pharmaceutical composition according to (21) or (22), wherein the humanized monoclonal antibody or an antibody fragment thereof comprises:
   a light chain variable region having the amino acid sequence of SEQ ID NO: 40 or 42; and
   a heavy chain variable region having the amino acid sequence of SEQ ID NO: 41,
   wherein one or more of the following substitutions are present:
      A) substitution of asparagine with lysine at position 33 in the amino acid sequence of SEQ ID NO: 40 or 42; and
      B) substitution of glycine with leucine or arginine at position 34 in the amino acid sequence of SEQ ID NO: 40 or 42; and
      C) substitution of asparagine with glutamine at position 58 in the amino acid sequence of SEQ ID NO: 40 or 42.
(24) The pharmaceutical composition according to any one of (21) to (23), wherein the humanized monoclonal antibody or an antibody fragment thereof comprises:
   a light chain variable region having the amino acid sequence of SEQ ID NO: 40 or 42; and
   a heavy chain variable region having the amino acid sequence of SEQ ID NO: 41,
   wherein asparagine at position 58 in the amino acid sequence of SEQ ID NO: 40 or 42 is substituted with glutamine, and
   one of the following substitutions is optionally present:
      A) substitution of asparagine with lysine at position 33 in the amino acid sequence of SEQ ID NO: 40 or 42; and
      B) substitution of glycine with leucine or arginine at position 34 in the amino acid sequence of SEQ ID NO: 40 or 42.
(25) The pharmaceutical composition according to (24), wherein the humanized monoclonal antibody or an antibody fragment thereof comprises:
   a light chain variable region having the amino acid sequence of SEQ ID NO: 42; and
   a heavy chain variable region having the amino acid sequence of SEQ ID NO: 41,
   wherein asparagine at position 58 in the amino acid sequence of SEQ ID NO: 42 is substituted with glutamine; and glycine at position 34 in the amino acid sequence of SEQ ID NO: 42 is substituted with arginine.
(26) The pharmaceutical composition according to (24), wherein the humanized monoclonal antibody or an antibody fragment thereof includes a light chain variable region having the amino acid sequence of SEQ ID NO: 59 and a heavy chain variable region having the amino acid sequence of SEQ ID NO: 41.
(26') The pharmaceutical composition according to (19), wherein the humanized monoclonal antibody or an antibody fragment thereof comprises a light chain variable region having the amino acid sequence of SEQ ID NO: 56 and a heavy chain variable region having the amino acid sequence of SEQ ID NO: 60.
(27) The pharmaceutical composition according to any one of (19) to (26),
   wherein the humanized monoclonal antibody or an antibody fragment thereof further comprises:
   a light chain variable region having the amino acid sequence of SEQ ID NO: 52; and
   a heavy chain variable region having the amino acid sequence of SEQ ID NO: 53,
   lysine is optionally added to the C-terminal in SEQ ID NO: 53.
(28) The pharmaceutical composition according to any one of (1) to (27),
   wherein the anti-CCR8 antibody is a neutralizing antibody.
(29) The pharmaceutical composition according to any one of (1) to (27),
   wherein the CCR8 antibody has ADCC activity.
(30) The pharmaceutical composition according to any one of (1) to (27),
   wherein the anti-CCR8 antibody is an IgG antibody.
(31) A method for treating cancer, comprising administering an anti-CCR8 antibody for use with a chemotherapeutic agent.
(32) A method for treating cancer, comprising administering a chemotherapeutic agent for use with an anti-CCR8 antibody.
(33) An anti-CCR8 antibody for treating cancer, which is used with a chemotherapeutic agent.
(34) A chemotherapeutic agent for treating cancer, which is used with an anti-CCR8 antibody.
(35) Use of an anti-CCR8 antibody for producing a medicament for cancer treatment, which is used with a chemotherapeutic agent.
(36) Use of a chemotherapeutic agent for producing a medicament for cancer treatment, which is used with an anti-CCR8 antibody.

(1-2) A medicament comprising a combination of an anti-CCR8 antibody and a chemotherapeutic agent.
(3-2) The medicament according to (1-2), for cancer treatment.
(4-2) The medicament according to (3-2), wherein the treatment with an anti-PD-1 antibody or an anti-PD-L1 antibody is ineffective.
(5-2) The medicament according to any one of (1-2) to (4-2), wherein the cancer is breast cancer, lung cancer, colon cancer, kidney cancer, sarcoma, liver cancer, bladder cancer, or ovary cancer.
(6-2) The medicament according to (5-2), wherein the cancer is breast cancer, lung cancer, bladder cancer, kidney cancer, or colon cancer.
(6'-2) The medicament according to (5-2), wherein the cancer is breast cancer, lung cancer, bladder cancer, or colon cancer.
(7-2) The medicament according to (5-2), wherein the cancer is breast cancer, lung cancer, or colon cancer.
(8-2) The medicament according to any one of (1-2) to (7-2), wherein the chemotherapeutic agent is a platinum complex, taxane, pemetrexed, gemcitabine, fluorouracil, irinotecan, etoposide, or doxorubicin.
(8'-2) The medicament according to any one of (1-2) to (7-2), wherein the chemotherapeutic agent is a platinum complex, taxane, gemcitabine, or fluorouracil.
(9-2) The medicament according to (8-2), wherein the chemotherapeutic agent is gemcitabine.
(10-2) The medicament according to (8-2), wherein the chemotherapeutic agent is a platinum complex, and the platinum complex is carboplatin, cisplatin, or oxaliplatin.
(11-2) The medicament according to (10-2), wherein the platinum complex is carboplatin.
(12-2) The medicament according to (8-2), wherein the chemotherapeutic agent is a taxane, and the taxane is paclitaxel or docetaxel.
(13-2) The medicament according to (8-2), wherein the chemotherapeutic agent is fluorouracil.
(14-2) The medicament according to any one of (1-2) to (13-2), wherein the anti-CCR8 antibody is a monoclonal antibody or an antibody fragment comprising:
   1) a light chain variable region including CDR1 having the amino acid sequence of SEQ ID NO: 2,
      CDR2 having the amino acid sequence of SEQ ID NO: 3, and
      CDR3 having the amino acid sequence of SEQ ID NO: 4, and
      a heavy chain variable region including CDR1 having the amino acid sequence of SEQ ID NO: 5,
      CDR2 having the amino acid sequence of SEQ ID NO: 6, and
      CDR3 having the amino acid sequence of SEQ ID NO: 7,
      wherein one or more of the following substitutions are optionally present:
         A) substitution of asparagine with lysine at position 10 in the amino acid sequence of SEQ ID NO: 2,
         B) substitution of glycine with glutamine, threonine, alanine, lysine, leucine or arginine at position 11 in the amino acid sequence of SEQ ID NO: 2,
         C) substitution of asparagine with glutamine at position 4 in the amino acid sequence of SEQ ID NO: 3,
         D) substitution of leucine with isoleucine at position 5 in the amino acid sequence of SEQ ID NO: 3,
         E) substitution of leucine with isoleucine at position 4 in the amino acid sequence of SEQ ID NO: 4,
         F) substitution of tyrosine with phenylalanine at position 6 in the amino acid sequence of SEQ ID NO: 4,
         G) substitution of serine with threonine at position 16 in the amino acid sequence of SEQ ID NO: 6, and
         H) substitution of aspartic acid with glutamic acid at position 19 in the amino acid sequence of SEQ ID NO: 6;
   2) a light chain variable region including CDR1 having the amino acid sequence of SEQ ID NO: 2,
      CDR2 having the amino acid sequence of SEQ ID NO: 3, and
      CDR3 having the amino acid sequence of SEQ ID NO: 4, and
      a heavy chain variable region including CDR1 having the amino acid sequence of SEQ ID NO: 8,
      CDR2 having the amino acid sequence of SEQ ID NO: 6, and
      CDR3 having the amino acid sequence of SEQ ID NO: 7;
   3) a light chain variable region including CDR1 having the amino acid sequence of SEQ ID NO: 2,
      CDR2 having the amino acid sequence of SEQ ID NO: 9, and
      CDR3 having the amino acid sequence of SEQ ID NO: 10, and
      a heavy chain variable region including CDR1 having the amino acid sequence of SEQ ID NO: 8,
      CDR2 having the amino acid sequence of SEQ ID NO: 6, and
      CDR3 having the amino acid sequence of SEQ ID NO: 11,
      wherein one or more of the following substitutions are optionally present:
         A) substitution of serine with threonine at position 2 in the amino acid sequence of SEQ ID NO: 2,
         B) substitution of serine with threonine at position 3 in the amino acid sequence of SEQ ID NO: 2,
         C) substitution of serine with threonine at position 5 in the amino acid sequence of SEQ ID NO: 2,
         D) substitution of glutamine with asparagine at position 2 in the amino acid sequence of SEQ ID NO: 10,
         E) substitution of threonine with serine at position 1 in the amino acid sequence of SEQ ID NO: 8,
         F) substitution of alanine with valine at position 14 in the amino acid sequence of SEQ ID NO: 6,
         G) substitution of lysine with arginine at position 18 in the amino acid sequence of SEQ ID NO: 6,
         H) substitution of aspartic acid with glutamic acid at position 19 in the amino acid sequence of SEQ ID NO: 6,
         I) substitution of asparagine with glutamine at position 5 in the amino acid sequence of SEQ ID NO: 11, and
         J) substitution of tyrosine with phenylalanine at position 12 in the amino acid sequence of SEQ ID NO: 11;
   4) a light chain variable region including CDR1 having the amino acid sequence of SEQ ID NO: 2,
      CDR2 having the amino acid sequence of SEQ ID NO: 3, and
      CDR3 having the amino acid sequence of SEQ ID NO: 10, and
      a heavy chain variable region including CDR1 having the amino acid sequence of SEQ ID NO: 12,
      CDR2 having the amino acid sequence of SEQ ID NO: 6, and
      CDR3 having the amino acid sequence of SEQ ID NO: 13;
   5) a light chain variable region including CDR1 having the amino acid sequence of SEQ ID NO: 14,
      CDR2 having the amino acid sequence of SEQ ID NO: 15, and
      CDR3 having the amino acid sequence of SEQ ID NO: 16, and
      a heavy chain variable region including CDR1 having the amino acid sequence of SEQ ID NO: 17,
      CDR2 having the amino acid sequence of SEQ ID NO: 18, and
      CDR3 having the amino acid sequence of SEQ ID NO: 19; or
   6) a light chain variable region including CDR1 having the amino acid sequence of SEQ ID NO: 20,
      CDR2 having the amino acid sequence of SEQ ID NO: 21, and
      CDR3 having the amino acid sequence of SEQ ID NO: 22, and
      a heavy chain variable region including CDR1 having the amino acid sequence of SEQ ID NO: 23,
      CDR2 having the amino acid sequence of SEQ ID NO: 24, and
      CDR3 having the amino acid sequence of SEQ ID NO: 25.
(15-2) The medicament according to (14-2), wherein the anti-CCR8 antibody is a monoclonal antibody or an antibody fragment comprising:
   a light chain variable region including CDR1 having the amino acid sequence of SEQ ID NO: 2,
   CDR2 having the amino acid sequence of SEQ ID NO: 3, and
   CDR3 having the amino acid sequence of SEQ ID NO: 4, and
   a heavy chain variable region including CDR1 having the amino acid sequence of SEQ ID NO: 5,
   CDR2 having the amino acid sequence of SEQ ID NO: 6, and
   CDR3 having the amino acid sequence of SEQ ID NO: 7,
   wherein one or more of the following substitutions are present:
      A) substitution of asparagine with lysine at position 10 in the amino acid sequence of SEQ ID NO: 2; and
      B) substitution of glycine with glutamine, threonine, alanine, lysine, leucine or arginine at position 11 in the amino acid sequence of SEQ ID NO: 2;
      C) substitution of asparagine with glutamine at position 4 in the amino acid sequence of SEQ ID NO: 3;
      D) substitution of leucine with isoleucine at position 5 in the amino acid sequence of SEQ ID NO: 3;
      E) substitution of leucine with isoleucine at position 4 in the amino acid sequence of SEQ ID NO: 4;
      F) substitution of tyrosine with phenylalanine at position 6 in the amino acid sequence of SEQ ID NO: 4,
      G) substitution of serine with threonine at position 16 in the amino acid sequence of SEQ ID NO: 6; and
      H) substitution of aspartic acid with glutamic acid at position 19 in the amino acid sequence of SEQ ID NO: 6.
(16-2) The medicament according to (15-2), wherein the anti-CCR8 antibody is a monoclonal antibody or an antibody fragment having one or more of the following substitutions:
   A) substitution of asparagine with lysine at position 10 in the amino acid sequence of SEQ ID NO: 2;
   B) substitution of glycine with leucine or arginine at position 11 in the amino acid sequence of SEQ ID NO: 2; and
   C) substitution of asparagine with glutamine at position 4 in the amino acid sequence of SEQ ID NO: 3.
(17-2) The medicament according to (15-2) or (16-2), wherein the anti-CCR8 antibody is a monoclonal antibody or an antibody fragment in which asparagine at position 4 in the amino acid sequence of SEQ ID NO: 3 is substituted with glutamine, and
   one of the following substitutions is optionally present:
   A) substitution of asparagine with lysine at position 10 in the amino acid sequence of SEQ ID NO: 2; and
   B) substitution of glycine with leucine or arginine at position 11 in the amino acid sequence of SEQ ID NO: 2.
(18-2) The medicament according to (17-2), wherein the anti-CCR8 antibody is a monoclonal antibody or an antibody fragment in which asparagine at position 4 in the amino acid sequence of SEQ ID NO: 3 is substituted with glutamine, and glycine at position 11 in the amino acid sequence of SEQ ID NO: 2 is substituted with arginine.
(18'-2) The medicament according to (14-2), wherein the anti-CCR8 antibody is a monoclonal antibody or an antibody fragment comprising:
   a light chain variable region including CDR1 having the amino acid sequence of SEQ ID NO: 2,
   CDR2 having the amino acid sequence of SEQ ID NO: 9, and
   CDR3 having the amino acid sequence of SEQ ID NO: 10, and
   a heavy chain variable region including CDR1 having the amino acid sequence of SEQ ID NO: 8,
   CDR2 having the amino acid sequence of SEQ ID NO: 6, and
   CDR3 having the amino acid sequence of SEQ ID NO: 11,
   wherein lysine at position 18 in the amino acid sequence of SEQ ID NO: 6 is substituted with arginine.
(18"-2) The medicament according to (14-2), wherein the anti-CCR8 antibody is a monoclonal antibody or an antibody fragment comprising:
   a light chain variable region including CDR1 having the amino acid sequence of SEQ ID NO: 20,
   CDR2 having the amino acid sequence of SEQ ID NO: 21, and
   CDR3 having the amino acid sequence of SEQ ID NO: 22, and
   a heavy chain variable region including CDR1 having the amino acid sequence of SEQ ID NO: 23,
   CDR2 having the amino acid sequence of SEQ ID NO: 24, and
   CDR3 having the amino acid sequence of SEQ ID NO: 25.
(19-2) The medicament according to any one of (14-2) to (18-2), wherein the anti-CCR8 antibody is a humanized monoclonal antibody or an antibody fragment thereof.
(20-2) The medicament according to (19-2), wherein the humanized monoclonal antibody or an antibody fragment thereof comprises:
   1) a light chain variable region having the amino acid sequence of SEQ ID NO: 40, and
      a heavy chain variable region having the amino acid sequence of SEQ ID NO: 41;
   2) a light chain variable region having the amino acid sequence of SEQ ID NO: 42, and
      a heavy chain variable region having the amino acid sequence of SEQ ID NO: 41;
   3) a light chain variable region having the amino acid sequence of SEQ ID NO: 43, and
      a heavy chain variable region having the amino acid sequence of SEQ ID NO: 41;
   4) a light chain variable region having the amino acid sequence of SEQ ID NO: 44, and
      a heavy chain variable region having the amino acid sequence of SEQ ID NO: 41;
   5) a light chain variable region having the amino acid sequence of SEQ ID NO: 45, and
      a heavy chain variable region having the amino acid sequence of SEQ ID NO: 41;
   6) a light chain variable region having the amino acid sequence of SEQ ID NO: 47, and
      a heavy chain variable region having the amino acid sequence of SEQ ID NO: 41;
   7) a light chain variable region having the amino acid sequence of SEQ ID NO: 40, and
      a heavy chain variable region having the amino acid sequence of SEQ ID NO: 46;
   8) a light chain variable region having the amino acid sequence of SEQ ID NO: 42, and
      a heavy chain variable region having the amino acid sequence of SEQ ID NO: 46;
   9) a light chain variable region having the amino acid sequence of SEQ ID NO: 43, and
      a heavy chain variable region having the amino acid sequence of SEQ ID NO: 46;
   10) a light chain variable region having the amino acid sequence of SEQ ID NO: 44, and
      a heavy chain variable region having the amino acid sequence of SEQ ID NO: 46;
   11) a light chain variable region having the amino acid sequence of SEQ ID NO: 45, and
      a heavy chain variable region having the amino acid sequence of SEQ ID NO: 46; or
   12) a light chain variable region having the amino acid sequence of SEQ ID NO: 47, and
      a heavy chain variable region having the amino acid sequence of SEQ ID NO: 46,
      wherein one or more of the following substitutions are optionally present:
         A) substitution of asparagine with lysine at position 33 in the amino acid sequence of SEQ ID NO: 40, 42, 43, 44, 45 or 47;
         B) substitution of glycine with glutamine, threonine, alanine, lysine, leucine or arginine at position 34 in the amino acid sequence of SEQ ID NO: 40, 42, 43, 44, 45 or 47;
         C) substitution of asparagine with glutamine at position 58 in the amino acid sequence of SEQ ID NO: 40, 42, 43, 44, 45 or 47;
         D) substitution of leucine with isoleucine at position 59 in the amino acid sequence of SEQ ID NO: 40, 42, 43, 44, 45 or 47;
         E) substitution of leucine with isoleucine at position 97 in the amino acid sequence of SEQ ID NO: 40, 42, 43, 44, 45 or 47;
         F) substitution of tyrosine with phenylalanine at position 99 in the amino acid sequence of SEQ ID NO: 40, 42, 43, 44, 45 or 47;
         G) substitution of serine with threonine at position 65 in the amino acid sequence of SEQ ID NO: 41 or 46; and
         H) substitution of aspartic acid with glutamic acid at position 68 in the amino acid sequence of SEQ ID NO: 41 or 46.
(21-2) The medicament according to (20-2), wherein the humanized monoclonal antibody or an antibody fragment thereof has one or more of the following substitutions:
   A) substitution of asparagine with lysine at position 33 in the amino acid sequence of SEQ ID NO: 40, 42, 43, 44, 45 or 47;
   B) substitution of glycine with glutamine, threonine, alanine, lysine, leucine or arginine at position 34 in the amino acid sequence of SEQ ID NO: 40, 42, 43, 44, 45 or 47;
   C) substitution of asparagine with glutamine at position 58 in the amino acid sequence of SEQ ID NO: 40, 42, 43, 44, 45 or 47;
   D) substitution of leucine with isoleucine at position 59 in the amino acid sequence of SEQ ID NO: 40, 42, 43, 44, 45 or 47;
   E) substitution of leucine with isoleucine at position 97 in the amino acid sequence of SEQ ID NO: 40, 42, 43, 44, 45 or 47;
   F) substitution of tyrosine with phenylalanine at position 99 in the amino acid sequence of SEQ ID NO: 40, 42, 43, 44, 45 or 47;
   G) substitution of serine with threonine at position 65 in the amino acid sequence of SEQ ID NO: 41 or 46; and
   H) substitution of aspartic acid with glutamic acid at position 68 in the amino acid sequence of SEQ ID NO: 41 or 46.
(22-2) The medicament according to (21-2), wherein the humanized monoclonal antibody or an antibody fragment thereof comprises:
   a light chain variable region having the amino acid sequence of SEQ ID NO: 40 or 42; and
   a heavy chain variable region having the amino acid sequence of SEQ ID NO: 41,
   wherein one or more of the following substitutions are present:
      A) substitution of asparagine with lysine at position 33 in the amino acid sequence of SEQ ID NO: 40 or 42;
      B) substitution of glycine with glutamine, threonine, alanine, lysine, leucine or arginine at position 34 in the amino acid sequence of SEQ ID NO: 40 or 42;
      C) substitution of asparagine with glutamine at position 58 in the amino acid sequence of SEQ ID NO: 40 or 42;
      D) substitution of leucine with isoleucine at position 59 in the amino acid sequence of SEQ ID NO: 40 or 42;
      E) substitution of leucine with isoleucine at position 97 in the amino acid sequence of SEQ ID NO: 40 or 42;
      F) substitution of tyrosine with phenylalanine at position 99 in the amino acid sequence of SEQ ID NO: 40 or 42;
      G) substitution of serine with threonine at position 65 in the amino acid sequence of SEQ ID NO: 41; and
      H) substitution of aspartic acid with glutamic acid at position 68 in the amino acid sequence of SEQ ID NO: 41.
(23-2) The medicament according to (21-2) or (22-2), wherein the humanized monoclonal antibody or an antibody fragment thereof comprises:
   a light chain variable region having the amino acid sequence of SEQ ID NO: 40 or 42; and
   a heavy chain variable region having the amino acid sequence of SEQ ID NO: 41,
   wherein one or more of the following substitutions are present:
      A) substitution of asparagine with lysine at position 33 in the amino acid sequence of SEQ ID NO: 40 or 42;
      B) substitution of glycine with leucine or arginine at position 34 in the amino acid sequence of SEQ ID NO: 40 or 42; and
      C) substitution of asparagine with glutamine at position 58 in the amino acid sequence of SEQ ID NO: 40 or 42.
(24-2) The medicament according to any one of (21-2) to (23-2), wherein the humanized monoclonal antibody or an antibody fragment thereof comprises:
   a light chain variable region having the amino acid sequence of SEQ ID NO: 40 or 42; and
   a heavy chain variable region having the amino acid sequence of SEQ ID NO: 41,
   wherein asparagine at position 58 in the amino acid sequence of SEQ ID NO: 40 or 42 is substituted with glutamine, and
   one of the following substitutions is optionally present:
      A) substitution of asparagine with lysine at position 33 in the amino acid sequence of SEQ ID NO: 40 or 42; and
      B) substitution of glycine with leucine or arginine at position 34 in the amino acid sequence of SEQ ID NO: 40 or 42.
(25-2) The medicament according to (24-2), wherein the humanized monoclonal antibody or an antibody fragment thereof comprises:
   a light chain variable region having the amino acid sequence of SEQ ID NO: 42; and
   a heavy chain variable region having the amino acid sequence of SEQ ID NO: 41,
   wherein asparagine at position 58 in the amino acid sequence of SEQ ID NO: 42 is substituted with glutamine; and glycine at position 34 in the amino acid sequence of SEQ ID NO: 42 is substituted with arginine.
(26-2) The medicament according to (24-2), wherein the humanized monoclonal antibody or an antibody fragment thereof comprises a light chain variable region having the amino acid sequence of SEQ ID NO: 59 and a heavy chain variable region having the amino acid sequence of SEQ ID NO: 41.
(26'-2) The medicament according to (19-2), wherein the humanized monoclonal antibody or an antibody fragment thereof comprises a light chain variable region having the amino acid sequence of SEQ ID NO: 56 and a heavy chain variable region having the amino acid sequence of SEQ ID NO: 60.
(27-2) The medicament according to any one of (19-2) to (26-2), wherein the humanized monoclonal antibody or an antibody fragment thereof further comprises:
   a light chain variable region having the amino acid sequence of SEQ ID NO: 52; and
   a heavy chain variable region having the amino acid sequence of SEQ ID NO: 53,
   wherein lysine is optionally added at the C-terminal in SEQ ID NO: 53.
(28-2) The medicament according to any one of (1-2) to (27-2), wherein the anti-CCR8 antibody is a neutralizing antibody.
(29-2) The medicament according to any one of (1-2) to (27-2), wherein the anti-CCR8 antibody has ADCC activity.
(30-2) The medicament according to any one of (1-2) to (27-2), wherein the anti-CCR8 antibody is an IgG antibody.
(31-2) A method for treating cancer, comprising administering an anti-CCR8 antibody and a chemotherapeutic agent.

### [EFFECT OF THE INVENTION]

The combination of an anti-CCR8 antibody and a chemotherapeutic agent according to the present invention has an effect which results from the combination and is more marked than the effect of the anti-CCR8 antibody alone and the effect of the chemotherapeutic agent alone. Therefore, the combination of an anti-CCR8 antibody and a chemotherapeutic agent according to the present invention is very useful as a pharmaceutical product, particularly a medicament for treating or preventing cancer.

### [BRIEF DESCRIPTION OF THE DRAWINGS]

[Fig. 1] Fig. 1 shows an amino acid sequence comparison of human CCR8, human CCR4, and each chimera in which the N-terminal region, loop1 region, loop2 region, or loop3 region, which are the extracellular domains of human CCR8, is replaced with a corresponding N-terminal region, loop1 region, loop2 region, or loop3 region of human CCR4, respectively.
[Fig. 2] Fig. 2 shows the Kabat numbering and alignment results for light chain variable regions of 10A11, 27G1, 1H4, 19D7, 8F7 and 2C7.
[Fig. 3] Fig. 3 shows the Kabat numbering and alignment results for heavy chain variable regions of 10A11, 27G1, 1H4, 19D7, 8F7 and 2C7.
[Fig. 4] Fig. 4 shows the Kabat numbering and alignment results for the light chain variable region of 10A11, IGKV4-1, IGKV3-20, IGKV1-39, IGKV2-40, IGKV2-28, and IGKV1-16.
[Fig. 5] Fig. 5 shows the Kabat numbering and alignment results for the heavy chain variable region of 10A11, IGHV3-15 T94R and IGHV3-73.
[Fig. 6] Fig. 6 shows the Kabat numbering and alignment results for the light chain variable region of 19D7, IGKV3-15, IGKV2-18 and IGKV3-20.
[Fig. 7] Fig. 7 shows the Kabat numbering and alignment results for the heavy chain variable region of 19D7, IGHV3-15 T94R and IGHV3-73.
[Fig. 8] Fig. 8 shows the antitumor activity of anti-human CCR8 antibodies which was evaluated in human CCR8 knock-in mice inoculated with colon cancer-derived CT26 cells by measuring the tumor volume after inoculation. For significance levels, ** indicates p < 0.01 and *** indicates p < 0.001 by Welch's t test.
[Fig. 9] Fig. 9 shows the antitumor activity of combination of an anti-mouse CCR8 antibody and carboplatin (CBDCA) which was evaluated in a mouse inoculated with breast cancer-derived 4T1 cells.
[Fig. 10] Fig. 10 shows the antitumor activity of combination of the anti-mouse CCR8 antibody and gemcitabine (GEM) which was evaluated in a mouse inoculated with breast cancer-derived 4T1 cells.
[Fig. 11] Fig. 11 shows the antitumor activity of combination of an anti-human CCR8 antibody and cisplatin (CDDP) which was evaluated in a human CCR8 knock-in mouse inoculated with breast cancer-derived 4T1 cells.
[Fig. 12] Fig. 12 shows the antitumor activity of combination of the anti-mouse CCR8 antibody and carboplatin (CBDCA) which was evaluated in a mouse inoculated with bladder cancer-derived MB 49 cells.
[Fig. 13] Fig. 13 shows the antitumor activity of combination of the anti-mouse CCR8 antibody and cisplatin (CDDP) which was evaluated in a mouse inoculated with bladder cancer-derived MB 49 cells.
[Fig. 14] Fig. 14 shows the antitumor activity of combination of the anti-mouse CCR8 antibody and oxaliplatin which was evaluated in a mouse inoculated with colon cancer-derived Colon26 cells.
[Fig. 15] Fig. 15 shows the antitumor activity of combination of the anti-mouse CCR8 antibody and fluorouracil (5-FU) which was evaluated in a mouse inoculated with colon cancer-derived Colon26 cells.
[Fig. 16] Fig. 16 shows the antitumor activity of combination of the anti-human CCR8 antibody and oxaliplatin which was evaluated in a human CCR8 knock-in mouse inoculated with colon cancer-derived Colon26 cells.
[Fig. 17] Fig. 17 shows the antitumor activity of combination of the anti-human CCR8 antibody and cisplatin (CDDP) which was evaluated in a human CCR8 knock-in mouse inoculated with lung cancer-derived ASB-XIV cells.
[Fig. 18] Fig. 18 shows the antitumor activity of combination of the anti-human CCR8 antibody and paclitaxel (PTX) which was evaluated in a human CCR8 knock-in mouse inoculated with lung cancer-derived ASB-XIV cells.
[Fig. 19] Fig. 19 shows the antitumor activity of combination of an anti-human CCR8 antibody and docetaxel (DTX) which was evaluated in a human CCR8 knock-in mouse inoculated with breast cancer-derived 4T1 cells.
[Fig. 20] Fig. 20 shows the antitumor activity of combination of the anti-mouse CCR8 antibody and docetaxel (DTX) which was evaluated in a mouse inoculated with breast cancer-derived 4T1 cells.
[Fig. 21] Fig. 21 shows the antitumor activity of combination of the anti-human CCR8 antibody and oxaliplatin which was evaluated in a human CCR8 knock-in mouse inoculated with colon cancer-derived Colon26 cells.
[Fig. 22] Fig. 22 shows the antitumor activity of the combination of the anti-human CCR8 antibody and oxaliplatin which was evaluated in a human CCR8 knock-in mouse inoculated with colon cancer-derived Colon26 cells.
[Fig. 23] Fig. 23 shows the antitumor activity of the combination of the anti-human CCR8 antibody and fluorouracil (5-FU) which was evaluated in a human CCR knock-in mouse inoculated with colon cancer-derived CT 26 cells.

### [MODE FOR CARRYING OUT THE INVENTION]

The term used in the present description is used in the meaning usually used in the art, unless specifically mentioned.

Antibody-producing techniques known in the art can be used in the present invention. Examples of the techniques include a method described in Immunochemistry in Practice (Blackwell Scientific Publiations).

Genetically engineered techniques known in the art can also be used. Examples of the techniques include methods described in Molecular Cloning, A Laboratory Manual, Forth Edition, Cold Spring Harbor Laboratory Press (2012), and Current Protocols Essential Laboratory Techniques, Current Protocols (2012).

The hybridoma producing the anti-CCR8 antibody according to the present invention can be produced by using, as an immunogen, a CCR8 protein, a gene encoding the full length of CCR8, a CCR8 expressing cell, or the like. For example, when a gene encoding the full length of human CCR8 is used an immunogen, a hybridoma producing the anti-CCR8 antibody can be prepared by fusing a spleen cell of a mouse which has been DNA-immunized with the gene as an antigen into a mouse myeloma cell.

The amino acid sequence of human CCR8 is shown in UniProtKB/Swiss-Prot: P51685 (SEQ ID NO: 1).

One aspect of the monoclonal antibody or an antibody fragment thereof according to the present invention includes a monoclonal antibody or a fragment thereof having a CDR or a heavy/light chain variable region described in the present description. The antibody or an antibody fragment may be from any class or subclass of immunoglobulin molecule (e.g., IgG, IgE, IgM, IgD, or IgA, preferably, IgG). The antibody or antibody fragment may also be obtained from any species, such as mouse, rat, shark, rabbit, pig, hamster, camel, llama, goat, or human. The antibody or antibody fragment thereof is preferably a humanized monoclonal antibody or an antibody fragment of a humanized monoclonal antibody.

Preferably, the monoclonal antibody according to the present invention has the following sequences as CDR sequences:
(1) light chain CDR1: SEQ ID NO: 2, 14 or 20;
(2) light chain CDR2: SEQ ID NO: 3, 9, 15, or 21;
(3) light chain CDR3: SEQ ID NO: 4, 10, 16, or 22;
(4) heavy chain CDR1: SEQ ID NO: 5, 8, 12, 17, or 23;
(5) heavy chain CDR2: SEQ ID NO: 6, 18, or 24; and
(6) heavy chain CDR3: SEQ ID NO: 7, 11, 13, 19, or 25.

More preferably, the monoclonal antibody has the following sequences:
(1) light chain CDR1: SEQ ID NO: 2;
(2) light chain CDR2: SEQ ID NO: 3, or 9;
(3) light chain CDR3: SEQ ID NO: 4, or 10;
(4) heavy chain CDR1: SEQ ID NO: 5, 8, or 12;
(5) heavy chain CDR2: SEQ ID NO: 6;
(6) heavy chain CDR3: SEQ ID NO: 7, 11, or 13.

Most preferably, the monoclonal antibody has the following sequences:
(1) light chain CDR1: SEQ ID NO: 2;
(2) light chain CDR2: SEQ ID NO: 3;
(3) light chain CDR3: SEQ ID NO: 4;
(4) heavy chain CDR1: SEQ ID NO: 5;
(5) heavy chain CDR2: SEQ ID NO: 6; and
(6) heavy chain CDR3: SEQ ID NO: 7.

In each of the amino acid sequences (SEQ ID NOS: 2 to 25), one or two amino acids may be deleted, substituted, inserted and/or added.

Herein, in the CDR sequence of the monoclonal antibody of the present invention, asparagine may be substituted with glutamine or lysine, aspartic acid may be substituted with glutamic acid, leucine may be substituted with isoleucine, tyrosine may be substituted with phenylalanine, serine may be substituted with threonine, and glycine may be substituted with glutamine, threonine, alanine, lysine, leucine, or arginine.

Preferably, one or more of the following substitutions are optionally present:
A) substitution of asparagine with lysine at position 10 in the amino acid sequence of SEQ ID NO: 2;
B) substitution of glycine with glutamine, threonine, alanine, lysine, leucine or arginine at position 11 in the amino acid sequence of SEQ ID NO: 2;
C) substitution of asparagine with glutamine at position 4 in the amino acid sequence of SEQ ID NO: 3.
D) substitution of leucine with isoleucine at position 5 in the amino acid sequence of SEQ ID NO: 3;
E) substitution of leucine with isoleucine at position 4 in the amino acid sequence of SEQ ID NO: 4;
F) substitution of tyrosine with phenylalanine at position 6 in the amino acid sequence of SEQ ID NO: 4;
G) substitution of serine with threonine at position 16 in the amino acid sequence of SEQ ID NO: 6; and
H) substitution of aspartic acid with glutamic acid at position 19 in the amino acid sequence of SEQ ID NO: 6.

More preferably, one or more of the following substitutions are optionally present:
A) substitution of asparagine with lysine at position 10 in the amino acid sequence of SEQ ID NO: 2;
B) substitution of glycine with leucine or arginine at position 11 in the amino acid sequence of SEQ ID NO: 2; and
C) substitution of asparagine with glutamine at position 4 in the amino acid sequence of SEQ ID NO: 3.

Further preferably, asparagine at position 4 in the amino acid sequence of SEQ ID NO: 3 is substituted with glutamine, and
one of the following substitutions is optionally present:
A) substitution of asparagine with lysine at position 10 in the amino acid sequence of SEQ ID NO: 2; and
B) substitution of glycine with leucine or arginine at position 11 in the amino acid sequence of SEQ ID NO: 2.

When the monoclonal antibody of the present invention has the following sequences:
(1) light chain CDR1: SEQ ID NO: 2;
(2) light chain CDR2: SEQ ID NO: 9;
(3) light chain CDR3: SEQ ID NO: 10;
(4) heavy chain CDR1: SEQ ID NO: 8;
(5) heavy chain CDR2: SEQ ID NO: 6; and
(6) heavy chain CDR3: SEQ ID NO: 11,
   one or more of the following substitutions are optionally present:
   A) substitution of serine with threonine at position 2 in the amino acid sequence of SEQ ID NO: 2;
   B) substitution of serine with threonine at position 3 in the amino acid sequence of SEQ ID NO: 2;
   C) substitution of serine with threonine at position 5 in the amino acid sequence of SEQ ID NO: 2;
   D) substitution of glutamine with asparagine at position 2 in the amino acid sequence of SEQ ID NO: 10;
   E) substitution of threonine with serine at position 1 in the amino acid sequence of SEQ ID NO: 8;
   F) substitution of alanine with valine at position 14 in the amino acid sequence of SEQ ID NO: 6;
   G) substitution of lysine with arginine at position 18 in the amino acid sequence of SEQ ID NO: 6;
   H) substitution of aspartic acid with glutamic acid at position 19 in the amino acid sequence of SEQ ID NO: 6;
   I) substitution of asparagine with glutamine at position 5 in the amino acid sequence of SEQ ID NO: 11; and
   J) substitution of tyrosine with phenylalanine at position 12 in the amino acid sequence of SEQ ID NO: 11.

The "antibody fragment of a monoclonal antibody" means a portion of the monoclonal antibody according to the present invention and a fragment that specifically binds to CCR8 and selectively inhibits CCR8 in the same manner as the monoclonal antibody.

Specifically, examples of the antibody fragment include Fab (fragment of antigen binding), Fab', F(ab')₂, a single chain antibody (single chain Fv; hereinafter referred to as scFv), a disulfide stabilized antibody (disulfide stabilized Fv; hereinafter referred to as dsFv), a dimerized V-region fragment (hereinafter referred to as diabody), a peptide comprising CDR, and the like, which specifically bind to CCR8 (Expert Opinion on Therapeutic Patents, vol. 6, No. 5, pp.441-456, 1996).

Fab is an antibody fragment having an antigen-binding activity of about 50,000 molecular weight, composed of about half of the N-terminal end side of the H-chain and the entire L-chain obtained by degrading the peptide portion of the upper part of the two disulfide bonds (S-S bonds) crosslinking the two H-chains in the hinge region of IgG with an enzyme papain. Fab used in the present invention can be obtained by treating the monoclonal antibody according to the present invention with papain. Fab can also be produced by inserting DNA encoding Fab of the monoclonal antibody according to the present invention into a cell expression vector, and introducing the obtained vector into a cell to express Fab.

Fab' is an antibody fragment having an antigen-binding activity of about 50,000 molecular weight, obtained by cleaving S-S bonds in the hinge of F(ab')₂. Fab' used in the present invention can be obtained by treating F(ab')₂ of the monoclonal antibody according to the present invention with a reducing agent dithiothreitol. Fab' can also be produced by inserting DNA encoding Fab' of the monoclonal antibody according to the present invention into a cell expression vector, and introducing the obtained vector into E. coli, yeast, or an animal cell to express Fab'.

F(ab')₂ is an antibody fragment having an antigen-binding activity of about 100,000 molecular weight, composed of two Fab' regions bonded at the hinge portion obtained by degrading the lower part of the two S-S bonds in the hinge region of IgG with an enzyme pepsin. F(ab')₂ used in the present invention can be obtained by treating the monoclonal antibody according to the present invention with pepsin. F(ab')₂ can also be produced by inserting DNA encoding F(ab')₂ of the monoclonal antibody of the present invention into a cell expression vector, and introducing the obtained vector into E. coli, yeast, or an animal cell to express F(ab')₂.

scFv is a VH-P-VL or VL-P-VH polypeptide in which one VH and one VL are linked with an appropriate peptide linker (hereinafter referred to as P), and is an antibody fragment having antigen-binding activity. The VH and VL contained in scFv used in the present invention may be those of the monoclonal antibody according to the present invention. scFv used in the present invention can also be produced by constructing a scFv expression vector using cDNA encoding the VH and VL of the monoclonal antibody according to the present invention and introducing the obtained vector into E. coli, yeast, or an animal cell to express scFv.

dsFv refers to an antibody fragment obtained by bonding polypeptides in which 1 amino acid residue in VH and VL is substituted with a cysteine residue, respectively, via an S-S bond. The amino acid residues to be substituted with cysteine residues can be selected based on stereostructural predictions of the antibody according to the method shown by Reiter et al. (Protein Engineering, 7, 697 (1994)). The VH or VL contained in the dsFv used in the present invention may be those of the monoclonal antibody according to the present invention. dsFv used in the present invention can also be produced by constructing a dsFv expression vector by inserting cDNA encoding the VH and VL of the monoclonal antibody according to the present invention into an appropriate expression vector and introducing the obtained vector into E. coli, yeast, or an animal cell to express dsFv.

The diabody is an antibody fragment in which scFvs having the same or different antigen-binding specificity form a dimer, and is an antibody fragment having divalent antigen-binding activity for the same antigen or two different specific antigen-binding activities for different antigens. For example, a divalent diabody that specifically reacts to the monoclonal antibody according to the present invention can be produced by using cDNA encoding the VH and VL of the monoclonal antibody according to the present invention to construct a DNA encoding scFv having a peptide linker of 3 to 10 residues, inserting the DNA into a cell expression vector, introducing the obtained expression vector into E. coli, yeast, or an animal cell to express the diabody.

A peptide comprising a CDR is composed of at least one or more regions of a CDR of VH or VL. The plurality of CDRs can be bound directly or via an appropriate peptide linker. The peptide comprising a CDR used in the present invention can be produced by constructing a CDR-encoding DNA using cDNA encoding the VH and VL of the monoclonal antibody according to the present invention, inserting the DNA into an animal cell expression vector, and introducing the obtained vector into E. coli, yeast, or an animal cell to express the peptide. The peptide comprising a CDR can also be produced by chemical synthesis methods such as the Fmoc method (fluorenylmethyloxycarbonyl method), the tBoc method (t-butyloxycarbonyl method), and the like.

The monoclonal antibody and an antibody fragment thereof according to the present invention are characterized in that they bind to CCR8. In particular, those that specifically bind to human CCR8 are preferred.

The specific binding can be characterized by an equilibrium dissociation constant of at least about 1 × 10⁻⁶ M or less (for example, the smaller Kd represents the closer binding). The Kd value is preferably 1 × 10⁻⁷ M or less, more preferably 1 × 10⁻⁸ M or less, further preferably 1 × 10⁻⁹ M or less. Methods for determining whether two molecules specifically bind are well known in the art, and examples thereof include competitive ELISA methods, surface plasmon resonance, and the like other method.

The monoclonal antibody or an antibody fragment thereof according to the present invention are characterized in that they inhibit a binding of CCR8 to a CCR8 ligand. The "CCR8 ligand" is not particularly limited as long as it is a substance that binds to CCR8, such as CCL1, CCL8, or CCL18, but is preferably CCL1, CCL18, and particularly preferably CCL1.

The inhibitory ability of binding of CCR8 to a CCR8 ligand can be determined, when the CCR8 ligand is human CCL1, for example, by using human CCR8-expressing 293 cells, determining Ca²⁺ influx by human CCL1 addition, and calculating an IC50 value with setting that the signal when human CCL1 is not added as the inhibition rate of 100% and the signal when human CCL1 is added and the antibody is not added as the inhibition rate of 0%. The inhibitory ability of binding to other CCR8 ligands can also be determined in a similar manner to human CCL1 as described above.

Human CCL1 has an amino acid sequence shown by UniProtKB/Swiss-Prot No. P22362 or the like. Human CCL8 has the amino acid sequence shown by GenBank No. AAI 26243.1 or the like. Human CCL18 has the amino acid sequence shown by GenBank No. EAW80102.1 or the like.

The monoclonal antibody according to the present invention can be produced by a conventional method in the art using a CDR or heavy chain variable region/light chain variable region described in the present description.

The monoclonal antibody according to the present invention also includes a chimeric antibody, a humanized antibody, a fully human antibody, an antibody-drug conjugate (ADC) and a bispecific antibody.

The humanized monoclonal antibody is useful when administered to humans for therapeutic purposes or the like, because it is less antigenic in humans. A humanized monoclonal antibody is an antibody in which a complementarity determining region (CDR) of a non-human mammal antibody, such as a mouse antibody, is implanted into a framework region (FR) of a human antibody. The FR of a humanized monoclonal antibody is thus derived from a human. Suitable FR can be selected by referring to the documents of Kabat E.A. et al. As the FR in this case, one with which the CDR can form an appropriate antigen binding site is selected. As necessary, amino acids of the FR of a variable region of the antibody may be substituted so that the CDR of the reconstituted humanized monoclonal antibody form an appropriate antigen binding site (Sato, K. et al., Cancer Res.1993, vol. 53, p.851). The percentage of amino acids of the FR to be substituted is from 0 to 15%, preferably from 0 to 5%, of the total FR region.

Preferably, the humanized monoclonal antibody according to the present invention includes:
a light chain variable region having the amino acid sequence of SEQ ID NO: 40, 42, 43, 44, 45 or 47, or
an amino acid sequence identical by 95% or more to the amino acid sequence 40, 42, 43, 44, 45 or 47; and
a heavy chain variable region having the amino acid sequence of SEQ ID NO: 41 or 46, or
an amino acid sequence identical by 95% or more to the amino acid sequence 41 or 46.

More preferably, the humanized monoclonal antibody includes:
1) a light chain variable region having the amino acid sequence of SEQ ID NO: 40, and
   a heavy chain variable region having the amino acid sequence of SEQ ID NO: 41;
2) a light chain variable region having the amino acid sequence of SEQ ID NO: 42, and
   a heavy chain variable region having the amino acid sequence of SEQ ID NO: 41;
3) a light chain variable region having the amino acid sequence of SEQ ID NO: 43, and
   a heavy chain variable region having the amino acid sequence of SEQ ID NO: 41;
4) a light chain variable region having the amino acid sequence of SEQ ID NO: 44, and
   a heavy chain variable region having the amino acid sequence of SEQ ID NO: 41;
5) a light chain variable region having the amino acid sequence of SEQ ID NO: 45, and
   a heavy chain variable region having the amino acid sequence of SEQ ID NO: 41;
6) a light chain variable region having the amino acid sequence of SEQ ID NO: 47, and
   a heavy chain variable region having the amino acid sequence of SEQ ID NO: 41;
7) a light chain variable region having the amino acid sequence of SEQ ID NO: 40, and
   a heavy chain variable region having the amino acid sequence of SEQ ID NO: 46;
8) a light chain variable region having the amino acid sequence of SEQ ID NO: 42, and
   a heavy chain variable region having the amino acid sequence of SEQ ID NO: 46;
9) a light chain variable region having the amino acid sequence of SEQ ID NO: 43, and
   a heavy chain variable region having the amino acid sequence of SEQ ID NO: 46;
10) a light chain variable region having the amino acid sequence of SEQ ID NO: 44, and
   a heavy chain variable region having the amino acid sequence of SEQ ID NO: 46;
11) a light chain variable region having the amino acid sequence of SEQ ID NO: 45, and
   a heavy chain variable region having the amino acid sequence of SEQ ID NO: 46; or
12) a light chain variable region having the amino acid sequence of SEQ ID NO: 47, and
   a heavy chain variable region having the amino acid sequence of SEQ ID NO: 46,
   wherein one or more of the following substitutions are optionally present:
      A) substitution of asparagine with lysine at position 33 in the amino acid sequence of SEQ ID NO: 40, 42, 43, 44, 45 or 47;
      B) substitution of glycine with glutamine, threonine, alanine, lysine, leucine or arginine at position 34 in the amino acid sequence of SEQ ID NO: 40, 42, 43, 44, 45 or 47;
      C) substitution of asparagine with glutamine at position 58 in the amino acid sequence of SEQ ID NO: 40, 42, 43, 44, 45 or 47;
      D) substitution of leucine with isoleucine at position 59 in the amino acid sequence of SEQ ID NO: 40, 42, 43, 44, 45 or 47;
      E) substitution of leucine with isoleucine at position 97 in the amino acid sequence of SEQ ID NO: 40, 42, 43, 44, 45 or 47;
      F) substitution of tyrosine with phenylalanine at position 99 in the amino acid sequence of SEQ ID NO: 40, 42, 43, 44, 45 or 47;
      G) substitution of serine with threonine at position 65 in the amino acid sequence of SEQ ID NO: 41 or 46; and
      H) substitution of aspartic acid with glutamic acid at position 68 in the amino acid sequence of SEQ ID NO: 41 or 46.

Further preferably, the humanized monoclonal antibody includes:
1) a light chain variable region having the amino acid sequence of SEQ ID NO: 40, and
   a heavy chain variable region having the amino acid sequence of SEQ ID NO: 41; or
2) a light chain variable region having the amino acid sequence of SEQ ID NO: 42, and
   a heavy chain variable region having the amino acid sequence of SEQ ID NO: 41,
   wherein one or more of the following substitutions are optionally present:
      A) substitution of asparagine with lysine at position 33 in the amino acid sequence of SEQ ID NO: 40 or 42;
      B) substitution of glycine with glutamine, threonine, alanine, lysine, leucine or arginine at position 34 in the amino acid sequence of SEQ ID NO: 40 or 42;
      C) substitution of asparagine with glutamine at position 58 in the amino acid sequence of SEQ ID NO: 40 or 42;
      D) substitution of leucine with isoleucine at position 59 in the amino acid sequence of SEQ ID NO: 40 or 42;
      E) substitution of leucine with isoleucine at position 97 in the amino acid sequence of SEQ ID NO: 40 or 42;
      F) substitution of tyrosine with phenylalanine at position 99 in the amino acid sequence of SEQ ID NO: 40 or 42;
      G) substitution of serine with threonine at position 65 in the amino acid sequence of SEQ ID NO: 41; and
      H) substitution of aspartic acid with glutamic acid at position 68 in the amino acid sequence of SEQ ID NO: 41.

Particularly preferably, the humanized monoclonal antibody includes:
1) a light chain variable region having the amino acid sequence of SEQ ID NO: 40, and
   a heavy chain variable region having the amino acid sequence of SEQ ID NO: 41; or
2) a light chain variable region having the amino acid sequence of SEQ ID NO: 42, and
   a heavy chain variable region having the amino acid sequence of SEQ ID NO: 41,
   wherein one or more of the following substitutions are optionally present:
      A) substitution of asparagine with lysine at position 33 in the amino acid sequence of SEQ ID NO: 40 or 42;
      B) substitution of glycine with leucine or arginine at position 34 in the amino acid sequence of SEQ ID NO: 40 or 42; and
      C) substitution of asparagine with glutamine at position 58 in the amino acid sequence of SEQ ID NO: 40 or 42;

Most preferably, the humanized monoclonal antibody includes:
1) a light chain variable region having the amino acid sequence of SEQ ID NO: 40, and
   a heavy chain variable region having the amino acid sequence of SEQ ID NO: 41; or
2) a light chain variable region having the amino acid sequence of SEQ ID NO: 42, and
   a heavy chain variable region having the amino acid sequence of SEQ ID NO: 41,
   wherein asparagine at position 58 in the amino acid sequence of SEQ ID NO: 40 or 42 is substituted with glutamine, and
   one of the following substitutions is optionally present:
      A) substitution of asparagine with lysine at position 33 in the amino acid sequence of SEQ ID NO: 40 or 42;
      B) substitution of glycine with leucine or arginine at position 34 in the amino acid sequence of SEQ ID NO: 40 or 42; and

The humanized monoclonal antibody is most preferably a humanized monoclonal antibody including a light chain variable region having the amino acid sequence of SEQ ID NO: 42 and a heavy chain variable region having the amino acid sequence of SEQ ID NO: 41, wherein asparagine at position 58 in the amino acid sequence of SEQ ID NO: 42 is substituted with glutamine, and glycine at position 34 in the amino acid sequence of SEQ ID NO: 42 is substituted with arginine, that is, a humanized monoclonal antibody including a light chain variable region having the amino acid sequence of SEQ ID NO: 59 and a heavy chain variable region having the amino acid sequence of SEQ ID NO: 41.

Examples of sequences of light and heavy chain variable regions in the humanized monoclonal antibodies according to the present invention include combinations in Table 1.

**[Table 1]**

| Combination | Light chain variable region SEQ ID NO: | Type of substitution | Heavy chain variable region SEQ ID NO: | Type of substitution |
|---|---|---|---|---|
| 1 | 42 | C | 41 | None |
| 2 | 42 | C+A | 41 | None |
| 3 | 42 | C+A | 41 | H |
| 4 | 42 | C+B5 | 41 | None |
| 5 | 42 | C+B5 | 41 | H |
| 6 | 42 | C+B6 | 41 | None |
| 7 | 42 | C+B6 | 41 | H |
| 8 | 40 | C | 41 | None |
| 9 | 40 | C+A | 41 | None |
| 10 | 40 | C+A | 41 | H |
| 11 | 40 | C+B5 | 41 | None |
| 12 | 40 | C+B5 | 41 | H |
| 13 | 40 | C+B6 | 41 | None |
| 14 | 40 | C+B6 | 41 | H |
| 15 | 42 | D | 41 | None |
| 16 | 42 | E | 41 | None |
| 17 | 42 | F | 41 | None |
| 18 | 42 | B1 | 41 | None |
| 19 | 42 | B6 | 41 | None |
| 20 | 42 | B2+C | 41 | None |
| 21 | 42 | B3+C+D | 41 | None |
| 22 | 42 | None | 41 | H |
| 23 | 42 | C | 41 | H |
| 24 | 42 | None | 41 | G+H |
| 25 | 42 | A | 41 | G+H |
| 26 | 40 | D | 41 | None |
| 27 | 40 | E | 41 | None |
| 28 | 40 | F | 41 | None |
| 29 | 40 | B4 | 41 | None |
| 30 | 40 | B5 | 41 | None |
| 31 | 40 | None | 41 | H |
| 32 | 40 | A | 41 | H |

Herein, the symbols in the "Type of substitution" in Table 1 respectively mean the following substitutions.
(1) Light chain variable region having the amino acid sequence of SEQ ID NO: 40 or 42
   A) Substitution of asparagine with lysine at position 33 in the amino acid sequence.
   B1) Substitution of glycine with glutamine at position 34 in the amino acid sequence.
   B2) Substitution of glycine with threonine at position 34 in the amino acid sequence.
   B3) Substitution of glycine with alanine at position 34 in the amino acid sequence.
   B4) Substitution of glycine with lysine at position 34 in the amino acid sequence.
   B5) Substitution of glycine with leucine at position 34 in the amino acid sequence.
   B6) Substitution of glycine with arginine at position 34 in the amino acid sequence.
   C) Substitution of asparagine with glutamine at position 58 in the amino acid sequence.
   D) Substitution of leucine with isoleucine at position 59 in the amino acid sequence.
   E) Substitution of leucine with isoleucine at position 97 in the amino acid sequence.
   F) Substitution of tyrosine with phenylalanine at position 99 in the amino acid sequence.
(2) Heavy chain variable region having the amino acid sequence of SEQ ID NO: 41
   G) substitution of serine with threonine at position 65 in the amino acid sequence.
   H) Substitution of aspartic acid with glutamic acid at position 68 in the amino acid sequence.

Another aspect of the humanized monoclonal antibody according to the present invention includes:
a light chain variable region having the amino acid sequence of SEQ ID NO: 40, 42, 43, 44, 45 or 47, or
an amino acid sequence identical by 95% or more to the amino acid sequence 40, 42, 43, 44, 45 or 47, wherein the amino acids at positions 26, 27, 30 and 98 in the amino acid sequence are serine, lysine, leucine and glutamic acid, respectively; and
a heavy chain variable region having the amino acid sequence of SEQ ID NO: 41 or 46, or
an amino acid sequence identical by 95% or more to the amino acid sequence of SEQ ID NO: 41 or 46, wherein the amino acids at positions 33, 35, 52, 56, 62, 102, 103, 104, 109, and 113 in the amino acid sequence are alanine, tyrosine, arginine, asparagine, tyrosine, arginine, phenylalanine, tyrosine, glycine, and aspartic acid, respectively.

More preferably, the humanized monoclonal antibody includes:
a light chain variable region having the amino acid sequence of SEQ ID NO: 40 or 42, or
an amino acid sequence identical by 95% or more to the amino acid sequence 40 or 42, wherein the amino acids at positions 26, 27, 30 and 98 in the amino acid sequence are serine, lysine, leucine and glutamic acid, respectively; and
a heavy chain variable region having the amino acid sequence of SEQ ID NO: 41 or 46, or
an amino acid sequence identical by 95% or more to the amino acid sequence of SEQ ID NO: 41 or 46, wherein the amino acids at positions 33, 35, 52, 56, 62, 102, 103, 104, 109, and 113 in the amino acid sequence are alanine, tyrosine, arginine, asparagine, tyrosine, arginine, phenylalanine, tyrosine, glycine, and aspartic acid, respectively.

Further preferably, the humanized monoclonal antibody includes:
a light chain variable region having the amino acid sequence of SEQ ID NO: 42, or
an amino acid sequence identical by 95% or more to the amino acid sequence 42, wherein the amino acids at positions 26, 27, 30 and 98 in the amino acid sequence are serine, lysine, leucine and glutamic acid, respectively; and
a light chain variable region having the amino acid sequence of SEQ ID NO: 41 or 46, or
an amino acid sequence identical by 95% or more to the amino acid sequence of SEQ ID NO: 41 or 46, wherein the amino acids at positions 33, 35, 52, 56, 62, 102, 103, 104, 109, and 113 in the amino acid sequence are alanine, tyrosine, arginine, asparagine, tyrosine, arginine, phenylalanine, tyrosine, glycine, and aspartic acid, respectively.

Preferably, the humanized monoclonal antibody according to the present invention includes:
a light chain variable region having the amino acid sequence of SEQ ID NO: 54, 55 or 56, or
an amino acid sequence identical by 95% or more to the amino acid sequence 54, 55 or 56; and
a heavy chain variable region having the amino acid sequence of SEQ ID NO: 57 or 58, or
an amino acid sequence identical by 95% or more to the amino acid sequence 57 or 58.

More preferably, the humanized monoclonal antibody includes:
1) a light chain variable region having the amino acid sequence of SEQ ID NO: 54, and
   a heavy chain variable region having the amino acid sequence of SEQ ID NO: 57;
2) a light chain variable region having the amino acid sequence of SEQ ID NO: 55, and
   a heavy chain variable region having the amino acid sequence of SEQ ID NO: 57;
3) a light chain variable region having the amino acid sequence of SEQ ID NO: 56, and
   a heavy chain variable region having the amino acid sequence of SEQ ID NO: 57;
4) a light chain variable region having the amino acid sequence of SEQ ID NO: 54, and
   a heavy chain variable region having the amino acid sequence of SEQ ID NO: 58;
5) a light chain variable region having the amino acid sequence of SEQ ID NO: 55, and
   a heavy chain variable region having the amino acid sequence of SEQ ID NO: 58;
6) a light chain variable region having the amino acid sequence of SEQ ID NO: 56, and
   a heavy chain variable region having the amino acid sequence of SEQ ID NO: 58,
   wherein one or more of the following substitutions are optionally present:
      A) substitution of serine with threonine at position 25 in the amino acid sequence of SEQ ID NO: 54, 55 or 56;
      B) substitution of serine with threonine at position 26 in the amino acid sequence of SEQ ID NO: 54, 55 or 56;
      C) substitution of serine with threonine at position 28 in the amino acid sequence of SEQ ID NO: 54, 55 or 56;
      D) substitution of glutamine with asparagine at position 95 in the amino acid sequence of SEQ ID NO: 54, 55 or 56;
      E) substitution of threonine with serine at position 31 in the amino acid sequence of SEQ ID NO: 57 or 58;
      F) substitution of alanine with valine at position 63 in the amino acid sequence of SEQ ID NO: 57 or 58;
      G) substitution of lysine with arginine at position 67 in the amino acid sequence of SEQ ID NO: 57 or 58;
      H) substitution of aspartic acid with glutamic acid at position 68 in the amino acid sequence of SEQ ID NO: 57 or 58;
      I) substitution of asparagine with glutamine at position 99 in the amino acid sequence of SEQ ID NO: 57 or 58; and
      J) substitution of tyrosine with phenylalanine at position 105 in the amino acid sequence of SEQ ID NO: 57 or 58.

Further preferably, the humanized monoclonal antibody includes:
the humanized monoclonal antibody of the present invention includes a light chain variable region having the amino acid sequence of SEQ ID NO: 56 and a heavy chain variable region having the amino acid sequence of SEQ ID NO: 57, wherein one or more of the following substitutions are optionally present:
A) substitution of serine with threonine at position 25 in the amino acid sequence of SEQ ID NO: 56;
B) substitution of serine with threonine at position 26 in the amino acid sequence of SEQ ID NO: 56;
C) substitution of serine with threonine at position 28 in the amino acid sequence of SEQ ID NO: 56;
D) substitution of glutamine with asparagine at position 95 in the amino acid sequence of SEQ ID NO: 56;
E) substitution of threonine with serine at position 31 in the amino acid sequence of SEQ ID NO: 57;
F) substitution of alanine with valine at position 63 in the amino acid sequence of SEQ ID NO: 57;
G) substitution of lysine with arginine at position 67 in the amino acid sequence of SEQ ID NO: 57;
H) substitution of aspartic acid with glutamic acid at position 68 in the amino acid sequence of SEQ ID NO: 57;
I) substitution of asparagine with glutamine at position 99 in the amino acid sequence of SEQ ID NO: 57; and
J) substitution of tyrosine with phenylalanine at position 105 in the amino acid sequence of SEQ ID NO: 57.

The humanized monoclonal antibody is most preferably a humanized monoclonal antibody including a light chain variable region having the amino acid sequence of SEQ ID NO: 56 and a heavy chain variable region having the amino acid sequence of SEQ ID NO: 57, wherein lysine at position 67 in the amino acid sequence of SEQ ID NO: 57 is substituted with arginine, that is, a humanized monoclonal antibody including a light chain variable region having the amino acid sequence of SEQ ID NO: 56 and a heavy chain variable region having the amino acid sequence of SEQ ID NO: 60.

It should be noted that the humanized monoclonal antibody according to the present invention uses a constant region of a human antibody. Preferred examples of the constant region of a human antibody include Cy, such as Cγ1, Cγ2, Cγ3, or Cγ4, as the heavy chain, and Cκ and Cλ, as the light chain. The C-region of the human antibody may be modified to improve stability of the antibody or its production. The human antibody used in producing the humanized antibody may be any isotype of human antibody, such as IgG, IgM, IgA, IgE or IgD, and, in the present invention, IgG is preferably used, and IgG1 or IgG4 is further preferably used.

In the humanized monoclonal antibodies according to the present invention, lysine may or may not be added to the C-terminal end of the heavy chain constant region. Preferably, the humanized monoclonal antibodies of the present invention have a light chain constant region of the amino acid sequence of SEQ ID NO: 52 and a heavy chain constant region of the amino acid sequence of SEQ ID NO: 53, wherein lysine may or may not be added to the C-terminal end of SEQ ID NO: 53.

The humanized monoclonal antibody can be made by general production methods (see, e.g., WO 95/14041, WO 96/02576). Specifically, at first, a DNA sequence encoding a variable region designed to link a CDR of a mouse antibody to a FR of a human antibody is synthesized by PCR method from several oligonucleotides which are made to have moieties overlapping the terminal ends (see, WO 98/13388). The obtained DNA is linked to a DNA encoding a constant region of a human antibody, and then incorporated into an expression vector. Alternatively, a DNA encoding a variable region of an antibody may be incorporated into an expression vector comprising a DNA of a constant region of an antibody. To produce an antibody used in the present invention, the antibody gene is incorporated into an expression vector such that it is expressed under the control of an expression control region, e.g., an enhancer/promoter. The expression vector can then be used to transform a host cell and express the antibody.

Examples of the host cell of the transformant include a vertebrate cell such as a COS cell or a CHO cell, a prokaryotic cell, and a yeast. The transformant can be cultured according to a method well known to those skilled in the art, and the monoclonal antibody of the present invention is produced intracellularly or extracellularly from the transformant. The medium used for the culture can be selected as appropriate depending on the adopted host cell from various types of media commonly used. When the host cell is a COS cell, examples of the medium include a medium such as RPMI-1640 medium or Dulbecco's Modified Eagle Minimum Essential Medium (DMEM) supplemented with serum components such as bovine fetal serum (FBS), as necessary. The culture temperature during culture of the transformant may be any temperature that does not significantly reduce protein synthesis capacity in the cell, and is preferably 32 to 42°C, most preferably 37°C. As necessary, the transformant can be cultured in an atmosphere containing 1 to 10% (v/v) of carbon dioxide.

Fractions comprising the monoclonal antibody according to the present invention produced intracellularly or extracellularly from the transformant as described above can be separated and purified by various known separation methods utilizing the physical and chemical properties or the like of the proteins. Specific examples of such methods include usual treatment with a protein precipitant, ultrafiltration, various chromatography such as molecular sieve chromatography (gel filtration), adsorption chromatography, ion exchange chromatography, affinity chromatography, or high-performance liquid chromatography (HPLC), a dialysis method, and a combination of these. With the methods, the monoclonal antibody according to the present invention can be readily produced in high yield and high purity.

The monoclonal antibody or an antibody fragment thereof according to the present invention may be further modified by various molecules such as polyethylene glycol (PEG), radioactive materials, toxins, and the like. As the method for modifying the antibody, known methods in the art can be used.

Furthermore, other proteins may be fused to the N- or C-terminal end of the monoclonal antibody or an antibody fragment thereof according to the present invention (Clinical Cancer Research, 2004, 10, 1274-1281). The protein to be fused can be appropriately selected by those skilled in the art.

The monoclonal antibody or an antibody fragment according to the present invention includes an antibody having an N-glycoside-linked glycochain attached to the Fc region of the antibody. Note that fucose may not be attached to N-acetylglucosamine at the reducing end of the N-glycoside-linked glycochain. Examples of the antibody in which an N-glycoside-linked glycochain attached to the Fc region of the antibody and no fucose is attached to N-acetylglucosamine at the reducing end of the N-glycoside-linked glycochain include an antibody produced using a CHO cell lacking an α1,6-fucose transferase gene (WO 2005/035586, WO 02/31140). The antibody of the present invention in which an N-glycoside-linked glycochain attached to the Fc region of the antibody and no fucose is attached to N-acetylglucosamine at the reducing end of the N-glycoside-linked glycochain has high ADCC activity.

From the viewpoint of removing Treg cells or macrophage cells, the anti-CCR8 antibody according to the present invention is preferably an antibody having antibody-dependent cell mediated cytotoxicity (ADCC) activity against cells expressing CCR8, or an antibody fragment thereof. ADCC activity means activity in vivo in which an antibody bound to a surface antigen of a cell, such as a target cell, activates an effector cell via a binding of the Fc region of the antibody to an Fc receptor present on the surface of the effector cell, and injures the target cell or the like. Examples of the effector cell include a natural killer cell and an activated macrophage. In the present invention, ADCC activity means activity in vivo in which an antibody bound to a surface antigen (CCR8) of a cell such as a Treg cell or macrophage cell, activates an effector cell via a binding of the Fc region of the antibody to an Fc receptor present on the surface of the effector cell, injures the Treg cell or macrophage cell or the like, and consequently injures a tumor cell or the like.

The anti-CCR8 antibody according to the present invention is preferably a neutralizing antibody or a neutralizing antibody fragment of CCR8. The neutralizing antibody or neutralizing antibody fragment of CCR8 means an antibody or antibody fragment having neutralizing activity against CCR8. Whether or not an antibody has neutralizing activity against CCR8 can be determined, for example, by measuring whether or not the antibody inhibits the physiological action of a CCR8 ligand (e.g., CCL1) against CCR8. Examples thereof include, but are not limited to, measuring the binding of CCL1 to CCR8, the migration or intracellular Ca²⁺ increase of CCR8-expressing cells by CCL1, or the expression variation of genes susceptible to CCL1 stimulation. It can also be measured by the method described in test examples below.

The neutralizing activity of the anti-CCR8 antibody according to the present invention for binding of CCR8 to CCL1 can be measured by addition of an antibody dilution diluted with a medium to human CCR8 expressing 293 cells in which a Ca²⁺ indicator is incorporated beforehand. The affinity of CCR8 to a CCR8 ligand is highest when the CCR8 ligand is CCL1, thus antibodies having high inhibitory activity against CCL1 are useful.

It is preferred that the neutralizing activity of the monoclonal antibody or an antibody fragment thereof according to the present invention is an IC50 value of 10 nM or less. More preferably, the neutralizing activity is IC50 value of 5 nM or less, even more preferably 2 nM or less, especially preferably 1 nM or less, most preferably 0.5 nM or less.

The anti-CCR8 antibody or an antibody fragment thereof according to the present invention is preferably an antibody that strongly recognizes CCR8, or an antibody fragment thereof. When selecting the antibody or an antibody fragment thereof that strongly recognizes CCR8, an antibody or an antibody fragment thereof that more strongly recognizes CCR8 can be selected by selecting the antibody or an antibody fragment thereof using the strength of neutralizing activity as index.

The anti-CCR8 antibody according to the present invention is preferably one having a tumor-infiltrating Treg cell removing action. Whether or not the monoclonal antibody according to the present invention has a tumor-infiltrating Treg cell removing action can be determined, for example, by the method described in Examples of Patent Document 2.

The anti-CCR8 antibody according to the present invention is preferably one having a tumor-infiltrating macrophage cell removing action. Whether or not the monoclonal antibody of the present invention has a tumor-infiltrating macrophage cell removing action can be determined, for example, by the method described in Examples of Patent Document 2.

The "chemotherapeutic agent" means an agent having an effect of suppressing proliferation of tumor cells.

The "immunotherapeutic agent" means an agent that injures tumor cells by activating immunity. The anti-CCR8 antibody according to the present invention is classified as an immunotherapeutic agent because the anti-CCR8 antibody binds to a cell surface antigen (CCR8) of an immunocompetent cell such as a macrophage, and activates an effector cell via binding pf the Fc region of the antibody to a Fc receptor present on the surface of the effector cell, so that the Treg cell, macrophage cell or the like is injured, resulting in injuring of a tumor cell or the like.

Examples of the chemotherapeutic agent according to the present invention include alkylating agents, platinum complexes, metabolic antagonists, topoisomerase inhibitors, taxane, and molecular target drugs.

The alkylating agent is an agent that exhibits an effect of suppressing proliferation of tumor cells when an alkyl group binds to DNA, and examples thereof include nitrogen mustards such as cyclophosphamide, ifosfamide, melphalan, busulfan and thiotepa, and nitrosoureas such as nimustine, ranimustine, dacarbazine, procarbazine, temozolomide, carmustine, streptozotocin, and bendamustine.

The platinum complex is an agent that has platinum in the structure of the agent and binds to DNA to induce apoptosis of tumor cells, and examples thereof include cisplatin, carboplatin, oxaliplatin, and nedaplatin. Cisplatin, carboplatin or oxaliplatin is preferable, and carboplatin is more preferable.

The metabolic antagonist is an agent that exhibits an effect of suppressing proliferation of tumor cells by inhibiting the uptake of purines and pyrimidines in the DNA synthesis process, and examples thereof include folic acid metabolic antagonists such as pemetrexed, sulfadiazine, sulfamethoxazole, methotrexate, trimethoprim and pyrimethamine; pyrimidine antimetabolites such as fluorouracil and flucytosine; purine metabolic antagonists such as 6-mercaptopurine, azathioprine and pentostatin; urea derivatives such as hydroxyurea; purine analogs such as thioguanine, fludarabine and cladribine; and pyrimidine analogs such as gemcitabine and cytarabine. Pemetrexed, fluorouracil or gemcitabine is preferable, and fluorouracil or gemcitabine is more preferable.

The topoisomerase inhibitor is an agent that exhibits an effect of suppressing proliferation of tumor cells by inhibiting topoisomerase which changes the helical structure of DNA, and examples thereof include type I topoisomerase inhibitors such as irinotecan and nogitecan, and type II topoisomerase inhibitors such as doxorubicin and etoposide. Irinotecan, doxorubicin or etoposide is preferable.

The taxane is an agent that inhibits depolymerization of microtubules which play an important role in cell division, and inhibits cell division to exhibit the effect of suppressing the proliferation of tumor cells, and examples thereof include paclitaxel and docetaxel.

The molecularly target drug is an agent that inhibits signal transduction of a specific molecule involved in proliferation of tumor cells, or the like, and examples thereof include tyrosine kinase inhibitors such as gefitinib, erlotinib, osimertinib, afatinib, dacomitinib, imatinib, dasatinib, bosutinib, vandetanib, sunitinib, axitinib, pazopanib, lenvatinib, lapatinib, nintedanib, nilotinib, ibrutinib, gilteritinib, crizotinib, ceritinib, alectinib and lorlatinib; Raf kinase inhibitors such as sorafenib, vemurafenib and dabrafenib; MEK inhibitors such as trametinib; cyclin-dependent kinase inhibitors such as valbociclib and abemaciclib; PARP inhibitors such as olaparib; and monoclonal antibodies such as cetuximab, trastuzumab, bevacizumab, pervacizumab, panitumumab and ramucirumab.

The chemotherapeutic agent according to the present invention is preferably a platinum complex, taxane, pemetrexed, gemcitabine, fluorouracil, irinotecan, etoposide or doxorubicin, more preferably a platinum complex, taxane, gemcitabine or fluorouracil, further preferably carboplatin, cisplatin, oxaliplatin, fluorouracil or gemcitabine, further preferably carboplatin, cisplatin, oxaliplatin or gemcitabine, further preferably carboplatin or gemcitabine.

The pharmaceutical composition or the medicament of the present invention is very useful as a medicament for treating and/or preventing a CCR8-related disease. In particular, it is very useful as a medicament for treating and/or preventing cancer in which intratumoral infiltration of CCR8-expressing Treg cells has occurred. For example, the pharmaceutical composition of the present invention is very useful as a medicament for treating and/or preventing cancer such as breast cancer, uterine corpus cancer, cervical cancer, ovary cancer, prostate cancer, lung cancer, stomach cancer (gastric adenocarcinoma), non-small cell lung cancer, pancreatic cancer, head and neck squamous cell cancer, esophageal cancer, bladder cancer, melanoma, colon cancer, kidney cancer, non-Hodgkin lymphoma, urothelial cancer, sarcoma, blood cell carcinoma (leukemia, lymphoma, etc.), bile duct carcinoma, gallbladder carcinoma, thyroid carcinoma, testicular cancer, thymic carcinoma, hepatocarcinoma, and the like, preferably breast cancer, lung cancer, colon cancer, kidney cancer, sarcoma, liver cancer, bladder cancer or ovary cancer, more preferably breast cancer, lung cancer, bladder cancer, kidney cancer or colon cancer, further preferably, preferably breast cancer, lung cancer, bladder cancer or colon cancer, further preferably breast cancer, lung cancer or colon cancer, further preferably breast cancer.

Here, the pharmaceutical composition or medicament of the present invention is also effective for treating and/or preventing cancer having low immunogenicity. For example, the pharmaceutical composition or medicament is also effective for treating and/or preventing cancer having a low tumor mutation burden (TMB) value. The TMB value is preferably 10 mutations/megabase or less, more preferably 5 mutations/megabase or less, further preferably 2 mutations/megabase or less, particularly preferably 1 mutation/megabese or less.

Grasselly et al. (Frontiers in Immunology, (2018), 9, Article 2100) indicates that in breast cancer-derived 4T1 cells used in Examples 1 and 2 herein, treatment with an anti-PD-1 antibody is ineffective. That is, the pharmaceutical composition or medicament of the present invention is also useful for treating cancer for which treatment with an anti-PD-1 antibody or an anti-PD-L1 antibody is ineffective. The phrase "treatment with an anti-PD-1 antibody or an anti-PD-L1 antibody is ineffective" means that cancer proliferation cannot be suppressed by administration of an anti-PD-1 antibody or an anti-PD-L1 antibody.

The "cancer" in "the use for cancer treatment" in the present invention includes all solid cancers and hematological cancers. Specific examples of the cancer include breast cancer, uterine corpus cancer, cervical cancer, ovary cancer, prostate cancer, lung cancer, stomach cancer (gastric adenocarcinoma), non-small cell lung cancer, pancreatic cancer, head and neck squamous cell cancer, esophageal cancer, bladder cancer, melanoma, colon cancer, kidney cancer, non-Hodgkin lymphoma, urothelial cancer, sarcoma, blood cell carcinoma (leukemia, lymphoma, etc.), bile duct carcinoma, gallbladder carcinoma, thyroid carcinoma, testicular cancer, thymic carcinoma, hepatocarcinoma. The cancer is preferably breast cancer, lung cancer, colon cancer, kidney cancer, sarcoma, liver cancer, bladder cancer or ovary cancer, more preferably breast cancer, lung cancer, bladder cancer, kidney cancer or colon cancer, further preferably, preferably breast cancer, lung cancer, bladder cancer or colon cancer, further preferably breast cancer, lung cancer or colon cancer, further preferably breast cancer.

The "cancer" in "the use for cancer treatment" in the present invention is preferably cancer that expresses a tumors-specific antigen.

Note that the "cancer" as described in the present description shall mean not only epithelial malignancies such as ovarian cancer, gastric cancer, and the like, but also non-epithelial malignancies including hematopoietic cancer such as chronic lymphocytic leukemia and Hodgkin lymphoma. Furthermore, in the present description, the terms such as "cancer," "carcinoma," "tumor," and "neoplasm" are not distinguishable from one another and used interchangeably.

In the "pharmaceutical composition which contains an anti-CCR8 antibody and is used with a chemotherapeutic agent", two or more chemotherapeutic agents according to the present invention may be used.

In the "pharmaceutical composition which contains a chemotherapeutic agent and is used with an anti-CCR8 antibody", or the "medicament obtained by combining an anti-CCR8 antibody and a chemotherapeutic agent" in the present invention, two or more chemotherapeutic agents according to the present invention may be used.

The combination of an anti-CCR8 antibody and a chemotherapeutic agent according to the present invention has an effect which results from the combination and is more marked than the effect of the anti-CCR8 antibody alone and the effect of the chemotherapeutic agent alone. Specifically, the combination of an anti-CCR8 antibody and a chemotherapeutic agent has a synergistic antitumor effect as compared to the antitumor effect of the anti-CCR8 antibody alone and the antitumor effect of the chemotherapeutic agent alone.

Since the combination of an anti-CCR8 antibody and a chemotherapeutic agent according to the present invention has an effect which results from the combination and is more marked than the effect of the anti-CCR8 antibody alone and the effect of the chemotherapeutic agent alone, the dosage of the anti-CCR8 antibody and the chemotherapeutic agent for exhibiting an antitumor effect is smaller than the dosage of each of the anti-CCR8 antibody and the chemotherapeutic agent administered alone. Therefore, the combination of an anti-CCR8 antibody and a chemotherapeutic agent according to the present invention can be expected to have an effect of reducing side effects of the anti-CCR8 antibody and/or the chemotherapeutic agent.

The pharmaceutical composition or medicament of the present invention can be administered orally or parenterally and systemically or locally. Examples of the parenteral administration include intravenous injection, such as infusion, intramuscular injection, intraperitoneal injection, subcutaneous injection, intranasal administration, and inhalation.

The "medicament comprising a combination of an anti-CCR8 antibody and a chemotherapeutic agent" in the present invention may be a combination agent of a pharmaceutical composition containing an anti-CCR8 antibody and a chemotherapeutic agent, or may be a compounding agent of an anti-CCR8 antibody and a chemotherapeutic agent.

The "compounding agent" means one in which two or more active ingredients are blended in one preparation. For example, in a compounding agent of an anti-CCR8 antibody and a chemotherapeutic agent, the anti-CCR8 antibody and the chemotherapeutic agent are contained in one preparation.

When the "medicament comprising a combination of an anti-CCR8 antibody and a chemotherapeutic agent" in the present invention is administered as a combination agent where a pharmaceutical composition containing the anti-CCR8 antibody and a pharmaceutical composition containing the chemotherapeutic agent are administered as separate preparations, the administration includes simultaneous administration and administration at different times. In the administration at different times, the administration of the pharmaceutical composition containing the chemotherapeutic agent may precede the administration of the pharmaceutical composition containing the anti-CCR8 antibody, or the administration of the pharmaceutical composition containing the anti-CCR8 antibody may precede the administration of the pharmaceutical composition containing the chemotherapeutic agent, and the administration methods for the former and the latter may be the same or different.

The value of a ratio of the anti-CCR8 antibody to the chemotherapeutic agent in the combination agent can be arbitrary. The weight ratio of the anti-CCR8 antibody and the chemotherapeutic agent in the combination agent is, for example, 1000 : 1 to 1 : 1000, preferably 100 : 1 to 1 : 100, more preferably 10 : 1 to 1 : 10, further preferably 5 : 1 to 1 : 5.

The "medicament comprising a combination of an anti-CCR8 antibody and a chemotherapeutic agent" in the present invention may be a combination agent of a pharmaceutical composition containing an anti-CCR8 antibody and a chemotherapeutic agent, or may be a compounding agent of an anti-CCR8 antibody and a chemotherapeutic agent. The ratio of the anti-CCR8 antibody and the chemotherapeutic agent in the compounding agent is, for example, 1000 : 1 to 1 : 1000, preferably 100 : 1 to 1 : 100, more preferably 10 : 1 to 1 : 10, further preferably 5 : 1 to 1 : 5.

If necessary, the pharmaceutical composition or compounding agent according to the present invention can be mixed with various pharmaceutical additives suitable for the dosage form thereof, such as excipients, binders, disintegrants, and lubricants.

The therapeutically effective amount of the pharmaceutical composition or compounding agent according to the present invention is an amount which ensures that the symptoms of the indicated disease are suppressed as compared to those in an individual who is not dosed. The specific effective amount is not fixed, and is appropriately set depending on the dosage form, the administration method, the intended use, and the age, weight, symptom and the like of the individual.

### [EXAMPLES]

Hereinafter, the present invention will be explained in more detail by way of Examples of the present invention, but the present invention is not limited by them.

### Test Example 1: Production of anti-human CCR8 antibody-producing mouse hybridoma

The gene encoding the full length of human CCR8 (UniProtKB/Swiss-Prot: P51685, SEQ ID NO: 1) was used as an antigen and DNA-immunized to A/J Jms Sic female mice. DNA immunization was repeated two or three times at two-week intervals and boosted by intraperitoneally administering human CCR8-expressing Expi293 cells one week after final immunization. After three days, spleen was removed, and splenocytes and mouse myeloma cells (p3x6363-Ag8., Tokyo Oncology Institute) were fused by the PEG method, and selection was made in a medium containing hypoxanthine, aminopterin and thymidine. With the obtained culture supernatant, anti-human CCR8 antibodies and anti-human CCR8 neutralizing antibodies were selected by the following method.

For anti-human CCR8 antibodies, the culture supernatant was reacted with human CCR8-expressing Expi293 cells and human CCR4-expressing Expi293 cells, respectively, and clones that specifically bind only to human CCR8 were selected by detecting with Alexa488-labeled anti-mouse IgG antibodies (manufactured by Thermo Fisher Scientific).

For anti-human CCR8 neutralizing antibodies, the culture supernatant was sufficiently reacted with human CCR8 expressing 293 cells in which a Ca²⁺ indicator was incorporated beforehand, then Ca²⁺ influx by addition of 200 nM hCCL1 (manufactured by BioLegend) was measured with FLIPR, and clones that inhibit Ca²⁺ influx by hCCL1 stimulation were selected.

Clones that exhibited particularly strong neutralizing activity (% inhibition > 80%) and clones that had no neutralizing activity were respectively cloned to establish hybridomas.

### Test Example 2: Production of anti-human CCR8 antibody-producing rat hybridoma

The immunogen, a human CCR8-expressing Rat-1 cell, was produced by transfecting Rat-1 cells with an expression vector in which the human CCR8 gene was cloned into pQCXIP (Clontech Laboratories, Inc.), then subjecting the cells to drug selection with puromycin (1 ug/ml) for one month.

The human CCR8-expressing Rat-1 cells were used to immunize rats once. About two weeks later, the lymph nodes were collected and hybridomas were produced by a routine method.

For anti-human CCR8 antibodies, the culture supernatant of the hybridomas was reacted with human CCR8-expressing 293 cells and 293 cells, respectively, and clones that are capable of specifically binding to human CCR8 were selected by detecting with Alexa647-labeled anti-rat IgG antibodies (manufactured by Thermo Fisher Scientific).

### Test Example 3: Epitope analysis of anti-human CCR8 neutralizing and non-neutralizing antibodies

The culture supernatants of the anti-human CCR8 antibody producing hybridoma established in Test Example 1 cultured in serum-free medium was subjected to Protein G purification and gel filtration purification to obtain purified antibodies of 40 clones. Of the obtained 40 clones, purified antibodies of 27 neutralizing antibodies and 13 non-neutralizing antibodies were used to perform an antigen-binding test by the method described below to identify epitopes important for neutralizing activity. For the neutralizing activity of each clone, inhibitory activity against Ca²⁺ influx by 100 nM hCCL1 (manufactured by BioLegend) addition was measured with FLIPR by the method described in Test Example 1. Each neutralizing antibody had an IC50 value of 2 nM or less of Ca²⁺ influx inhibitory activity by 100 nM hCCL1 stimulation.

The gene encoding the full length of human CCR8 (UniProtKB/Swiss-Prot: P51685, SEQ ID NO: 1), the gene encoding the full length of human CCR4 (UniProtKB/Swiss-Prot: P51679, SEQ ID NO: 48) and the gene encoding the full length of mouse CCR8 (UniProtKB/Swiss-Prot: P56484, SEQ ID NO: 39) were cloned into pcDNA 3.4 vectors, respectively. These vectors were used for transfection to produce Expi293 cells transiently expressing human CCR8, human CCR4, or mouse CCR8. These cells were reacted with a dilution series of the purified antibodies of each clone, and the binding properties thereof were evaluated by detection with Alexa488-labeled anti-mouse IgG antibody (manufactured by Thermo Fisher Scientific). As a result, every antibody bound to human CCR8, but not to human CCR4 and mouse CCR8 at all.

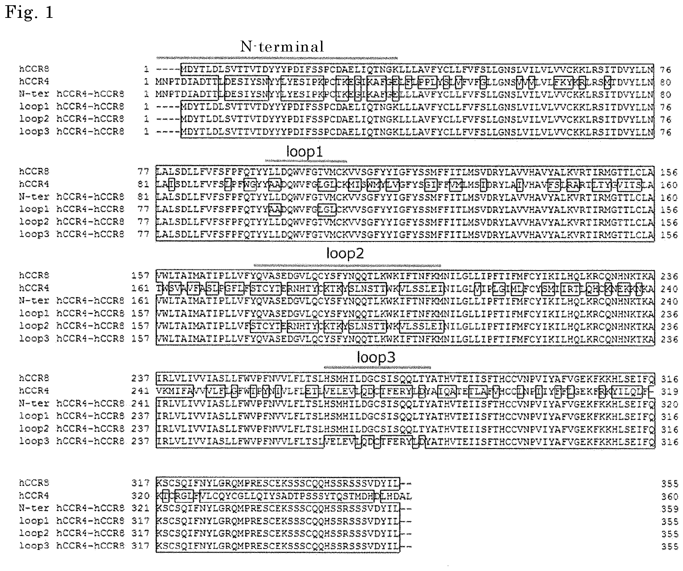

Then, chimeras (Fig. 1) in which the extracellular domains of human CCR8, the N-terminal region (amino acids 1-35 of SEQ ID NO: 1), the loop1 region (amino acids 94-107 of SEQ ID NO: 1), the loop2 region (amino acids 172-202 of SEQ ID NO: 1) and the loop3 region (amino acids 264-280 of SEQ ID NO: 1) are substituted with the corresponding regions of human CCR4, the N-terminal region (amino acids 1-39 of SEQ ID NO: 48), the loop1 region (amino acids 98-111 of SEQ ID NO: 48), the loop2 region (amino acids 176-206 of SEQ ID NO: 48) and the loop3 region (amino acids 268-284 of SEQ ID NO: 48), respectively, were produced, and binding of each antibody was evaluated by a method similar to that described above.

Binding at 5 ug/mL, 0.5 ug/mL, and 0.05 ug/mL was evaluated, and, in comparison to wild-type human CCR8 (hCCR8), an antibody having significantly reduced binding activity was indicated as Δ and an antibody having binding activity of the detection limit or less was indicated as ×. The blank column means that it exhibits the same binding activity as the wild-type human CCR8 (hCCR8).

As a result, as shown in Table 2, all antibodies having neutralizing activity had the binding activity of the detection limit or less by substituting the N-terminal region with human CCR4 (N-ter hCCR4-hCCR8), thus it was revealed that the N-terminal region is an important epitope for exerting neutralizing activity. Further, the binding activities to loop1-human CCR4-substituted human CCR8 and loop2-human CCR4-substituted human CCR8 were also reduced. From the above, it is considered that anti-CCR8 antibodies having strong neutralizing activity have stereo-structural recognition to strongly recognize the N-terminal region and bind to loop1 and loop2.

Note that although an antibody that does not recognize any of human CCR8, human CCR4, and mouse CCR8 was used as a control, the control antibody did not bind to each antigen. It was also confirmed that expression of hCCR8 has no reduction due to mutation by attaching a tag to the N-terminal of each antigen and detecting it with an anti-tag antibody.

Further, to analyze in more detail the N-terminal region that is an epitope region common to all clones having strong neutralization, the human CCR8 point mutants shown in Table 2 were produced. As a result of evaluating the binding activity of each clone to the mutants by a similar method to the method described above, all 27 neutralizing antibodies described above had significantly reduction to the detection limit or less in their binding activities to the mutant in which Y at position 17 of human CCR8 is substituted for A (hCCR8 (Y17A)). It is thus considered that the neutralizing antibodies recognize Y at position 17. In addition, although clone No. 8F7 is only shown in Table 2, plural other neutralizing antibodies had reduced binding activity to the mutant in which I at position 20 of human CCR8 is substituted with A (hCCR8 (I20A)). It is thus considered that some neutralizing antibodies have stereo-structural recognition to bind to isoleucine at position 20.

Meanwhile, 13 non-neutralizing antibodies described above did not reduce their binding activities to hCCR8 (Y17A), thus it is considered that they recognize the same N-terminal region, but not Y at position 17. From the above, it is considered that Y at position 17 is an amino acid that is extremely important for exerting neutralizing activity. The results for representative neutralizing antibodies (clone Nos.10A11, 27G1, 1H4, 8F7, 2C7) and non-neutralizing antibodies (clone No. 5B5) are shown in Table 2.

**[Table 2]**

| clone No. | 10A11 | 27G1 | 1H4 | 8F7 | 2C7 | 5B5* |
|---|---|---|---|---|---|---|
| Neutralizing activity IC50 (nM) | 0.08 | 0.09 | 0.32 | 1.56 | 0.40 | > 1000 |
| hCCR8 | | | | | | |
| hCCR4 | × | × | × | × | × | × |
| mCCR8 | × | × | × | × | × | × |
| N-ter hCCR4-hCCR8 | × | × | × | × | × | Δ |
| loop1 hCCR4-hCCR8 | Δ | Δ | Δ | × | × | |
| loop2 hCCR4-hCCR8 | Δ | Δ | Δ | Δ | Δ | |
| loop3 hCCR4-hCCR8 | | | | | | |
| hCCR8 (L5A) | | | Δ | | Δ | |
| hCCR8 (D6A) | | | | | | |
| hCCR8 (L7A) | | | | | | |
| hCCR8 (S8A) | | | | | | |
| hCCR8 (T10A) | | | | | Δ | |
| hCCR8 (T11A) | | | Δ | | | |
| hCCR8 (V12A) | | | | | | |
| hCCR8 (T13A) | | | | | | |
| hCCR8 (Y15A) | | | | | | |
| hCCR8 (Y16A) | | | | | | |
| hCCR8 (Y17A) | × | × | × | × | × | |
| hCCR8 (I20A) | | | | Δ | | |
| hCCR8 (S22A) | | | | | Δ | |
| hCCR8 (S23A) | | | | | | |
| hCCR8 (L29A) | | | | | | |
| hCCR8 (I30A) | | | | Δ | | |
| hCCR8 (Q31A) | | | | | | |
| hCCR8 (T32A) | | | | | | |
| hCCR8 (N33A) | | | | | | |
| hCCR8 (G34A) | | | | | | |
| hCCR8 (K35A) | | | × | Δ | | |

| | | | | | | |
|---|---|---|---|---|---|---|
| *: Non-neutralizing antibody | | | | | | |

### Test Example 4: Determination of antibody sequences

Of the established clones, for the mouse antibody shown in Table 3 and the rat antibody shown in Table 4, the amino acid sequences of the light and heavy chain variable regions of the antibodies were determined with the hybridoma cells according to a routine method.

**[Table 3]**

| mAb | | SEQ ID NO: | | SEQ ID NO: | | SEQ ID NO: | | SEQ ID NO: |
|---|---|---|---|---|---|---|---|---|
| 10A11 | Light chain | 26 | CDR1 | 2 | Heavy chain | 27 | CDR1 | 5 |
| | | | CDR2 | 3 | | | CDR2 | 6 |
| | | | CDR3 | 4 | | | CDR3 | 7 |
| 27G1 | Light chain | 26 | CDR1 | 2 | Heavy chain | 28 | CDR1 | 8 |
| | | | CDR2 | 3 | | | CDR2 | 6 |
| | | | CDR3 | 4 | | | CDR3 | 7 |
| 1H4 | Light chain | 29 | CDR1 | 2 | Heavy chain | 30 | CDR1 | 8 |
| | | | CDR2 | 3 | | | CDR2 | 6 |
| | | | CDR3 | 4 | | | CDR3 | 7 |
| 19D7 | Light chain | 31 | CDR1 | 2 | Heavy chain | 32 | CDR1 | 8 |
| | | | CDR2 | 9 | | | CDR2 | 6 |
| | | | CDR3 | 10 | | | CDR3 | 11 |
| 8F7 | Light chain | 33 | CDR1 | 2 | Heavy chain | 34 | CDR1 | 12 |
| | | | CDR2 | 3 | | | CDR2 | 6 |
| | | | CDR3 | 10 | | | CDR3 | 13 |
| 2C7 | Light chain | 35 | CDR1 | 14 | Heavy chain | 36 | CDR1 | 17 |
| | | | CDR2 | 15 | | | CDR2 | 18 |
| | | | CDR3 | 16 | | | CDR3 | 19 |

**[Table 4]**

| mAb | | SEQ ID NO: | | SEQ ID NO: | | SEQ ID NO: | | SEQ ID NO: |
|---|---|---|---|---|---|---|---|---|
| 2-7B | Light chain | 37 | CDR1 | 20 | Heavy chain | 38 | CDR1 | 23 |
| | | | CDR2 | 21 | | | CDR2 | 24 |
| | | | CDR3 | 22 | | | CDR3 | 25 |

### Test Example 5: Alignment of Antibody Sequences

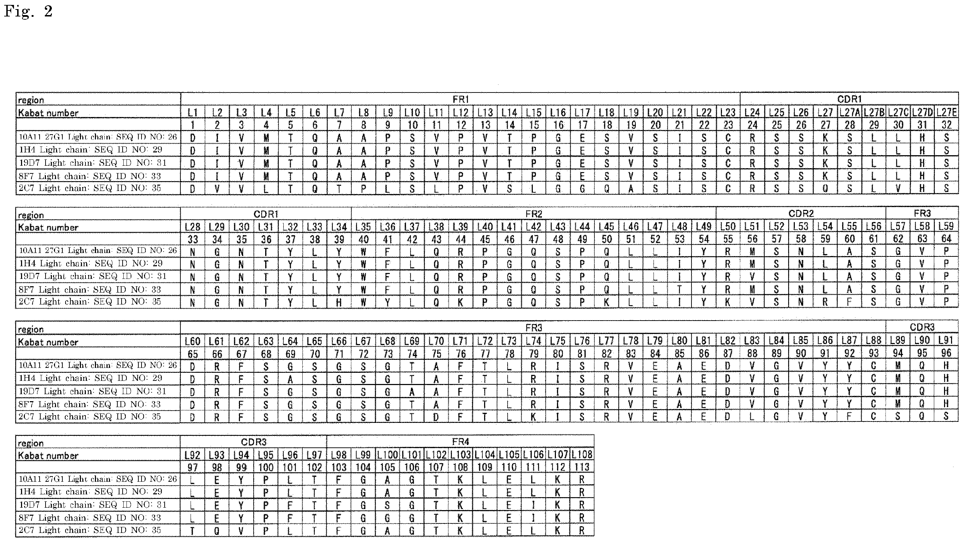

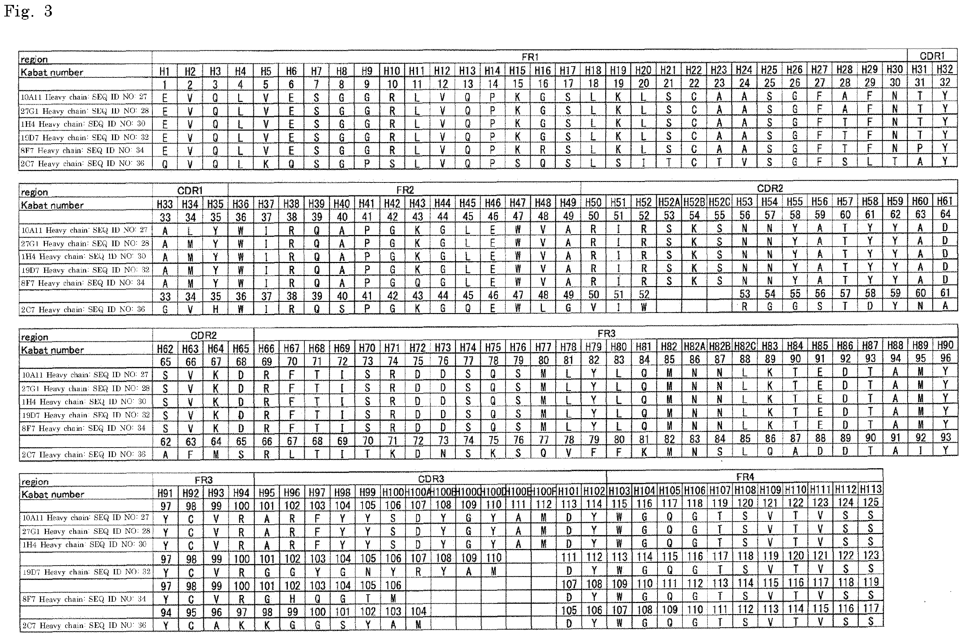

The amino acid sequences of the light and heavy chain of anti-human CCR8 neutralizing antibodies from mouse hybridoma cells described in Test Example 4 were aligned to Kabat numbering with an antibody sequence analysis software abYsis (Figs. 2 and 3). As a result, the amino acid sequences of 10A11, 27G1, 1H4, 19D7, and 8F7 comprised sequences similar to CDRs as described below.
CDR1 of the light chain consisted of 16 amino acids of SEQ ID NO: 2.
CDR2 of the light chain consisted of 7 amino acids of R-Xaa1-S-N-L-A-S (wherein Xaal is M or V: SEQ ID NO: 49) (SEQ ID NO: 3 or 9).
CDR3 of the light chain consisted of 9 amino acids of M-Q-H-L-E-Y-P-Xaa1-T (wherein Xaal is L or F: SEQ ID NO: 50) (SEQ ID NO: 4 or 10).
CDR1 of the heavy chain consisted of 5 amino acids of Xaa1-Y-A-Xaa2-Y (wherein Xaal is T or P and Xaa2 is L or M: SEQ ID NO: 51) (SEQ ID NO: 5, 8 or 12).
CDR2 of the heavy chain consisted of 19 amino acids of SEQ ID NO: 6.
CDR3 of the heavy chain, which were common to 10A11, 27G1, 1H4, consisted of 14 amino acids of SEQ ID NO: 7.

### Test Example 6: Evaluation of Neutralizing Activity

The established hybridomas were cultured in serum-free medium and the culture supernatant was subjected to Protein G affinity purification and gel filtration purification to give a purified antibody. Neutralizing activity of the purified antibody was measured by the method described below.

The antibody dilution diluted with a medium was added to human CCR8 expressing 293 cells in which a Ca²⁺ indicator was incorporated beforehand, and Ca²⁺ influx by addition of 200 nM hCCL1 (manufactured by BioLegend) was measured with FLIPR. The inhibition rate was calculated by setting the signal when hCCL1 was not added as the inhibition rate of 100% and the signal when hCCL1 was added and the antibody was not added as the inhibition rate of 0%, and the antibody concentration showing the inhibition rate of 50% was taken as IC50. The evaluation was performed at least three times, and IC50 was expressed as average ± SD. (Table 5)

**[Table 5]**

| Clone | IC50 (nM) |
|---|---|
| 10A11 | 0.23 ± 0.10 |
| 27G1 | 0.27 ± 0.06 |
| 1H4 | 0.36 ± 0.10 |
| 19D7 | 0.18 ± 0.06 |
| 8F7 | 3.03 ± 0.91 |
| 2C7 | 1.19 ± 0.64 |
| 2-7B | 0.23 ± 0.10 |

### Test Example 7: Humanization of Antibodies (10A11, 2C7)

Humanization was performed for 10A11, 2C7 by the methods described below.

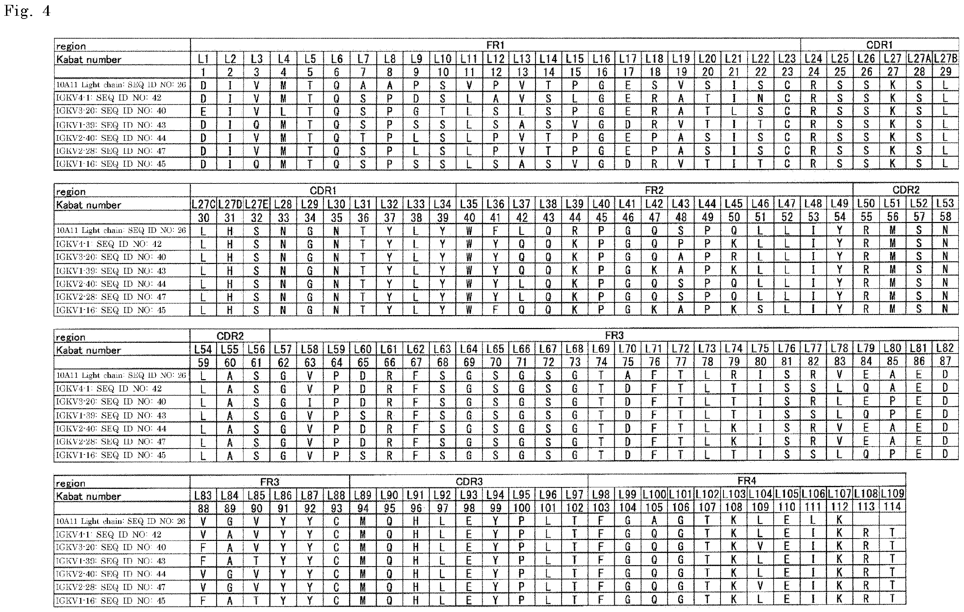

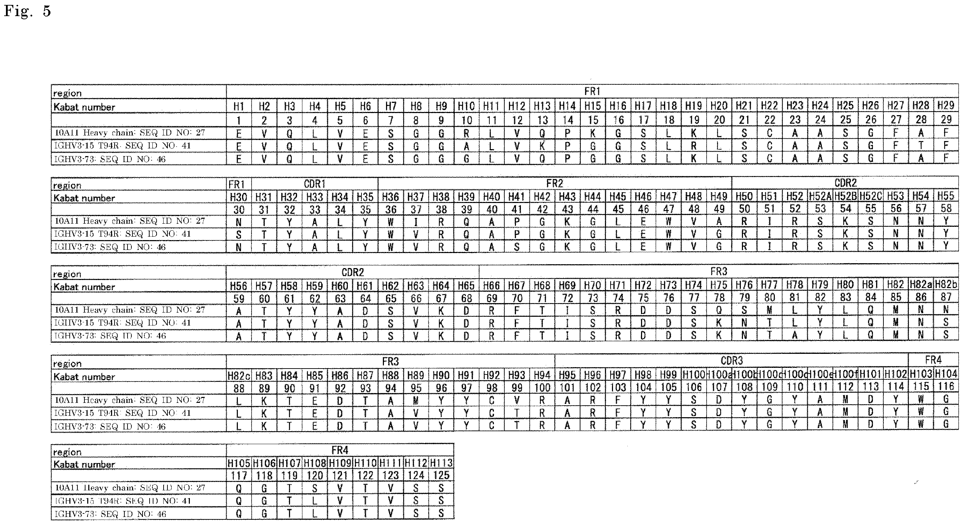

The definition of Kabat numbering and CDR was made with the antibody sequence analysis software abYsis. Human reproductive system acceptor sequences analogous to the V gene region sequences of the heavy and light chains of the mouse antibody amino acid sequences were searched and selected with the sequence analysis software Absis. For the J-chain region, sequences that are highly homologous to mouse antibody DNA sequences were searched with IMGT (http://www.imgt.org/) and used as the human framework sequences. To this human framework sequences, mouse antibody heavy chains CDR1, CDR2, CDR3 and mouse antibody light chains CDR1, CDR2, CDR3 as defined by Kabat Numbering (Wu, T. T. and Kabat, E.A., J Exp. Med. Aug 1; 132 (2): 211-50.(1970)) were implanted to design a humanized monoclonal antibody sequence (light chain; Fig. 4, heavy chain; Fig. 5). The neutralizing activity was measured by the method described in Test Example 6, resulting that the humanized monoclonal antibody shown in Table 6 exhibited affinity equal to or greater than the mouse antibody.

**[Table 6]**

| mAb | Light chain SEQ ID NO: | Heavy chain SEQ ID NO: | IC50 (nM) |
|---|---|---|---|
| h10A11 (IGKV3-20/IGHV3-15 T94R) | 40 | 41 | 0.16 ± 0.08 |
| h10A11 (IGKV4-1/IGHV3-15 T94R) | 42 | 41 | 0.15 ± 0.10 |
| h10A11 (IGKV1-39/IGHV3-15 T94R) | 43 | 41 | 0.20 ± 0.08 |
| h10A11 (IGKV2-40/IGHV3-15 T94R) | 44 | 41 | 0.30 ± 0.12 |
| h10A11 (IGKV1-16/IGHV3-15 T94R) | 45 | 41 | 0.70 ± 0.36 |
| h10A11 (IGKV3-20/IGHV3-73) | 40 | 46 | 0.22 ± 0.07 |
| h10A11 (IGKV4-1/IGHV3-73) | 42 | 46 | 0.17 ± 0.09 |
| h10A11 (IGKV1-39/IGHV3-73) | 43 | 46 | 0.16 ± 0.08 |
| h10A11 (IGKV2-40/IGHV3-73) | 44 | 46 | 0.21 ± 0.06 |
| h10A11 (IGKV2-28/IGHV3-73) | 47 | 46 | 0.20 ± 0.05 |
| h10A11 (IGKV1-16/IGHV3-73) | 45 | 46 | 0.31 ± 0.08 |
| m10A11 | 26 | 27 | 0.23 ± 0.10 |

### Test Example 7-2: Humanization of antibody (19D7)

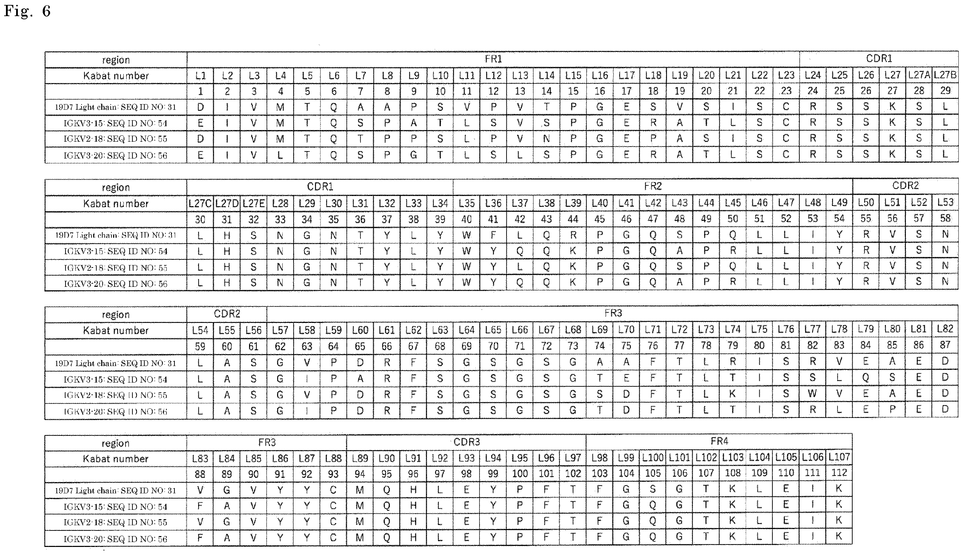

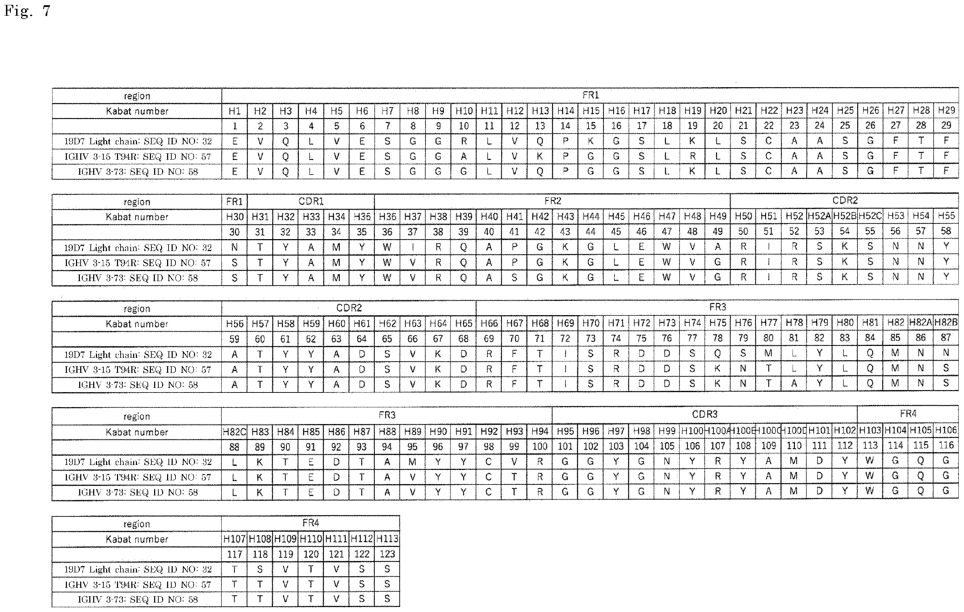

For 19D7, humanization was performed in a similar manner to Test Example 7 (light chain; Fig. 6, heavy chain; Fig. 7). The neutralizing activity was measured by the method described in Test Example 6, resulting that the humanized monoclonal antibody shown in Table 7 exhibited affinity equal to or greater than the mouse antibody.

**[Table 7]**

| mAb | Light chain SEQ ID NO: | Heavy chain SEQ ID NO: | IC50 (nM) |
|---|---|---|---|
| h19D7 (IGKV3-15/IGHV3-15 T94R) | 54 | 57 | 0.18 ± 0.04 |
| h19D7 (IGKV2-18/IGHV3-15 T94R) | 55 | 57 | 0.27 ± 0.05 |
| h19D7 (IGKV3-20/IGHV3-15 T94R) | 56 | 57 | 0.24 ± 0.04 |
| h19D7 (IGKV3-15/IGHV3-73) | 54 | 58 | 0.27 ± 0.06 |
| h19D7 (IGKV2-18/IGHV3-73) | 55 | 58 | 0.46 ± 0.12 |
| h19D7 (IGKV3-20/IGHV3-73) | 56 | 58 | 0.31 ± 0.04 |
| m19D7 | 31 | 32 | 0.18 ± 0.06 |

### Test Example 8: Identification of amino acids important for activity of humanized 10A11

For humanized 10A11 shown in Figs. 4 and 5, mutants in which point mutations were introduced into amino acids corresponding to CDRs were produced, and the neutralizing activity of each mutant was calculated by the method described in Test Example 6. Evaluation of neutralizing activity was performed multiple times for each mutant, and the IC50 value was expressed by IC50 ratio (WT IC50/mutant IC50) which is the ratio to the IC50 value of WT.

The results are shown in Tables 8 and 9. The n.d.(= not detectable) indicates that the IC50 value of the mutant is 10 nM or more, the limit value of the measurement system, and the activity is reduced to the detection limit or less. As a result of the neutralizing activity evaluation, for light chains, the activities of each mutant of S26T, K27R, L27cI and E93D having mutations at L26, L27, L27c, and L93 amino acid positions by Kabat Numbering, respectively, were reduced by 10-fold or more (Table 8). For heavy chains, the activities of each mutant of A33V, Y35F, R52K, N53Q, Y59F, R96K, F97L, Y98F, G100cA, D101E having mutations at the H33, H35, H52, H53, H59, H96, H97, H98, H100c, H101 amino acid position by Kabat Numbering, respectively, were reduced by 10-fold or more (Table 9). These amino acids are extremely important for the activity.

**[Table 8]**

| Light chain frame | Heavy chain frame | Light chain CDR mutation | IC50 ratio |
|---|---|---|---|
| IGKV4-1 mutant | IGHV3-15 T94R | R24K | 0.16 |
| | | S26T | 0.05 |
| | | K27R | n.d. |
| | | L27cI | 0.05 |
| | | H27dR | 0.18 |
| | | N28Q | 0.79 |
| | | G29A | 0.18 |
| | | N30Q | 0.19 |
| | | T31S | 0.50 |
| | | L33I | 0.70 |
| | | Y34F | 0.25 |
| | | M51L | 0.24 |
| | | N53Q | 2.10 |
| | | L54I | 1.18 |
| | | M89L | 0.49 |
| | | H91R | 0.15 |
| | | L92I | 1.27 |
| | | E93D | n.d. |
| | | Y94F | 0.87 |
| | | P95G | 0.20 |
| | | L96I | 0.56 |
| | | wt | 1.00 |

| | | | |
|---|---|---|---|
| * The amino acid position is the position by Kabat Numbering. | | | |

**[Table 9]**

| Light chain frame | Heavy chain frame | Heavy chain CDR mutation | IC50 ratio |
|---|---|---|---|
| IGKV4-1 | IGHV3-73 mutant | T31A | 0.17 |
| | | Y32F | 0.44 |
| | | A33V | n.d. |
| | | L34I | 0.43 |
| | | Y35F | n.d. |
| | | R50K | 0.12 |
| | | I51L | 0.22 |
| | | R52K | n.d. |
| | | S52aT | 0.22 |
| | | K52bR | 0.47 |
| | | S52cT | 0.53 |
| | | N53Q | 0.03 |
| | | Y55F | 0.47 |
| | | A56V | 0.55 |
| | | T57S | 0.49 |
| | | Y58F | 0.50 |
| | | Y59F | 0.03 |
| | | A60V | 0.67 |
| | | D61E | 0.25 |
| | | S62T | 1.60 |
| | | V63L | 0.56 |
| | | K64R | 0.54 |
| | | D65E | 1.30 |
| | | R96K | n.d. |
| | | F97L | n.d. |
| | | Y98F | 0.07 |
| | | Y99F | 0.80 |
| | | S100T | 0.64 |
| | | D100aE | 0.30 |
| | | Y100bF | 0.75 |
| | | G100cA | 0.07 |
| | | Y100dF | 0.55 |
| | | A100eV | 0.49 |
| | | M100fL | 0.72 |
| | | D101E | n.d. |
| | | Y102F | 0.99 |
| | | wt | 1.00 |

| | | | |
|---|---|---|---|
| * The amino acid position is the position by Kabat Numbering. | | | |

### Test Example 8-2: Identification of amino acids important for activity of humanized 19D7

For humanized 19D7 shown in Figs. 6 and 7, mutants in which point mutations were introduced into amino acids corresponding to CDRs were produced, and the neutralizing activity of each mutant was calculated by the method described in Test Example 6. Evaluation of neutralizing activity was performed multiple times for each mutant, and the IC50 value was expressed by IC50 ratio (WT IC50/mutant IC50) which is the ratio to the IC50 value of WT.

The results are shown in Tables 10 and 11. The n.d.(= not detectable) indicates that the IC50 value of the mutant is 10 nM or more, the limit value of the measurement system, and the activity is reduced to the detection limit or less.

**[Table 10]**

| Light chain frame | Heavy chain frame | Light chain CDR mutation | IC50 ratio |
|---|---|---|---|
| IGKV3-20 mutant | IGHV3-15 T94R | R24K | 0.94 |
| | | S25T | 0.63 |
| | | S26T | 0.79 |
| | | K27R | 0.77 |
| | | S27aT | 1.01 |
| | | L27bI | 0.26 |
| | | L27cI | 0.68 |
| | | H27dR | n.d. |
| | | S27eT | 0.58 |
| | | N28Q | 0.02 |
| | | G29A | 0.75 |
| | | N30Q | 0.64 |
| | | T31S | 0.61 |
| | | Y32F | 0.33 |
| | | L33I | 0.20 |
| | | Y34F | 0.78 |
| | | R50K | 0.52 |
| | | V51L | 0.67 |
| | | S52T | 0.60 |
| | | N53Q | 0.49 |
| | | L54I | 0.85 |
| | | A55V | 0.66 |
| | | S56T | 0.69 |
| | | M89L | 0.85 |
| | | Q90N | 1.01 |
| | | H91R | 0.05 |
| | | L92I | 0.43 |
| | | E93D | 0.58 |
| | | Y94F | 0.69 |
| | | P95G | 0.34 |
| | | F96L | 0.10 |
| | | T97S | 0.53 |
| | | N28A | 0.05 |
| | | N28E | n.d. |
| | | N28F | n.d. |
| | | N28G | n.d. |
| | | N28I | n.d. |
| | | N28K | n.d. |
| | | N28L | n.d. |
| | | N28P | n.d. |
| | | N28R | n.d. |
| | | N28S | n.d. |
| | | N28T | n.d. |
| | | N28Y | n.d. |
| | | N28V | 0.14 |

| | | | |
|---|---|---|---|
| * The amino acid position is the position by Kabat Numbering. | | | |

**[Table 11]**

| Light chain frame | Heavy chain frame | Heavy chain CDR mutation | IC50 ratio |
|---|---|---|---|
| IGKV3-20 | IGHV3-15 T94R mutant | T31S | 1.22 |
| | | Y32F | 0.77 |
| | | A33V | 0.32 |
| | | M34L | 0.64 |
| | | Y35F | n.d. |
| | | R50K | n.d. |
| | | I51L | 0.79 |
| | | R52K | n.d. |
| | | S52aT | 0.58 |
| | | K52bR | 0.69 |
| | | S52cT | 0.25 |
| | | N53Q | n.d. |
| | | N54Q | 0.48 |
| | | Y55F | 0.98 |
| | | A56V | 0.60 |
| | | T57S | 0.61 |
| | | Y58F | 0.67 |
| | | Y59F | 0.50 |
| | | A60V | 0.74 |
| | | D61E | 0.65 |
| | | S62T | 0.74 |
| | | V63L | 0.73 |
| | | K64R | 1.45 |
| | | D65E | 0.88 |
| | | G95A | 0.53 |
| | | G96A | 0.02 |
| | | Y97F | 0.80 |
| | | G98A | 0.73 |
| | | N99Q | 0.85 |
| | | Y100F | 0.60 |
| | | R100aK | 0.11 |
| | | Y700bF | n.d. |
| | | A700cV | n.d. |
| | | M100dL | 0.60 |
| | | D101E | 0.19 |
| | | Y102F | 0.62 |

| | | | |
|---|---|---|---|
| * The amino acid position is the position by Kabat Numbering. | | | |

### Test Example 9: Enhancement of activity of humanized 10A11

Optimization of the humanized 10A11 was performed by combining the activity-enhancing mutations found in Test Example 8 and mutations of N at L28 and G at L29 in amino acid position by Kabat numbering, corresponding to the sequence for deamidation risk, with the humanized framework found in Test Example 7. As a result, the humanized 10A11 mutant shown in Table 12 has been found.

**[Table 12]**

| Light chain variable region | | Heavy chain variable region | IC50 (nM) |
|---|---|---|---|
| Frame | Mutation | Frame | |
| IGKV4-1 | N53Q | IGHV3-15 T94R | 0.12 ± 0.02 |
| IGKV4-1 | N53Q, N28K | IGHV3-15 T94R | 0.29 ± 0.04 |
| IGKV4-1 | N53Q, G29L | IGHV3-15 T94R | 0.18 ± 0.02 |
| IGKV4-1 | N53Q, G29R | IGHV3-15 T94R | 0.12 ± 0.04 |
| IGKV4-1 | G29R | IGHV3-15 T94R | 0.20 ± 0.05 |
| IGKV3-20 | N53Q | IGHV3-15 T94R | 0.18 ± 0.05 |
| IGKV3-20 | N53Q, N28K | IGHV3-15 T94R | 0.29 ± 0.06 |
| IGKV3-20 | N53Q, G29L | IGHV3-15 T94R | 0.21 ± 0.03 |
| IGKV3-20 | N53Q, G29R | IGHV3-15 T94R | 0.19 ± 0.02 |

| | | | |
|---|---|---|---|
| * The amino acid position is the position by Kabat Numbering. | | | |

### Test Example 9-2: Enhancement of activity of humanized 19D7

Optimization of the humanized 19D7 was performed by introducing point mutations into the humanized framework found in Example 7-2. As a result, the humanized 19D7 mutant shown in Table 13 has been found.

**[Table 13]**

| Light chain variable region | | Heavy chain variable region | | IC50 (nM) |
|---|---|---|---|---|
| Frame | CDR mutation | Frame | CDR mutation | |
| IGKV3-20 | S25T | IGHV3-15 T94R | None | 0.31 ± 0.04 |
| | S26T | | None | 0.20 ± 0.01 |
| | S27aT | | None | 0.22 ± 0.04 |
| | Q90N | | None | 0.30 ± 0.17 |
| | None | | T31S | 0.26 ± 0.04 |
| | None | | A60V | 0.27 ± 0.04 |
| | None | | K64R | 0.24 ± 0.05 |
| | None | | D65E | 0.27 ± 0.04 |
| | None | | N99Q | 0.22 ± 0.04 |
| | None | | Y102F | 0.31 ± 0.06 |

### Test Example 10: antitumor activity of anti-human CCR8 antibody in tumor-inoculated human CCR8 knock-in mice (hereinafter referred to as hCCR8-KI (KI/KI) mice)

### (1) Production of hCCR8-KI (KI/KI) mice

The full-length of ORF of the gene encoding mouse CCR8 (SEQ ID NO: 39) was removed while the full-length of ORF of the gene encoding human CCR8 (SEQ ID NO: 1) was inserted, thereby producing hCCR8-KI (KI/KI) mice in which the CCR8 protein expressed is fully humanized.

DNA fragments to be used as homologous recombinantion arms were obtained by PCR amplification of mouse genomic sequences about 2 kb (kilobase) upstream and downstream of the ORF of the mouse CCR8 gene, respectively. The homologous recombinantion arms were designed so that only ORF was removed. The homologous recombinantion arms were seamlessly connected to both sides of the full-length sequence of ORF of the human CCR8 gene, respectively, to produce a targeting vector. The targeting vector was subjected to homologous recombination to produce a hCCR8-KI (KI/+) Balb/c mouse. The produced hCCR8-KI (KI/+) Balb/c mouse was confirmed that the full-length ORF of the mouse CCR8 gene was correctly replaced to the full-length ORF of the human CCR8 gene and there were no insertions or deletions by PCR and DNA nucleotide sequence analysis. The hCCR8-KI (KI/+) Balb/c mice were crossed to produce a hCCR8-KI (KI/KI) mouse.

### (2) Confirmation of human CCR8 expression in intratumoral infiltrating cells in CT26 colon cancer inoculated in hCCR8-KI(KI/KI) mice

3.5 x 10⁵ (50 µL) CT26 cells were inoculated on back skin of hCCR8-KI (KI/KI) mice (6 weeks old, female) and tumors were recovered from 5 individuals 17 days after inoculation (N = 5). Tumor masses of CT26 cells were finely cut with scissors, and tumor-infiltrating cells were prepared with commercially available kits (Tumor Dissociation Kit, mouse, Miltenyi Biotec, and The gentleMACS (TM) Dissociator, Miltenyi Biotec) according to the attached kit protocols.

The prepared cells were passed through a 70 um cell strainer and then washed twice with 10 mM HEPES/HBSS/2% FBS. The cells were then treated with a red blood cell lysing solution (BD Biosciences) for 5 minutes to remove red blood cells, and further washed twice with a 2% FBS/10 mM HEPES/HBSS buffer. Tumor-infiltrating cells were stained by the following method with the antibodies described below.

Infiltrating cells were stained in ice with a Zombie NIR Fixable Viability Kit (BioLegend) reagent for 30 minutes. After one wash with 2% FBS/10 mM HEPES/HBSS, the cells were stained with Bv510-labeled anti-mouse CD45 (30-F11, BioLegend), FITC-labeled anti-mouse CD4 (RM4-4, BioLegend), PE/Cy7-labeled anti-mouse CD8 (53-6.7, BioLegend), PerCP/Cy5.5-labeled anti-mouse TCRβ (H57-597, BioLegend), PE-labeled anti-mouse CD25 (PC61, BioLegend), BV421-labeled anti-human CCR8 antibody (433H, BD Biosciences) (or BV421-labeled isotope control antibody). Staining was performed in ice for 30 minutes. After washing twice with 2% FBS/HEPES/HBSS, the cells were analyzed using a flow cytometer.

Human CCR8 expression in CD45+TCRβ+CD4+CD25+ T cells was analyzed. Negative cell regions were determined by staining with isotype-controlled antibodies, and the frequency of positive in intratumoral infiltrating cells in cancer in 17 days after inoculation was calculated by taking cells becoming positive with anti-human CCR8 antibodies as human CCR8+ cells. As a result, human CCR8 was detected in approximately 47% of CD45+TCRβ+CD4+CD25+ cells in mouse tumors.

### (3) Evaluation of antitumor effect of anti-human CCR8 antibody administration in mice inoculated with colon cancer-derived CT26 cells

On back skin of hCCR8-KI (KI/KI) mice (8 weeks old, female), 4 x 10⁵ (50 uL) colon cancer-derived CT26 cells were inoculated. 4 and 11 days after tumor inoculation, 100 µg (100 µL) or 200 µg (100 µL) of the humanized 10A11 antibody (light chain variable region: IGKV4-1 N53Q + G29R (SEQ ID NO: 59)/heavy chain variable region: IGHV3-15 T94R (SEQ ID NO: 41)) was administered intravenously (N = 10). As a control, 100 µL of vehicle (phosphate buffered saline) (N = 10) was administered. Tumor volumes were measured 4, 7, 10, 11, 14, 16, 18, 21, 24 days after tumor inoculation. The tumor volume (mm³) was measured in long diameter (mm) x short diameter (mm) x short diameter (mm)/2.

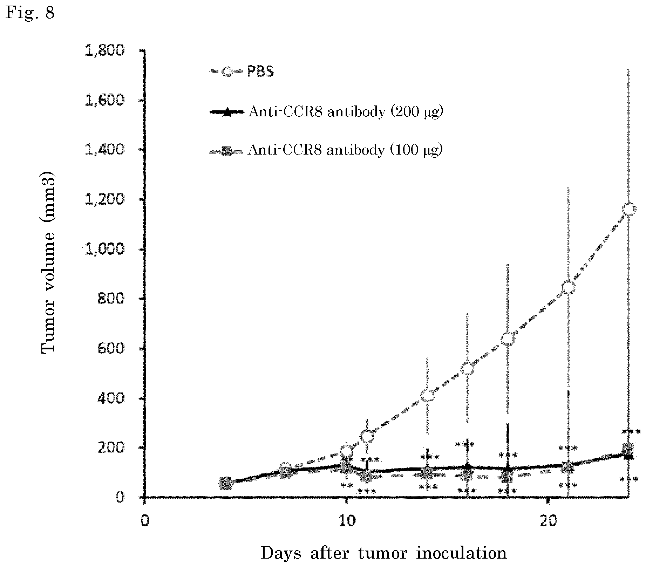

As a result, tumor volumes were significantly smaller in the anti-human CCR8 antibody dosing group at any dose 11, 14, 16, 18, 21 and 24 days after inoculation (Fig. 8, significance levels by Welch's t test were **; p < 0.01 on day 10, and ***; p < 0.001 on day 11 and later, at any dose.). Also in the anti-human CCR8 antibody administration group, individuals indicating complete regression appeared. After 24 days, tumors disappeared almost completely in 5 out of 10 mice in the 100 pg anti-human CCR8 antibody administration group, and 6 out of 10 mice in the 200 pg anti-human CCR8 antibody administration group.

### Example 1 Evaluation of antitumor effect of combination of anti-mouse CCR8 antibody and carboplatin administration in mice inoculated with breast cancer-derived 4T1 cells

The back of each Balb/c mouse (6 weeks old, female) was intracutaneously inoculated with 2 x 10⁵ breast cancer-derived 4T1 cells (50 µL).

To an anti-mouse CCR8 antibody single administration group, 50 pg (200 µL) of an anti-mouse CCR8 antibody (clone SA214G2, BioLegend, Inc.) was intravenously administered 5 and 8 days after tumor inoculation, and saline was intravenously administered at a volume of 11.4 mL/kg body weight 5 days after tumor inoculation (N = 10). To a carboplatin single administration group, 50 pg (200 µL) of an isotype control antibody (clone LTF-2, Bio X Cell, Inc.) was intravenously administered 5 and 8 days after tumor inoculation, and carboplatin (trade name: PARAPLATIN, Bristol-Myers Squibb Company) at 114 mg/kg body weight was intravenously administered at a volume of 11.4 mL/kg body weight 5 days after tumor inoculation (N = 10). To a carboplatin and anti-mouse CCR8 antibody combined administration group, 50 pg of an anti-mouse CCR8 antibody was intravenously administered 5 and 8 days after tumor inoculation, and carboplatin at 114 mg/kg body weight was intravenously administered 5 days after tumor inoculation (N = 10). To a control group, 50 pg of an isotype control antibody was intravenously administered 5 and 8 days after tumor inoculation, and saline was intravenously administered 5 days after tumor inoculation (N = 10). Tumor volumes were measured every 3 to 4 days from 5 days after tumor inoculation. The tumor volume (mm³) was measured in long diameter (mm) x short diameter (mm) x short diameter (mm)/2, and a significant difference test based on a Mann-Whitney U method (significance level: P < 0.05) was conducted.

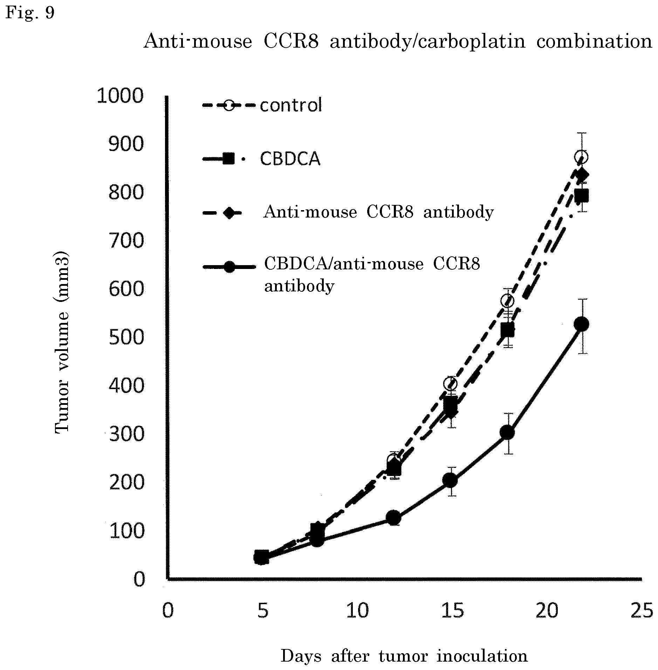

The results are shown in Fig. 9. There was no significant difference in average tumor volume between the control group and the anti-mouse CCR8 antibody single administration group and between the control group and the carboplatin single administration group 8, 12, 15, 18 and 22 days after tumor inoculation. On the other hand, in the anti-mouse CCR8 antibody and carboplatin combined administration group, there was a significant decrease in tumor volume as compared to the control group from 8 days after tumor inoculation. Further, there was a significant decrease in tumor volume as compared to the anti-mouse CCR8 antibody single administration group and the carboplatin single administration group from 8 days after tumor inoculation.

The above results revealed that the combined administration of an anti-mouse CCR8 antibody and carboplatin which have no significant effect on the tumor volume when administered alone had a synergistic antitumor effect as compared to the single administration groups.

### Example 2 Evaluation of antitumor effect of combination of anti-mouse CCR8 antibody and gemcitabine administration in mice inoculated with breast cancer-derived 4T1 cells

The back of each Balb/c mouse (6 weeks old, female) was intracutaneously inoculated with 2 x 10⁵ breast cancer-derived 4T1 cells (50 µL).

To an anti-mouse CCR8 antibody single administration group, 50 pg (200 µL) of an anti-mouse CCR8 antibody (clone SA214G2, BioLegend, Inc.) was intravenously administered 5 and 8 days after tumor inoculation, and saline was intravenously administered at a volume of 10 mL/kg body weight 5 days after tumor inoculation (N = 10). To a gemcitabine single administration group, 50 pg (200 µL) of an isotype control antibody (clone LTF-2, Bio X Cell, Inc.) was intravenously administered 5 and 8 days after tumor inoculation, and gemcitabine (trade name: GEMZAR, Eli Lilly Inc.) at 50 mg/kg body weight was intravenously administered at a volume of 10 mL/kg body weight 5 days after tumor inoculation (N = 10). To a gemcitabine and anti-mouse CCR8 antibody combined administration group, 50 pg of an anti-mouse CCR8 antibody was intravenously administered 5 and 8 days after tumor inoculation, and gemcitabine at 50 mg/kg body weight was intravenously administered 5 days after tumor inoculation (N = 10). To a control group, 50 pg of an isotype control antibody was intravenously administered 5 and 8 days after tumor inoculation, and saline was intravenously administered 5 days after tumor inoculation (N = 10). Tumor volumes were measured every 3 to 4 days from 5 days after tumor inoculation. The tumor volume (mm³) was measured in long diameter (mm) x short diameter (mm) x short diameter (mm)/2, and a significant difference test based on a Mann-Whitney U method (significance level: P < 0.05) was conducted.

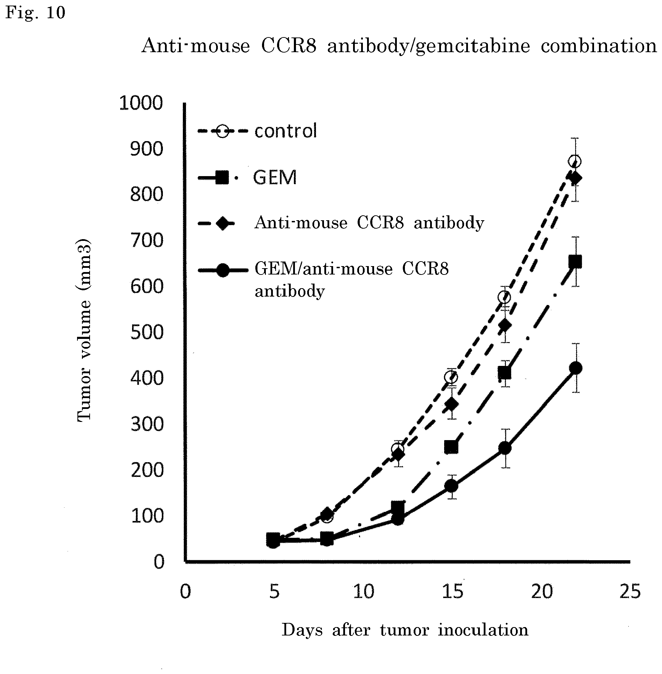

The results are shown in Fig. 10. In the gemcitabine single administration group, there was a significant decrease in tumor volume as compared to the control group 8, 12, 15, 18 and 22 days after tumor inoculation. There was no significant difference in tumor volume between the anti-mouse CCR8 antibody single administration group and the control group. On the other hand, in the anti-mouse CCR8 antibody and gemcitabine combined administration group, there was a significant decrease in tumor volume as compared to the control group or the anti-mouse CCR8 antibody single administration group from 8 days after tumor inoculation, and the tumor volume significantly decreased as compared to the gemcitabine single administration group from 12 days after tumor inoculation.

The above results revealed that the combined administration of an anti-mouse CCR8 antibody and gemcitabine which have no significant effect on the tumor volume when administered alone had a synergistic antitumor effect as compared to the single administration groups.

### Example 3 Evaluation of antitumor effect of combination of anti-human CCR8 antibody and cisplatin administration in hCCR8-KI (KI/KI) mice inoculated with breast cancer-derived 4T1 cells

The back of each hCCR8-KI (KI/KI) mouse (14 weeks old, female) prepared in Test Example 10 was intracutaneously inoculated with 2 x 10⁵ breast cancer-derived 4T1 cells (50 µL).

To an anti-human CCR8 antibody single administration group, 400 pg (200 µL) of an anti-human CCR8 antibody (humanized 10A11 antibody (light chain variable region: IGKV4-1 N53Q+G29R (SEQ ID NO: 59)/heavy chain variable region: IGHV3-15 T94R (SEQ ID NO: 41))) was intravenously administered 5 and 8 days after tumor inoculation, and saline was intravenously administered at a volume of 16 mL/kg body weight 5 days after tumor inoculation (N = 10). To a cisplatin single administration group, 200 pL of a control solution (obtained by diluting a buffer solution used for the antibody with saline in line with an antibody dilution ratio) was intravenously administered 5 and 8 days after tumor inoculation, and cisplatin (trade name: RANDA, Nippon Kayaku Co.,Ltd.) at 8 mg/kg body weight was intravenously administered at a volume of 16 mL/kg body weight 5 days after tumor inoculation (N = 10). To an anti-human CCR8 antibody and cisplatin combined administration group, 400 pg of an anti-human CCR8 antibody was intravenously administered 5 and 8 days after tumor inoculation, and cisplatin at 8 mg/kg body weight was intravenously administered 5 days after tumor inoculation (N = 10). To a control group, 200 pL of a control solution was intravenously administered 5 and 8 days after tumor inoculation, and saline was intravenously administered 5 days after tumor inoculation (N = 10). Tumor volumes were measured every 2 to 4 days from 5 days after tumor inoculation. The tumor volume (mm³) was measured in long diameter (mm) x short diameter (mm) x short diameter (mm)/2, and a significant difference test based on a Mann-Whitney U method (significance level: P < 0.05) was conducted.

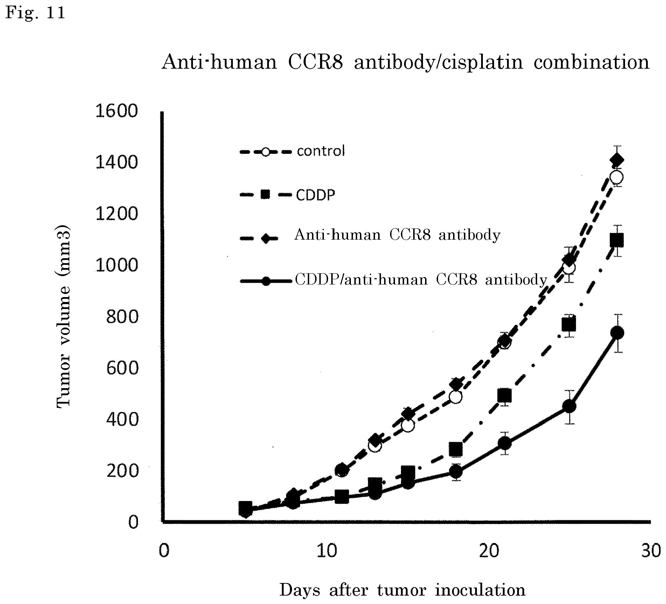

The results are shown in Fig. 11. In the cisplatin single administration group, there was a significant decrease in tumor volume as compared to the control group 8, 11, 13, 15, 18, 21, 25 and 29 days after tumor inoculation. In the anti-human CCR8 antibody single administration group, there was no significant decrease in tumor volume. On the other hand, in the anti-human CCR8 antibody and cisplatin combined administration group, there was a significant decrease in tumor volume as compared to the control group or the anti-human CCR8 antibody single administration group from 8 days after tumor inoculation, and the tumor volume significantly decreased as compared to the cisplatin single administration group from 18 days after tumor inoculation.

The above results revealed that the combined administration of an anti-human CCR8 antibody and cisplatin which have no significant effect on the tumor volume when administered alone had a synergistic antitumor effect as compared to the single administration groups.

### Example 4 Evaluation of antitumor effect of combination of anti-mouse CCR8 antibody and carboplatin or cisplatin administration in mice inoculated with bladder cancer-derived MB49 cells

The back of each C57BL/6 mouse (6 weeks old, male) was intracutaneously inoculated with 2 × 10⁵ bladder cancer-derived MB49 cells (50 µL).

To an anti-mouse CCR8 antibody single administration group, 50 pg (200 µL) of an anti-mouse CCR8 antibody (clone SA214G2, BioLegend, Inc.) was intravenously administered 4 and 7 days after tumor inoculation, and saline was intravenously administered at a volume of 10 mL/kg body weight 4 days after tumor inoculation (N = 10).

To a carboplatin single administration group, 50 pg (200 µL) of an isotype control antibody (clone LTF-2, Bio X Cell, Inc.) was intravenously administered 4 and 7 days after tumor inoculation, and carboplatin (trade name: PARAPLATIN, Bristol-Myers Squibb Company) at 40 mg/kg body weight was intravenously administered at a volume of 10 mL/kg body weight 4 days after tumor inoculation (N = 10). To an anti-mouse CCR8 antibody and carboplatin combined administration group, 50 pg of an anti-mouse CCR8 antibody was intravenously administered 4 and 7 days after tumor inoculation, and carboplatin at 40 mg/kg body weight was intravenously administered 4 days after tumor inoculation (N = 10).

To a cisplatin single administration group, 50 pg (200 µL) of an isotype control antibody was intravenously administered 4 and 7 days after tumor inoculation, and cisplatin (trade name: RANDA, Nippon Kayaku Co.,Ltd.) at 3 mg/kg body weight was intravenously administered at a volume of 10 mL/kg body weight 4 days after tumor inoculation (N = 10). To a cisplatin and anti-mouse CCR8 antibody combined administration group, 50 pg of an anti-mouse CCR8 antibody was intravenously administered 4 and 7 days after tumor inoculation, and cisplatin at 3 mg/kg body weight was intravenously administered 4 days after tumor inoculation (N = 10).

To a control group, 50 pg of an isotype control antibody was intravenously administered 4 and 7 days after tumor inoculation, and saline was intravenously administered 4 days after tumor inoculation (N = 10).

Tumor volumes were measured every 1 to 4 days from 4 days after tumor inoculation. The tumor volume (mm³) was measured in long diameter (mm) x short diameter (mm) x short diameter (mm)/2, and a significant difference test based on a Mann-Whitney U method (significance level: P < 0.05) was conducted.

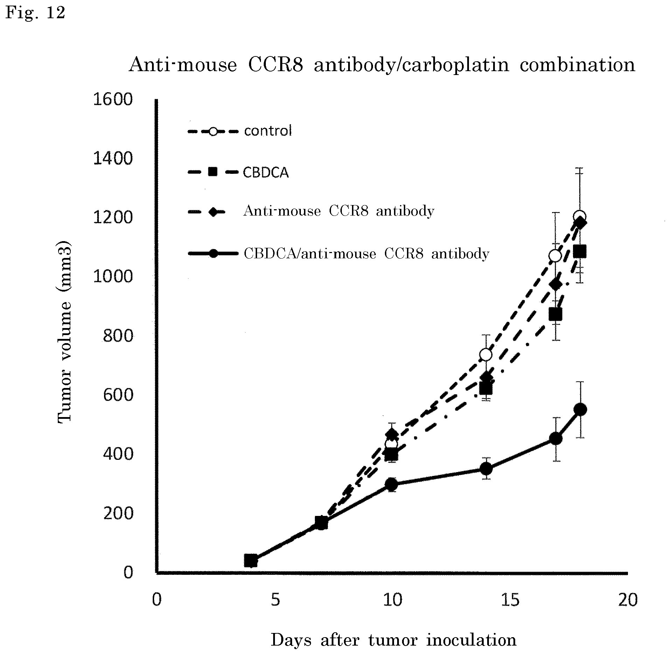

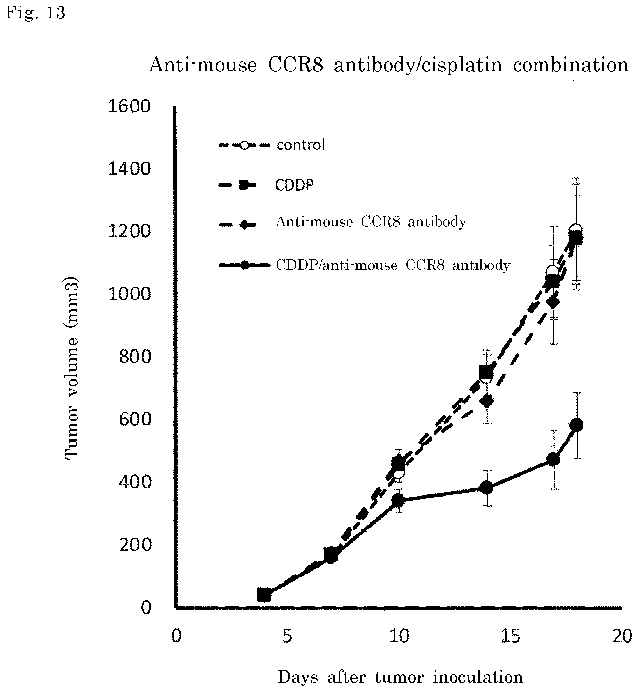

The results are shown in Fig. 12 (carboplatin) and Fig. 13 (cisplatin). There was no significant difference in average tumor volume between the control group and the anti-mouse CCR8 antibody single administration group, between the control group and the carboplatin single administration group and between the control group and the cisplatin single administration group 7, 10, 14, 17 and 18 days after tumor inoculation. On the other hand, in the anti-mouse CCR8 antibody and carboplatin combined administration group, there was a significant decrease in tumor volume as compared to the control group from 10 days after tumor inoculation. Further, there was a significant decrease in tumor volume as compared to the anti-mouse CCR8 antibody single administration group and the carboplatin single administration group from 10 days after tumor inoculation. In the anti-mouse CCR8 antibody and cisplatin combined administration group, there was a significant decrease in tumor volume as compared to the control group from 14 days after tumor inoculation. Further, there was a significant decrease in tumor volume as compared to the anti-mouse CCR8 antibody single administration group and the cisplatin single administration group from 10 days after tumor inoculation.

The above results revealed that the combined administration of an anti-mouse CCR8 antibody and carboplatin or cisplatin which have no significant effect on the tumor volume when administered alone had a synergistic antitumor effect as compared to the single administration groups.

### Example 5 Evaluation of antitumor effect of combination of the anti-mouse CCR8 antibody and oxaliplatin or fluorouracil (5-FU) in mice inoculated with colon cancer-derived Colon26

The back of each Balb/c mouse (5 weeks old, female) was intracutaneously inoculated with 3.5 x 10⁵ colon cancer-derived Colon26 cells (50 µL).

To an anti-mouse CCR8 antibody single administration group, 50 pg (200 µL) of an anti-mouse CCR8 antibody (clone SA214G2, BioLegend, Inc.) was intravenously administered 5 days after tumor inoculation, and saline was intravenously administered at a volume of 10 ml/kg body weight 1 hour after the administration of the anti-mouse CCR8 antibody (N = 10).

To an oxaliplatin single administration group, 50 pg (200 µL) of an isotype control antibody was intravenously administered 5 days after tumor inoculation, and oxaliplatin (trade name: ELPLAT, Yakult Honsha Company, Limited) at 5 mg/kg body weight was intravenously administered at a volume of 10 mL/kg body weight 1 hour after the administration of the isotype control antibody (N = 10). To an anti-mouse CCR8 antibody and oxaliplatin combined administration group, 50 pg of an anti-mouse CCR8 antibody was intravenously administered 5 days after tumor inoculation, and oxaliplatin at 5 mg/kg body weight was intravenously administered 1 hour after the administration of the anti-mouse CCR8 antibody (N = 10).

To a 5-FU single administration group, 50 pg (200 µL) of an isotype control antibody was intravenously administered 5 days after tumor inoculation, and 5-FU (trade name: 5-FU INJECTION 250 KYOWA, Kyowa Hakko Kirin Co. Ltd) at 30 mg/kg body weight was intravenously administered at a dose of 30 mg/kg body weight and a volume of 10 mL/kg body weight 1 hour after the administration of the isotype control antibody (N = 10). To an anti-mouse CCR8 antibody and 5-FU combined administration group, 50 pg of an anti-mouse CCR8 antibody was intravenously administered 5 days after tumor inoculation, and 5-FU was intravenously administered at 30 mg/kg body weight 1 hour after the administration of the anti-mouse CCR8 antibody (N = 10).

To a control group, 50 pg of an isotype control antibody was intravenously administered 5 days after tumor inoculation, and saline was intravenously administered 1 hour after the administration of the isotype control antibody (N = 10).

Tumor volumes were measured every 2 to 3 days from 4 days after tumor inoculation (a day before antibody administration). The tumor volume (mm³) was measured in long diameter (mm) x short diameter (mm) x short diameter (mm)/2, and a significant difference test based on a Mann-Whitney U method (significance level: P < 0.05) was conducted.

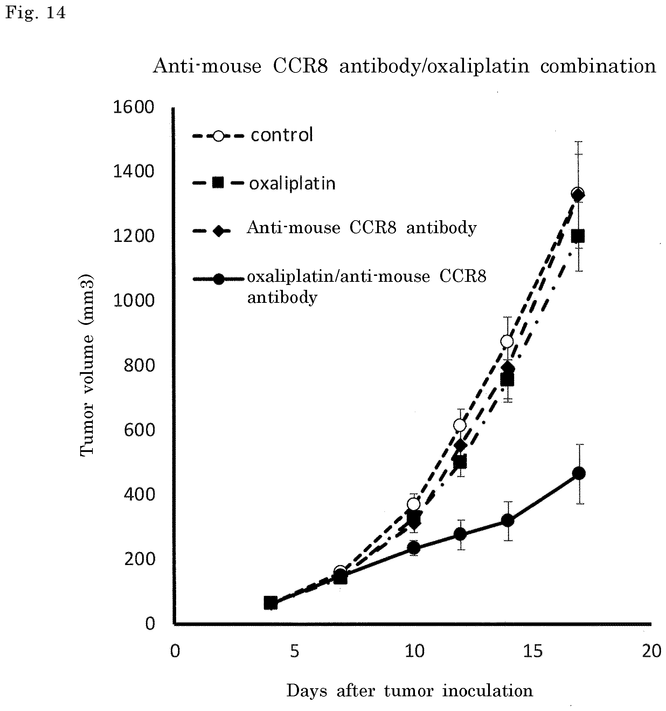

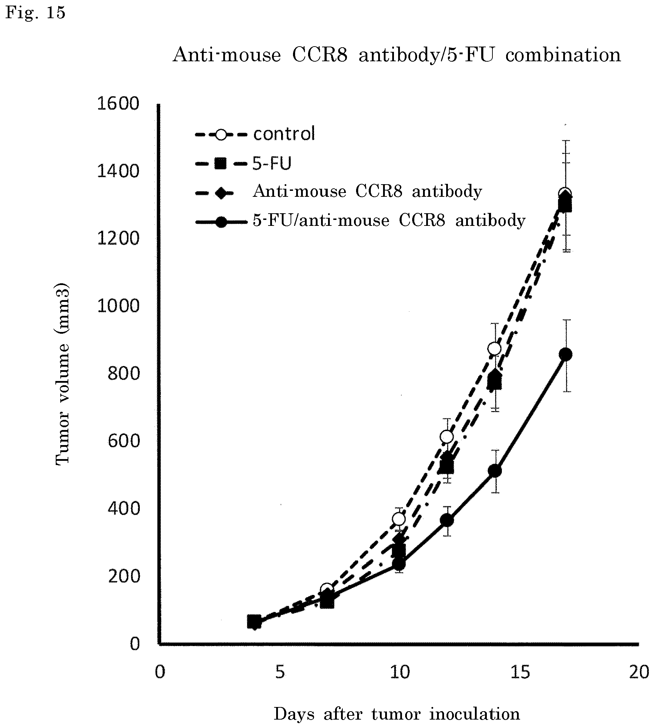

The results are shown in Fig. 14 (oxaliplatin) and Fig. 15 (5-FU).

In the oxaliplatin single administration group and the anti-mouse CCR8 antibody single administration group, there was no significant decrease in tumor volume 4, 7, 10, 12, 14 and 17 days after tumor inoculation. On the other hand, in the anti-mouse CCR8 antibody and oxaliplatin combined administration group, there was a significant decrease in tumor volume as compared to the control group from 10 days after tumor inoculation, as compared to the oxaliplatin single administration group from 10 days after tumor inoculation, and as compared to the anti-mouse CCR8 antibody single administration group from 12 days after tumor inoculation. In the 5-FU single administration group, there was a significant decrease in tumor volume as compared to the control group only 7 days after tumor inoculation. On the other hand, in the anti-mouse CCR8 antibody and 5-FU combined administration group, there was a significant decrease in tumor volume as compared to the control group from 10 days after tumor inoculation, as compared to the 5-FU single administration group from 12 days after tumor inoculation, and as compared to the anti-mouse CCR8 antibody single administration group from 14 days after tumor inoculation.

The above results revealed that the combined administration of an anti-mouse CCR8 antibody and oxaliplatin or 5-FU which have no significant effect on the tumor volume when administered alone had a synergistic antitumor effect as compared to the single administration groups.

### Example 6 Evaluation of antitumor effect of combination of anti-human CCR8 antibody and oxaliplatin administration in hCCR8-KI (KI/KI) mice inoculated with colon cancer-derived Colon26 cells

The back of each hCCR8-KI (KI/KI) mouse (12 weeks old, female) prepared in Test Example 10 was intracutaneously inoculated with 3.5 x 10⁵ colon cancer-derived Colon26 cells (50 µL).

To an anti-human CCR8 antibody single administration group, an anti-human CCR8 antibody (humanized 10A11 antibody (light chain variable region: IGKV4-1 N53Q+G29R (SEQ ID NO: 59)/heavy chain variable region: IGHV3-15 T94R (SEQ ID NO: 41))) at 15 mg/kg body weight was intravenously administered 5 days after tumor inoculation, and saline was intravenously administered 3 hours after the administration of the anti-human CCR8 antibody (N = 10). To an oxaliplatin single administration group, saline was intravenously administered 5 days after tumor inoculation, and oxaliplatin (trade name: ELPLAT, Yakult Honsha Company, Limited) at 5 mg/kg body weight was intravenously administered 3 hours after the administration of the saline (N = 10). To an anti-mouse CCR8 antibody and oxaliplatin combined administration group, an anti-human CCR8 antibody at 15 mg/kg body weight was intravenously administered 5 days after tumor inoculation, and oxaliplatin at 5 mg/kg body weight was intravenously administered 3 hours after the administration of the anti-mouse CCR8 antibody (N = 10). To a control group, saline was intravenously administered 5 days after tumor inoculation, and saline was intravenously administered 3 hours after the administration of the saline (N = 10). For all the analytes, tail vein administration was carried out at a volume of 10 mL/kg body weight. Tumor volumes were measured every 1 to 3 days from 4 days after tumor inoculation (a day before antibody administration). The tumor volume (mm³) was measured in long diameter (mm) x short diameter (mm) x short diameter (mm)/2, and a significant difference test based on a Mann-Whitney U method (significance level: P < 0.05) was conducted.

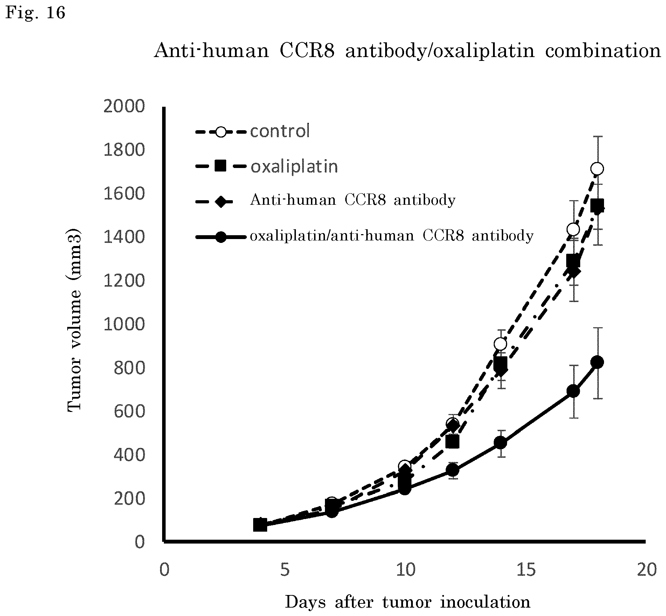

The results are shown in Fig. 16. In the oxaliplatin single administration group and the anti-human CCR8 antibody single administration group, there was no significant decrease in tumor volume 4, 7, 10, 12, 14, 17 and 18 days after tumor inoculation. On the other hand, in the anti-human CCR8 antibody and oxaliplatin combined administration group, there was a significant decrease in tumor volume as compared to the control group from 7 days after tumor inoculation, as compared to the anti-human CCR8 antibody single administration group from 10 days after tumor inoculation, and as compared to oxaliplatin single administration group from 12 days after tumor inoculation.

The above results revealed that the combined administration of an anti-human CCR8 antibody and cisplatin which have no significant effect on the tumor volume when administered alone had a synergistic antitumor effect as compared to the single administration groups.

### Example 7 Evaluation of antitumor effect of combination of anti-human CCR8 antibody and cisplatin administration in hCCR8-KI (KI/KI) mice inoculated with lung cancer-derived ASB-XIV cells

The back of each hCCR8-KI (KI/KI) mouse (8 weeks old, female) prepared in Test Example 10 was intracutaneously inoculated with 5 x 10⁵ lung cancer-derived ASB-XIV cells (50 µL).

To an anti-human CCR8 antibody single administration group, an anti-human CCR8 antibody (humanized 10A11 antibody (light chain variable region: IGKV4-1 N53Q+G29R (SEQ ID NO: 59)/heavy chain variable region: IGHV3-15 T94R (SEQ ID NO: 41))) at 1 mg/10 mL/kg body weight and saline at 10 mL/kg body weight were intravenously administered 5 days after tumor inoculation (N = 10). To a cisplatin single administration group, a control solution at 10 mL/kg body weight and cisplatin (trade name: RANDA, Nippon Kayaku Co.,Ltd.) at 3 mg/10 mL/kg body weight were intravenously administered 5 days after tumor inoculation (N = 10). To an anti-human CCR8 antibody and cisplatin combined administration group, an anti-human CCR8 antibody at 1 mg/10 mL/kg body weight and cisplatin at 3 mg/10 mL/kg body weight were intravenously administered 5 days after tumor inoculation (N = 10). To a control group, a control solution at 10 mL/kg body weight and saline at 10 mL/kg body weight were intravenously administered 5 days after tumor inoculation (N = 10). Tumor volumes were measured every 2 to 5 days from 5 days after tumor inoculation. The tumor volume (mm³) was measured in long diameter (mm) x short diameter (mm) x short diameter (mm)/2, and a significant difference test based on a Mann-Whitney U method (significance level: P < 0.05) was conducted.

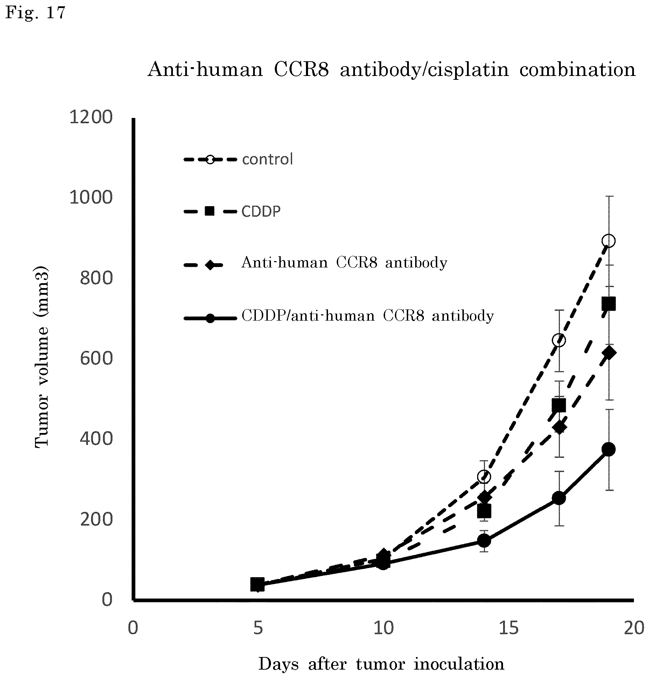

The results are shown in Fig. 17. In the anti-human CCR8 antibody single administration group and the cisplatin single administration group, there was no significant decrease in tumor volume 14, 17 and 19 days after tumor inoculation. On the other hand, in the anti-human CCR8 antibody and cisplatin combined administration group, there was a significant decrease in tumor volume as compared to the control group, and there was a significant decrease in tumor volume as compared to the cisplatin single administration group or the anti-human CCR8 antibody single administration group 14 days after tumor inoculation.

The above results revealed that by combined administration of an anti-human CCR8 antibody and cisplatin which have no significant effect on the tumor volume when administered alone, a significant antitumor effect was exhibited.

### Example 8 Evaluation of antitumor effect of combination of anti-human CCR8 antibody and paclitaxel administration in hCCR8-KI (KI/KI) mice inoculated with lung cancer-derived ASB-XIV cells

The back of each hCCR8-KI (KI/KI) mouse (9 weeks old, female) prepared in Test Example 10 was intracutaneously inoculated with 5 x 10⁵ lung cancer-derived ASB-XIV cells (50 µL).

To an anti-human CCR8 antibody single administration group, an anti-human CCR8 antibody (humanized 10A11 antibody (light chain variable region: IGKV4-1 N53Q+G29R (SEQ ID NO: 59)/heavy chain variable region: IGHV3-15 T94R (SEQ ID NO: 41))) at 0.5 mg/10 mL/kg body weight and saline at 10 mL/kg body weight were intravenously administered 5 days after tumor inoculation (N = 10). To a paclitaxel single administration group, saline at 10 mL/kg body weight and paclitaxel (trade name: TAXOL, Bristol-Myers Squibb Company) at 8 mg/10 mL/kg body weight were intravenously administered 5 days after tumor inoculation (N = 10). To an anti-human CCR8 antibody and paclitaxel combined administration group, an anti-human CCR8 antibody at 0.5 mg/10 mL/kg body weight and paclitaxel at 8 mg/10 mL/kg body weight were intravenously administered 5 days after tumor inoculation (N = 10). To a control group, saline was intravenously administered at 20 mL/kg 5 days after tumor inoculation (N = 10). Tumor volumes were measured every 2 to 5 days from 5 days after tumor inoculation. The tumor volume (mm³) was measured in long diameter (mm) x short diameter (mm) x short diameter (mm)/2, and a significant difference test based on a Student's T: one-sided test method (significance level: P < 0.05) was conducted.

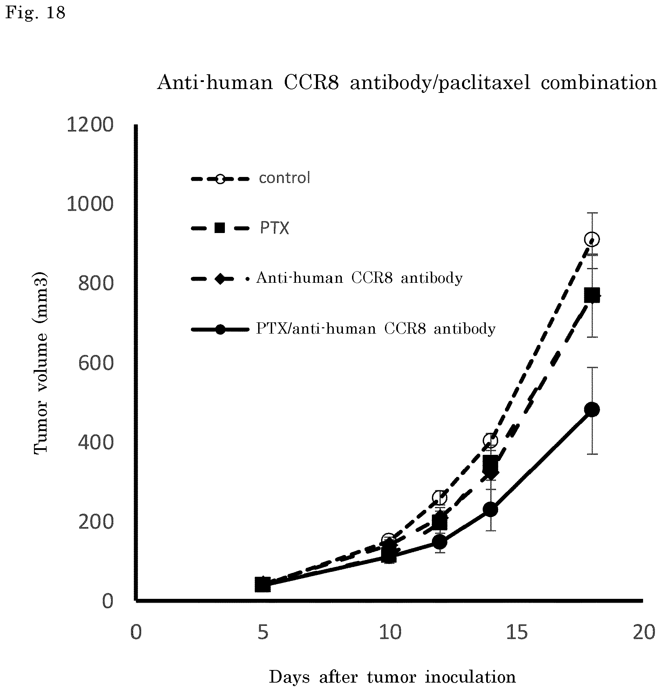

The results are shown in Fig. 18. In the anti-human CCR8 antibody single administration group, there was no significant decrease in tumor volume 10, 12, 14 and 18 days after tumor inoculation. In the paclitaxel single administration group, there was a significant decrease in tumor volume 10 and 12 days after tumor inoculation, but the significant difference disappeared 14 and 17 days after tumor inoculation. On the other hand, in the anti-human CCR8 antibody and paclitaxel combined administration group, there was a significant decrease in tumor volume as compared to the control group from 10 days after tumor inoculation, and the tumor volume significantly decreased as compared to the paclitaxel single administration group or the anti-human CCR8 antibody single administration group from 18 days after tumor inoculation.

The above results revealed that the combined administration of an anti-human CCR8 antibody and paclitaxel which have no significant effect on the tumor volume when administered alone had a synergistic antitumor effect as compared to the single administration groups.

### Example 9 Evaluation of antitumor effect of combination of anti-human CCR8 antibody and docetaxel administration in hCCR8-KI (KI/KI) mice inoculated with breast cancer-derived 4T1 cells

The back of each hCCR8-KI (KI/KI) mouse (9 weeks old, female) prepared in Test Example 10 was intracutaneously inoculated with 2 x 10⁵ breast cancer-derived 4T1 cells (50 µL).

To an anti-human CCR8 antibody single administration group, an anti-human CCR8 antibody (humanized 10A11 antibody (light chain variable region: IGKV4-1 N53Q+G29R (SEQ ID NO: 59)/heavy chain variable region: IGHV3-15 T94R (SEQ ID NO: 41))) at 15 mg/10 mL/kg body weight and saline at 10 mL/kg body weight were intravenously administered 5 days after tumor inoculation (N = 10). To a docetaxel single administration group, saline at 10 mL/kg body weight and docetaxel (trade name: ONETAXOTERE, Sanofi S.A.) at 30 mg/10 mL/kg body weight were intravenously administered 5 days after tumor inoculation (N = 10). To an anti-human CCR8 antibody and docetaxel combined administration group, an anti-human CCR8 antibody at 15 mg/10 mL/kg body weight and docetaxel at 30 mg/10 mL/kg body weight were intravenously administered 5 days after tumor inoculation (N = 10). To a control group, saline was intravenously administered at 20 mL/kg 5 days after tumor inoculation (N = 10). Tumor volumes were measured every 3 to 4 days from 5 days after tumor inoculation. The tumor volume (mm³) was measured in long diameter (mm) x short diameter (mm) x short diameter (mm)/2, and a significant difference test based on a Mann-Whitney U method (significance level: P < 0.05) was conducted.

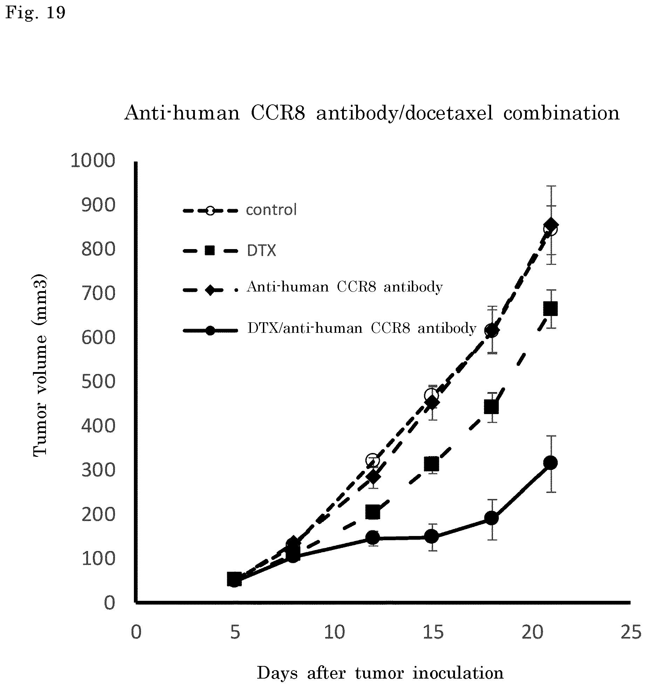

The results are shown in Fig. 19. In the anti-human CCR8 antibody single administration group, there was no significant decrease in tumor volume 8, 12, 15, 18 and 21 days after tumor inoculation. On the other hand, in the anti-human CCR8 antibody and docetaxel combined administration group, there was a significant decrease in tumor volume as compared to the control group or the anti-human CCR8 antibody single administration group, and the tumor volume significantly decreased as compared to the docetaxel single administration group from 12 days after tumor inoculation.

The above results revealed that the combined administration of an anti-human CCR8 antibody and docetaxel which have no significant effect on the tumor volume when administered alone had a synergistic antitumor effect as compared to the single administration groups.

### Example 10 Evaluation of antitumor effect of combination of anti-mouse CCR8 antibody and docetaxel administration in mice inoculated with breast cancer-derived 4T1 cells

The back of each Balb/c mouse (6 weeks old, female) was intracutaneously inoculated with 2 x 10⁵ breast cancer-derived 4T1 cells (50 µL).

To an anti-mouse CCR8 antibody single administration group, an anti-human CCR8 antibody (clone SA214G2, BioLegend, Inc.) was intravenously administered at 2.5 mg/10 mL/kg body weight 5 and 8 days after tumor inoculation, and saline was intravenously administered at 10 mL/kg body weight 5 days after tumor inoculation (N = 10). To a docetaxel single administration group, an isotype control antibody (clone LTF-2, Bio X Cell, Inc.) was intravenously administered at 2.5 mg/10 mL/kg 5 and 8 days after tumor inoculation, and docetaxel (trade name: ONETAXOTERE, Sanofi S.A.) was intravenously administered at 30 mg/10 mL/kg body weight 5 days after tumor inoculation (N = 10). To an anti-mouse CCR8 antibody and docetaxel combined administration group, an anti-mouse CCR8 antibody was intravenously administered at 2.5 mg/10 mL/kg body weight 5 and 8 days after tumor inoculation, and docetaxel was intravenously administered at 30 mg/10 mL/kg body weight 5 days after tumor inoculation (N = 10). To a control group, an isotype control antibody at 2.5 mg/10 mL/kg and saline at 10 mL/kg body weight were intravenously administered 5 days after tumor inoculation (N = 10). Tumor volumes were measured every 3 to 4 days from 5 days after tumor inoculation. The tumor volume (mm³) was measured in long diameter (mm) x short diameter (mm) x short diameter (mm)/2, and a significant difference test based on a Mann-Whitney U method (significance level: P < 0.05) was conducted.

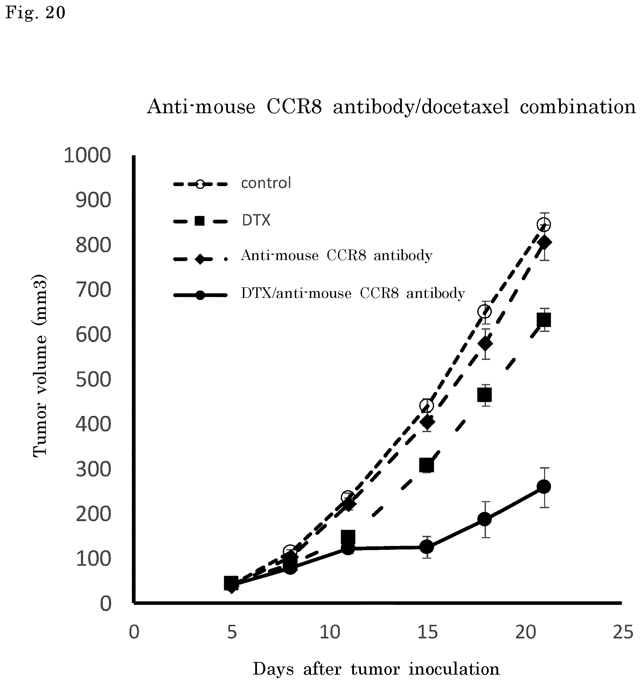

The results are shown in Fig. 20. In the anti-mouse CCR8 antibody single administration group, there was no significant decrease in tumor volume 8, 11, 15, 18 and 21 days after tumor inoculation. On the other hand, in the anti-mouse CCR8 antibody and docetaxel combined administration group, there was a significant decrease in tumor volume as compared to the control group or the anti-mouse CCR8 antibody single administration group, and the tumor volume significantly decreased as compared to the docetaxel single administration group from 15 days after tumor inoculation.

The above results revealed that the combined administration of an anti-mouse CCR8 antibody and docetaxel which have no significant effect on the tumor volume when administered alone had a synergistic antitumor effect as compared to the single administration groups.

### Example 11 Evaluation of antitumor effect of combination of anti-human CCR8 antibody and oxaliplatin administration in hCCR8-KI (KI/KI) mice inoculated with colon cancer-derived Colon26 cells (part 2)

Evaluation was performed in the same manner as in Example 6 except that as the anti-human CCR8 antibody, other two antibodies (2-7B antibody (light chain variable region: SEQ ID NO: 37/heavy chain variable region: SEQ ID NO: 38) and humanized 19D7 antibody (light chain variable region: IGKV3-20 (SEQ ID NO: 56)/heavy chain variable region: IGHV3-15 K64R+T94R (SEQ ID NO: 60))) were used. For the significant difference test, a significant difference test based on a Student's T: one-sided test method (significance level: P < 0.05) was conducted.

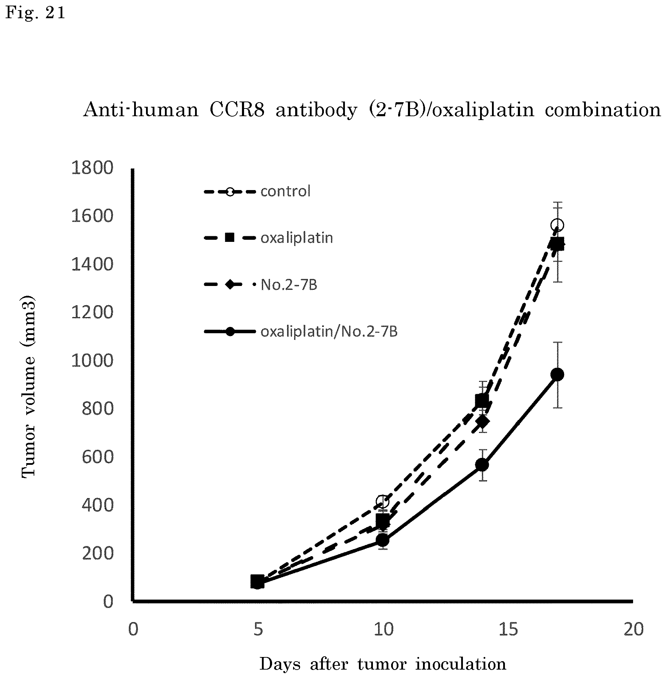

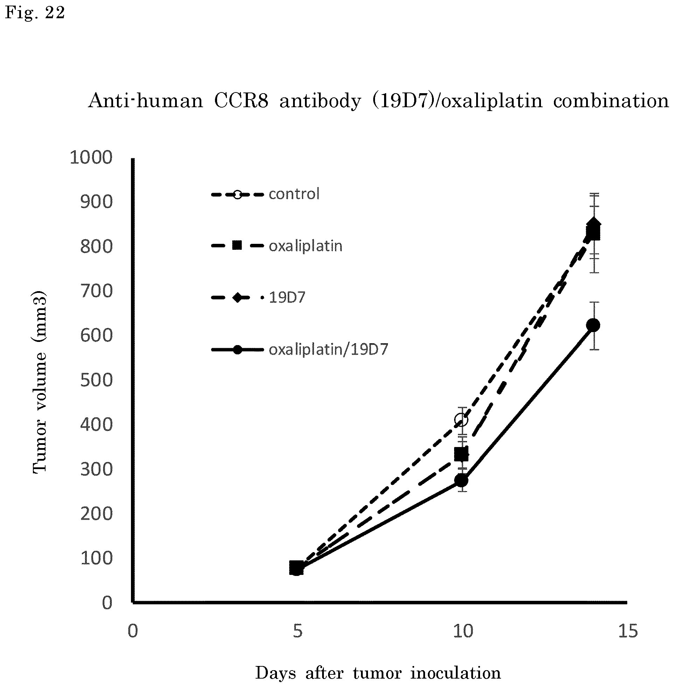

The results are shown in Fig. 21 (2-7B antibody) and Fig. 22 (humanized 19D7 antibody).

### 1) 2-7B antibody

There was no significant decrease in tumor volume as compared to the control group 10, 14 and 17 days after tumor inoculation in the oxaliplatin single administration group and 14 and 17 days after tumor inoculation in the anti-human CCR8 antibody single administration group. On the other hand, in the anti-human CCR8 antibody and oxaliplatin combined administration group, there was a significant decrease in tumor volume as compared to the control group and the anti-human CCR8 antibody single administration group from 10 days after tumor inoculation, and as compared to the oxaliplatin single administration group from 14 days after tumor inoculation.

### 2) Humanized 19D7 antibody

There was no significant decrease in tumor volume as compared to the control group 10 and 14 days after tumor inoculation in the oxaliplatin single administration group and 14 days after tumor inoculation in the anti-human CCR8 antibody single administration group. On the other hand, in the anti-human CCR8 antibody and oxaliplatin combined administration group, there was a significant decrease in tumor volume as compared to the control group and the anti-human CCR8 antibody single administration group from 10 days after tumor inoculation, and as compared to the oxaliplatin single administration group from 14 days after tumor inoculation.

The above results revealed that the combined administration of an anti-human CCR8 antibody and cisplatin which have no significant effect on the tumor volume when administered alone had a synergistic antitumor effect as compared to the single administration groups.

### Example 12 Evaluation of antitumor effect of combination of anti-human CCR8 antibody and fluorouracil (5-FU) administration in hCCR8-KI (KI/KI) mice inoculated with colon cancer-derived CT26 cells

The back of each hCCR8-KI (KI/KI) mouse (14 weeks old, female) prepared in Test Example 10 was intracutaneously inoculated with 3.5 x 10⁵ colon cancer-derived CT26 cells (50 µL).

To an anti-human CCR8 antibody single administration group, an anti-human CCR8 antibody (humanized 10A11 antibody (light chain variable region: IGKV4-1 N53Q+G29R (SEQ ID NO: 59)/heavy chain variable region: IGHV3-15 T94R (SEQ ID NO: 41)) was intravenously administered at 0.1 mg/kg body weight 5 days after tumor inoculation, and saline was intravenously administered 3 hours after the administration of the anti-human CCR8 antibody (N = 8). To a 5-FU single administration group, a saline was intravenously administered 5 days after tumor inoculation, and 5-FU (trade name: 5-FU INJECTION 250 KYOWA, Kyowa Hakko Kirin Co. Ltd) was intravenously administered at 30 mg/kg body weight 3 hours after the administration of the saline (N = 8). To an anti-human CCR8 antibody and 5-FU combined administration group, an anti-human CCR8 antibody was intravenously administered at 0.1 mg/kg 5 days after tumor inoculation, and 5-FU was intravenously administered at 30 mg/kg body weight 3 hours after the administration of the anti-human CCR8 antibody (N = 8). To a control group, saline was intravenously administered 5 days after tumor inoculation, and saline was intravenously administered 3 hours after the administration of the saline (N = 8). For all the analytes, tail vein administration was carried out at a volume of 10 mL/kg body weight. Tumor volumes were measured every 3 to 4 days from 5 days after tumor inoculation (a day before antibody administration). The tumor volume (mm³) was measured in long diameter (mm) x short diameter (mm) x short diameter (mm)/2, and a significant difference test based on a Mann-Whitney U method (significance level: P < 0.05) was conducted.

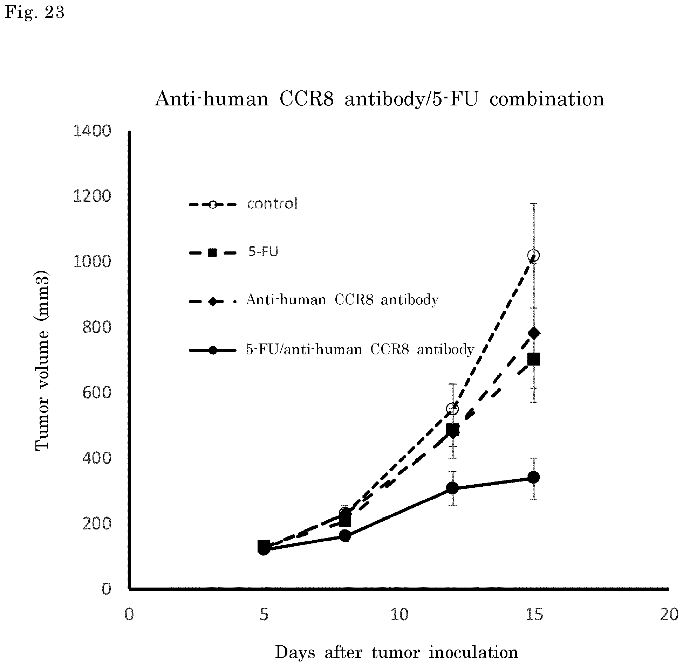

The results are shown in Fig. 23. In the 5-FU single administration group and the anti-human CCR8 antibody single administration group, there was no significant decrease in tumor volume as compared to the control group 8, 12 and 15 days after tumor inoculation. On the other hand, in the anti-human CCR8 antibody and 5-FU combined administration group, there was a significant decrease in tumor volume as compared to the control group, the anti-human CCR8 antibody single administration group and the 5-FU single administration group from 8 days after tumor inoculation.

The above results revealed that the combined administration of an anti-human CCR8 antibody and 5-FU which have no significant effect on the tumor volume when administered alone had a synergistic antitumor effect as compared to the single administration groups.

### Example 13 Evaluation of antitumor effect of combination of chemotherapy and anti-mCCR8 antibody using cell lines derived from mouse breast cancer, lung cancer, colon cancer, kidney cancer, sarcoma, liver cancer, bladder cancer and ovary cancer

The backs of Balb/c mice or C57BL/6 mice are intracutaneously inoculated with cell lines derived from mouse breast cancer, lung cancer, colon cancer, kidney cancer, sarcoma, liver cancer, bladder cancer and ovary cancer, respectively. After the tumor inoculation, rat anti-mouse CD198 (CCR8) antibody (Clone SA214G2, BioLegend Inc.) and chemotherapy are applied in combination. As a chemotherapeutic agent, carboplatin, cisplatin, oxaliplatin, paclitaxel, docetaxel, pemetrexed, gemcitabine, fluorouracil, irinotecan, etoposide or doxorubicin is intravenously administered in combination. As a control, an isotype control antibody is administered. The tumor volume (mm³) is measured every 3 to 4 days. The tumor volume (mm³) is measured in diameter (mm) x short diameter (mm) x short diameter (mm)/2.

From the results, whether the combined administration of the anti-mouse CCR8 antibody and the chemotherapy has a synergistic effect as compared to a case where the anti-mouse CCR8 antibody alone or the chemotherapy alone is administered is verified.

### Example 14 Evaluation of antitumor effect of combination of anti-human CCR8 antibody and chemotherapy in human CCR8 knock-in mice (hereinafter referred to as hCCR8-KI (KI/KI) mice) inoculated with cancer-derived cell line

The back of hCCR8-KI (KI/KI) mice in Test Example 10 is intracutaneously inoculated with a cell line derived from mouse breast cancer, lung cancer, colon cancer, kidney cancer, sarcoma, liver cancer, bladder cancer or ovary cancer. After the tumor inoculation, administration of the anti-human CCR8 antibody and chemotherapy are applied in combination.

As a chemotherapeutic agent, carboplatin, cisplatin, oxaliplatin, paclitaxel, docetaxel, pemetrexed, gemcitabine, fluorouracil, irinotecan, etoposide or doxorubicin is intravenously administered in combination. As a control, a medium (phosphate buffered saline) is administered. The tumor volume (mm³) is measured every 3 to 4 days. The tumor volume (mm³) is measured in diameter (mm) x short diameter (mm) x short diameter (mm)/2.

As the anti-human CCR8 antibody, the anti-CCR8 antibody according to any one of (14) to (18), the humanized monoclonal antibody according to any one of (19) to (27), or an antibody fragment thereof is used. For example, the antibody according to (26) is used.

From the results, whether the combined administration of the anti-human CCR8 antibody and the chemotherapy has a synergistic effect as compared to a case where the anti-human CCR8 antibody alone or the chemotherapy alone is administered is verified.

### [INDUSTRIAL APPLICABILITY]

The combination of an anti-CCR8 antibody and a chemotherapeutic agent is useful for treating or preventing cancer.

## Claims

1. A pharmaceutical composition which is used with a chemotherapeutic agent, the pharmaceutical composition comprising an anti-CCR8 antibody.

2. A pharmaceutical composition which is used with an anti-CCR8 antibody, the pharmaceutical composition comprising a chemotherapeutic agent.

3. The pharmaceutical composition according to claim 1 or 2, for cancer treatment.

4. The pharmaceutical composition according to claim 3, wherein the treatment with an anti-PD-1 antibody or an anti-PD-L1 antibody is ineffective.

5. The pharmaceutical composition according to any one of claims 1 to 4, wherein the cancer is breast cancer, lung cancer, colon cancer, kidney cancer, sarcoma, liver cancer, bladder cancer, or ovary cancer.

6. The pharmaceutical composition according to claim 5, wherein the cancer is breast cancer, lung cancer, bladder cancer, or colon cancer.

7. The pharmaceutical composition according to any one of claims 1 to 6, wherein the chemotherapeutic agent is a platinum complex, taxane, pemetrexed, gemcitabine, fluorouracil, irinotecan, etoposide, or doxorubicin.

8. The pharmaceutical composition according to claim 7, wherein the chemotherapeutic agent is a platinum complex, taxane, gemcitabine, or fluorouracil.

9. The pharmaceutical composition according to claim 8, wherein the chemotherapeutic agent is gemcitabine.

10. The pharmaceutical composition according to claim 8, wherein the chemotherapeutic agent is a platinum complex, and the platinum complex is carboplatin, cisplatin, or oxaliplatin.

11. The pharmaceutical composition according to claim 10, wherein the platinum complex is carboplatin.

12. The pharmaceutical composition according to claim 8, wherein the chemotherapeutic agent is a taxane, and the taxane is paclitaxel or docetaxel.

13. The pharmaceutical composition according to claim 8, wherein the chemotherapeutic agent is fluorouracil.

14. The pharmaceutical composition according to any one of claims 1 to 13,
wherein the anti-CCR8 antibody is a monoclonal antibody or an antibody fragment comprising:
1) a light chain variable region including
CDR1 having an amino acid sequence of SEQ ID NO: 2,
CDR2 having an amino acid sequence of SEQ ID NO: 3, and
CDR3 having an amino acid sequence of SEQ ID NO: 4, and
a heavy chain variable region including
CDR1 having an amino acid sequence of SEQ ID NO: 5,
CDR2 having an amino acid sequence of SEQ ID NO: 6, and
CDR3 having an amino acid sequence of SEQ ID NO: 7,
wherein one or more of following substitutions are optionally present:
A) substitution of asparagine with lysine at position 10 in the amino acid sequence of SEQ ID NO: 2,
B) substitution of glycine with glutamine, threonine, alanine, lysine, leucine or arginine at position 11 in the amino acid sequence of SEQ ID NO: 2,
C) substitution of asparagine with glutamine at position 4 in the amino acid sequence of SEQ ID NO: 3,
D) substitution of leucine with isoleucine at position 5 in the amino acid sequence of SEQ ID NO: 3,
E) substitution of leucine with isoleucine at position 4 in the amino acid sequence of SEQ ID NO: 4,
F) substitution of tyrosine with phenylalanine at position 6 in the amino acid sequence of SEQ ID NO: 4,
G) substitution of serine with threonine at position 16 in the amino acid sequence of SEQ ID NO: 6, and
H) substitution of aspartic acid with glutamic acid at position 19 in the amino acid sequence of SEQ ID NO: 6;
2) a light chain variable region including
CDR1 having the amino acid sequence of SEQ ID NO: 2,
CDR2 having the amino acid sequence of SEQ ID NO: 3, and
CDR3 having the amino acid sequence of SEQ ID NO: 4, and
a heavy chain variable region including
CDR1 having an amino acid sequence of SEQ ID NO: 8,
CDR2 having the amino acid sequence of SEQ ID NO: 6, and
CDR3 having the amino acid sequence of SEQ ID NO: 7;
3) a light chain variable region including
CDR1 having the amino acid sequence of SEQ ID NO: 2,
CDR2 having an amino acid sequence of SEQ ID NO: 9, and
CDR3 having an amino acid sequence of SEQ ID NO: 10, and
a heavy chain variable region including
CDR1 having the amino acid sequence of SEQ ID NO: 8,
CDR2 having the amino acid sequence of SEQ ID NO: 6, and
CDR3 having an amino acid sequence of SEQ ID NO: 11,
wherein one or more of following substitutions are optionally present:
A) substitution of serine with threonine at position 2 in the amino acid sequence of SEQ ID NO: 2,
B) substitution of serine with threonine at position 3 in the amino acid sequence of SEQ ID NO: 2,
C) substitution of serine with threonine at position 5 in the amino acid sequence of SEQ ID NO: 2,
D) substitution of glutamine with asparagine at position 2 in the amino acid sequence of SEQ ID NO: 10,
E) substitution of threonine with serine at position 1 in the amino acid sequence of SEQ ID NO: 8,
F) substitution of alanine with valine at position 14 in the amino acid sequence of SEQ ID NO: 6,
G) substitution of lysine with arginine at position 18 in the amino acid sequence of SEQ ID NO: 6,
H) substitution of aspartic acid with glutamic acid at position 19 in the amino acid sequence of SEQ ID NO: 6,
I) substitution of asparagine with glutamine at position 5 in the amino acid sequence of SEQ ID NO: 11, and
J) substitution of tyrosine with phenylalanine at position 12 in the amino acid sequence of SEQ ID NO: 11;
4) a light chain variable region including
CDR1 having the amino acid sequence of SEQ ID NO: 2,
CDR2 having the amino acid sequence of SEQ ID NO: 3, and
CDR3 having the amino acid sequence of SEQ ID NO: 10, and
a heavy chain variable region including
CDR1 having an amino acid sequence of SEQ ID NO: 12,
CDR2 having the amino acid sequence of SEQ ID NO: 6, and
CDR3 having an amino acid sequence of SEQ ID NO: 13;
5) a light chain variable region including
CDR1 having an amino acid sequence of SEQ ID NO: 14,
CDR2 having an amino acid sequence of SEQ ID NO: 15, and
CDR3 having an amino acid sequence of SEQ ID NO: 16, and
a heavy chain variable region including
CDR1 having an amino acid sequence of SEQ ID NO: 17,
CDR2 having an amino acid sequence of SEQ ID NO: 18, and
CDR3 having an amino acid sequence of SEQ ID NO: 19; or
6) a light chain variable region including
CDR1 having an amino acid sequence of SEQ ID NO: 20,
CDR2 having an amino acid sequence of SEQ ID NO: 21, and
CDR3 having an amino acid sequence of SEQ ID NO: 22, and
a heavy chain variable region including
CDR1 having an amino acid sequence of SEQ ID NO: 23,
CDR2 having an amino acid sequence of SEQ ID NO: 24, and
CDR3 having an amino acid sequence of SEQ ID NO: 25.

15. The pharmaceutical composition according to claim 14, wherein the anti-CCR8 antibody is a monoclonal antibody or an antibody fragment comprising:
a light chain variable region including
CDR1 having the amino acid sequence of SEQ ID NO: 2,
CDR2 having the amino acid sequence of SEQ ID NO: 3, and
CDR3 having the amino acid sequence of SEQ ID NO: 4, and
a heavy chain variable region including
CDR1 having the amino acid sequence of SEQ ID NO: 5,
CDR2 having the amino acid sequence of SEQ ID NO: 6, and
CDR3 having the amino acid sequence of SEQ ID NO: 7,
wherein asparagine at position 4 in the amino acid sequence of SEQ ID NO: 3 is substituted with glutamine, and glycine at position 11 in the amino acid sequence of SEQ ID NO: 2 is substituted with arginine.

16. The pharmaceutical composition according to claim 14, wherein the anti-CCR8 antibody is a monoclonal antibody or an antibody fragment comprising:
a light chain variable region including
CDR1 having the amino acid sequence of SEQ ID NO: 2,
CDR2 having the amino acid sequence of SEQ ID NO: 9, and
CDR3 having the amino acid sequence of SEQ ID NO: 10, and
a heavy chain variable region including
CDR1 having the amino acid sequence of SEQ ID NO: 8,
CDR2 having the amino acid sequence of SEQ ID NO: 6, and
CDR3 having the amino acid sequence of SEQ ID NO: 11,
wherein lysine at position 18 in the amino acid sequence of SEQ ID NO: 6 is substituted with arginine.

17. The pharmaceutical composition according to claim 14, wherein the anti-CCR8 antibody is a monoclonal antibody or an antibody fragment comprising:
a light chain variable region including
CDR1 having the amino acid sequence of SEQ ID NO: 20,
CDR2 having the amino acid sequence of SEQ ID NO: 21, and
CDR3 having the amino acid sequence of SEQ ID NO: 22, and
a heavy chain variable region including
CDR1 having the amino acid sequence of SEQ ID NO: 23,
CDR2 having the amino acid sequence of SEQ ID NO: 24, and
CDR3 having the amino acid sequence of SEQ ID NO: 25.

18. The pharmaceutical composition according to any one of claims 14 to 17,
wherein the anti-CCR8 antibody is a humanized monoclonal antibody or an antibody fragment thereof.

19. The pharmaceutical composition according to claim 18, wherein the humanized monoclonal antibody or an antibody fragment thereof comprises:
1) a light chain variable region having an amino acid sequence of SEQ ID NO: 40, and
a heavy chain variable region having an amino acid sequence of SEQ ID NO: 41;
2) a light chain variable region having an amino acid sequence of SEQ ID NO: 42, and
a heavy chain variable region having the amino acid sequence of SEQ ID NO: 41;
3) a light chain variable region having an amino acid sequence of SEQ ID NO: 43, and
a heavy chain variable region having the amino acid sequence of SEQ ID NO: 41;
4) a light chain variable region having an amino acid sequence of SEQ ID NO: 44, and
a heavy chain variable region having the amino acid sequence of SEQ ID NO: 41;
5) a light chain variable region having an amino acid sequence of SEQ ID NO: 45, and
a heavy chain variable region having the amino acid sequence of SEQ ID NO: 41;
6) a light chain variable region having an amino acid sequence of SEQ ID NO: 47, and
a heavy chain variable region having the amino acid sequence of SEQ ID NO: 41;
7) a light chain variable region having the amino acid sequence of SEQ ID NO: 40, and
a heavy chain variable region having an amino acid sequence of SEQ ID NO: 46;
8) a light chain variable region having the amino acid sequence of SEQ ID NO: 42, and
a heavy chain variable region having the amino acid sequence of SEQ ID NO: 46;
9) a light chain variable region having the amino acid sequence of SEQ ID NO: 43, and
a heavy chain variable region having the amino acid sequence of SEQ ID NO: 46;
10) a light chain variable region having the amino acid sequence of SEQ ID NO: 44, and
a heavy chain variable region having the amino acid sequence of SEQ ID NO: 46;
11) a light chain variable region having the amino acid sequence of SEQ ID NO: 45, and
a heavy chain variable region having the amino acid sequence of SEQ ID NO: 46; or
12) a light chain variable region having the amino acid sequence of SEQ ID NO: 47, and
a heavy chain variable region having the amino acid sequence of SEQ ID NO: 46,
wherein one or more of following substitutions are optionally present:
A) substitution of asparagine with lysine at position 33 in the amino acid sequence of SEQ ID NO: 40, 42, 43, 44, 45 or 47;
B) substitution of glycine with glutamine, threonine, alanine, lysine, leucine or arginine at position 34 in the amino acid sequence of SEQ ID NO: 40, 42, 43, 44, 45 or 47;
C) substitution of asparagine with glutamine at position 58 in the amino acid sequence of SEQ ID NO: 40, 42, 43, 44, 45 or 47;
D) substitution of leucine with isoleucine at position 59 in the amino acid sequence of SEQ ID NO: 40, 42, 43, 44, 45 or 47;
E) substitution of leucine with isoleucine at position 97 in the amino acid sequence of SEQ ID NO: 40, 42, 43, 44, 45 or 47;
F) substitution of tyrosine with phenylalanine at position 99 in the amino acid sequence of SEQ ID NO: 40, 42, 43, 44, 45 or 47;
G) substitution of serine with threonine at position 65 in the amino acid sequence of SEQ ID NO: 41 or 46; and
H) substitution of aspartic acid with glutamic acid at position 68 in the amino acid sequence of SEQ ID NO: 41 or 46.

20. The pharmaceutical composition according to claim 18 or 19, wherein the humanized monoclonal antibody or an antibody fragment thereof comprises a light chain variable region having an amino acid sequence of SEQ ID NO: 59 and a heavy chain variable region having the amino acid sequence of SEQ ID NO: 41.

21. The pharmaceutical composition according to claim 18, wherein the humanized monoclonal antibody or an antibody fragment thereof comprises a light chain variable region having an amino acid sequence of SEQ ID NO: 56 and a heavy chain variable region having an amino acid sequence of SEQ ID NO: 60.

22. The pharmaceutical composition according to any one of claims 18 to 21,
wherein the humanized monoclonal antibody or an antibody fragment thereof comprises:
a light chain constant region having an amino acid sequence of SEQ ID NO: 52, and
a heavy chain constant region having an amino acid sequence of SEQ ID NO: 53, wherein
lysine is optionally added to a C-terminal of SEQ ID NO: 53.

23. The pharmaceutical composition according to any one of claims 1 to 22,
wherein the anti-CCR8 antibody is a neutralizing antibody.

24. The pharmaceutical composition according to any one of claims 1 to 22,
wherein the CCR8 antibody has ADCC activity.

25. The pharmaceutical composition according to any one of claims 1 to 22,
wherein the anti-CCR8 antibody is an IgG antibody.

26. A medicament comprising a combination of an anti-CCR8 antibody and a chemotherapeutic agent.

27. The medicament according to claim 26, for cancer treatment.
